# EUROPEAN PATENT SPECIFICATION

(11) **EP 3 515 880 B1**
(45) Date of publication and mention of the grant of the patent: **10.05.2023**
(21) Application number: 18768118.4
(22) Date of filing: 16.03.2018
(51) Int. Cl.: C04B 40/06, C12N 15/10, C12Q 1/6806

(54) **METHODS AND COMPOSITIONS FOR SYNTHESIS OF ENCODED LIBRARIES**
VERFAHREN UND ZUSAMMENSETZUNGEN ZUR SYNTHESE VON CODIERTEN BIBLIOTHEKEN
PROCÉDÉS ET COMPOSITIONS POUR LA SYNTHÈSE DE BIBLIOTHÈQUES CODÉES

(30) Priority: 17.03.2017 WO PCT/CN2017/077108
(43) Date of publication of application: 31.07.2019
(73) Proprietor: Hitgen Inc., High-Tech Zone Chengdu Chengdu Sichuan 610041 (CN)
(72) Inventor: LI, Jin, Chengdu Sichuan 610041 (CN); MORGAN, Barry A., Houston Texas 77004 (US); WAN, Jinqiao, Chengdu Sichuan 610041 (CN); DOU, Dengfeng, Chengdu Sichuan 610041 (CN); LIU, Guansai, Chengdu Sichuan 610041 (CN); CHANG, Yong, Chengdu Sichuan 610041 (CN); CHEN, Qiuxia, Chengdu Sichuan 610041 (CN); WANG, Xing, Chengdu Sichuan 610041 (CN); XU, Yanshan, Chengdu Sichuan 610041 (CN); HU, Dongyan, Chengdu Sichuan 610041 (CN); LIU, Jian, Chengdu Sichuan 610041 (CN); CHENG, Xuemin, Chengdu Sichuan 610041 (CN)
(74) Representative: Vitina, Maruta
(86) International application number: PCT/CN2018/079315
(87) International publication number: WO 2018/166532

(56) References cited:
- WO-A1-93/20242
- WO-A1-2015/091207
- WO-A2-2004/039825
- CN-A- 101 006 177
- CN-A- 106 048 736
- JP-A- H10 251 292
- MATTHEW A CLARK ET AL: "Design, synthesis and selection of DNA-encoded small-molecule libraries (+ Errata)", NATURE CHEMICAL BIOLOGY, NATURE PUBLISHING GROUP, BASINGSTOKE , vol. 5, no. 9 1 September 2009 (2009-09-01), pages 647-654, 772, XP002677464, ISSN: 1552-4450, DOI: 10.1038/NCHEMBIO.211 Retrieved from the Internet: URL:http://www.nature.com/nchembio/journal /v5/n9/full/nchembio.211.html [retrieved on 2009-09-17] & MATTHEW A CLARK ET AL: "Design, synthesis and selection of DNA-encoded small-molecule libraries", NATURE CHEMICAL BIOLOGY, vol. 5, no. 9, 2 August 2009 (2009-08-02), pages 647-654, XP055618604, Basingstoke ISSN: 1552-4450, DOI: 10.1038/nchembio.211
- RAPHAEL M. FRANZINI ET AL: "Identification of Structure-Activity Relationships from Screening a Structurally Compact DNA-Encoded Chemical Library", ANGEWANDTE CHEMIE, INTERNATIONAL EDITION, vol. 54, no. 13, 3 February 2015 (2015-02-03), pages 3927-3931, XP055618607, DE ISSN: 1433-7851, DOI: 10.1002/anie.201410736
- RAPHAEL M. FRANZINI ET AL: "Chemical Space of DNA-Encoded Libraries : Miniperspective", JOURNAL OF MEDICINAL CHEMISTRY, vol. 59, no. 14, 25 February 2016 (2016-02-25), pages 6629-6644, XP055497080, US ISSN: 0022-2623, DOI: 10.1021/acs.jmedchem.5b01874
- SCHEUERMANN, J. et al.: "Dual-pharmacophore DNA-encoded chemical libraries", Current Opinion in Chemical Biology, 13 March 2015 (2015-03-13), pages 99-103, XP055211951,

## Description

### SEQUENCE LISTING

The ASCII text file submitted herewith, entitled "HUB sequence.txt" created on March 15, 2018, having a size of 123,392 bytes, is hereby incorporated by reference in its entirety.

### FIELD

The present disclosure provides method and compositions for synthesizing libraries of molecules each having an encoding oligonucleotide tag.

### BACKGROUND

The search for more efficient methods of identifying compounds having useful biological activities has led to the development of methods for screening vast numbers of distinct compounds, present in collections referred to as combinatorial libraries. Such libraries can include 10⁵ or more distinct compounds. A variety of methods exist for producing combinatorial libraries, and combinatorial synthesis of peptides, peptidomimetics and small organic molecules have been reported.

The two major challenges in the use of combinatorial approaches in drug discovery are the synthesis of libraries of sufficient complexity and diversity, and the identification of molecules which are active in the screens used by effective deconvolution of these libraries. It is generally acknowledged that greater the degree of complexity of a library, i.e., the number of distinct structures present in the library, the greater the probability that the library contains molecules with the activity of interest. Therefore, the chemistry employed in library synthesis must be capable of producing appropriate numbers of compounds within a reasonable time frame. However, for a given scale of synthesis, increasing the number of distinct members within the library lowers the concentration of any particular library member: this complicates the identification of active molecules by deconvolution from high complexity libraries.

One approach for overcoming these obstacles has been the development of strategies involving library encoding, and in particular, libraries in which each compound includes an attached amplifiable entity. Such libraries include DNA-encoded libraries, in which a DNA entity identifying a library member can be amplified and characterized using the techniques of molecular biology, such as the polymerase chain reaction.

Numerous methods exist that utilize a double stranded DNA construct to encode one copy of a small molecule, or the chemistry sequence developed to assemble that molecule. It has been proposed that an approach using a covalently linked, double stranded oligonucleotide could give rise to problems involving such species as substrates for polymerase reactions and consequently lower the fidelity in sequencing analyses for such constructs, which in turn, compromises the identification of the encoded small molecules. Consequently, there is still a significant need for developing improved, more robust methods for the design and construction of such libraries which can enhance the potential for the discovery of small molecule ligands for biological targets. Matthew A Clark et al. ("Design, synthesis and selection of DNA-encoded small-molecule libraries", NATURE CHEMICAL BIOLOGY, NATURE PUBLISHING GROUP, BASINGSTOKE, vol. 5, no. 9, 1 September 2009) disclosed a library synthesis method in which small molecules are covalently attached to an encoding DNA duplex appended with AOP (AOP-headpiece) to form a monovalent conjugate.

Raphael M. Franzini et al. ("Identification of Structure-Activity Relationships from Screening a Structurally Compact DNA-Encoded Chemical Library", ANGEWANDTE CHEMIE, INTERNATIONAL EDITION, vol. 54, no. 13, 3 February 2015) disclosed the synthesis of DAL-100K, a DNA-encoded chemical library containing 103200 structurally compact compounds, where diverse carboxylic acids attached though amide bonds to two diversity elements on a DNA-terminal diamine.

Raphael M. Franzini et al. ("Chemical Space of DNA-Encoded Libraries: Miniperspective", JOURNAL OF MEDICINAL CHEMISTRY, vol. 59, no. 14, 25 February 2016) disclosed DNA-encoded chemical libraries (DECLs) prepared by assembling sets of building blocks in predefined geometries based on the number of diversity points (nBB).

### SUMMARY

In one aspect, provided herein is a compound of Formula II: 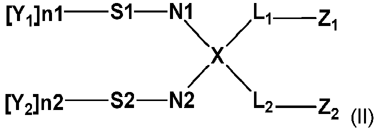 wherein:
X is an atom having a valence of at least 4;
L1 is a first linker having a valence of at least 2, optionally comprising a phosphate group;
Z1 is a first single-stranded oligonucleotide attached at its 3' terminus to the first linker, optionally comprising a phosphate group;
L2 is a second linker having a valence of at least 2;
Z2 is a second single-stranded oligonucleotide attached at its 5' terminus to the second linker;
N1 comprises a first functional group capable of forming at least one covalent bond with S1;
N2 comprises a second functional group capable of forming at least one covalent bond with S2;
S1 and S2 are each independently a spacer having a valence of at least 2;
[Y1]n1 comprises the first functional moiety and [Y2]n2 comprises the second functional moiety, wherein:
   Y1 and Y2, at each occurrence, are each independently a functional moiety comprising one or more synthons; and
   n1 and n2 are each independently an integer of at least 1;
Y1 and Y2 are the same functional moiety; and Z1 and Z2 are substantially complementary. In various embodiments, Y1 and Y2 each independently comprise a functional group,

In some embodiments, S1 and S2 each independently comprises an alkylene chain (such as -(CH₂)ₙ-) or an polyethylene glycol) chain (such as-(CH₂CH₂O)ₙ-), wherein n is an integer from 1 to 20, preferably from 2 to about 12, more preferably from 4 to 10.

In some embodiments, Z1 and Z2 each further comprise a PCR primer binding site sequence.

In some embodiments, L1 and L2 each are -OPO⁻₃-(CH₂CH₂O)ₙ-PO⁻₃-, wherein n is an integer selected from 1 to 10, preferably from 2 to about 6, more preferably 3.

A further aspect relates to a compound library comprising at least about 10² distinct compound species, each species having any one of the compounds disclosed herein (e.g., formula I-IV), wherein each species comprises at least two functional moieties that are operably linked to a double-stranded oligonucleotide which uniquely identifies the at least two functional moieties. In some embodiments, the library comprises at least about 10⁵ or at least about 10⁷ copies of each of the distinct compound species.

Another aspect relates to a method of synthesizing the compound of formula (II), comprising:
(a) providing an initiator compound comprising at least two reactive groups operably linked to an initial oligonucleotide;
(b) reacting the initiator compound with a first synthon comprising a complementary reactive group that is complementary to the at least two reactive groups, thereby producing a cycle 1 product have two copies of the first synthon attached thereto;
(c) joining the initial oligonucleotide with a first incoming oligonucleotide which identifies the first synthon, thereby forming a first encoding oligonucleotide that encodes the cycle 1 product;
(d) optionally, reacting the cycle 1 product with a second synthon and joining the first encoding oligonucleotide with a second incoming oligonucleotide, thereby producing a cycle 2 product encoded by a second encoding oligonucleotide; and
(e) optionally, repeating step (d) i-1 more times using an ith synthon and an ith incoming oligonucleotide, wherein i is an integer of 2 or greater, thereby forming the compound of any one of claims 4-10 having the first and second functional moieties.

In some embodiments, step (c) precedes step (b) or step (b) precedes step (c).

In some embodiments, each synthon can be an amino acid or an activated amino acid.

In some embodiments, the reactive groups and the complementary reactive group are each independently selected from the group consisting of an amino group, an aldehyde group, a ketone group, a hydroxyl group, an alkyne group, an azide group, a carboxyl group, a sulfonyl group, a phosphonyl group, an epoxide group, an aziridine group, a phosphorous ylide group, an isocyanate group, a halogenated heteroaromatic group, and a nucleophile; wherein preferably the halogenated heteroaromatic group is selected from the group consisting of chlorinated pyrimidines, chlorinated triazines and chlorinated purines, and/or wherein preferably the nucleophile comprises an amino group.

In some embodiments, step (b) is conducted under reducing conditions. In certain embodiments, step (b) comprises reacting via cycloaddition to form a cyclic structure.

In some embodiments, step (c) is conducted in the presence of an enzyme that is preferably selected from the group consisting of a DNA ligase, an RNA ligase, a DNA polymerase, an RNA polymerase and a topoisomerase. In some embodiments, the initial oligonucleotide comprises a PCR primer binding site sequence.

In some embodiments, the initial oligonucleotide is single-stranded and each incoming oligonucleotide is single-stranded; or the initial oligonucleotide is double-stranded and each incoming oligonucleotide is double-stranded. In some embodiments, each incoming oligonucleotide is from 3 to 30 nucleotides in length.

In some embodiments, the ith incoming oligonucleotide comprises a PCR closing primer.

In some embodiments, the method further comprises after step (e), cyclizing the first and/or second functional moiety. For example, the first and/or second functional moiety can each comprise an alkynyl group and an azido group, and the compound can be subjected to conditions suitable for cycloaddition of the alkynyl group and the azido group to form a triazole group, thereby cyclizing the first and/or second functional moiety.

Also provided herein is a method of synthesizing a library of compounds, wherein the compounds comprise at least two functional moieties comprising two or more building blocks which are operably linked to an oligonucleotide which identifies the structure of the at least two functional moieties, the method comprising the steps of:
(a) providing a solution comprising m initiator compounds, wherein m is an integer of 1 or greater, wherein the m initiator compounds each comprise at least two initial functional moieties comprising n building blocks, where n is an integer of 1 or greater, which is operably linked to an initial oligonucleotide which identifies the n building blocks;
(b) dividing the solution of step (a) into r reaction vessels, wherein r is an integer of 2 or greater, thereby producing r aliquots of the solution;
(c) reacting the initiator compounds in each reaction vessel with one of r building blocks, thereby producing r aliquots comprising compounds comprising at least two functional moieties comprising n+1 building blocks operably linked to the initial oligonucleotide; and
(d) reacting the initial oligonucleotide in each aliquot with one of a set of r distinct incoming oligonucleotides in the presence of an enzyme which catalyzes the ligation of the incoming oligonucleotide and the initial oligonucleotide, under conditions suitable for enzymatic ligation of the incoming oligonucleotide and the initial oligonucleotide;
thereby producing r aliquots comprising compounds comprising at least two functional moieties operably linked to an elongated oligonucleotide which encodes the building blocks.

In some embodiments, the method further comprises the step of (e) combining two or more of the r aliquots, thereby producing a solution comprising compounds comprising at least two functional moieties which are operably linked to an elongated oligonucleotide which encodes the building blocks.

In some embodiments, steps (a) to (e) are conducted one or more times to yield cycles 1 to i, where i is an integer of 2 or greater, wherein in cycle s+1, where s is an integer of i-1 or less, the solution comprising m initiator compounds of step (a) is the solution of step (e) of cycle s. In some embodiments, in at least one of cycles 1 to i step (d) precedes step (c).

Another aspect relates to a method for identifying one or more compounds which bind to a biological target, the method comprising the steps of:
(a) contacting the biological target with a compound library prepared by any one of the methods disclosed herein, under conditions suitable for at least one member of the compound library to bind to the target;
(b) removing library members that do not bind to the target;
(c) amplifying the encoding oligonucleotides of the at least one member of the compound library which binds to the target;
(d) sequencing the encoding oligonucleotides of step (c); and
(e) using the sequences determined in step (d) to determine the structure of the functional moieties of the members of the compound library which bind to the biological target;
thereby identifying one or more compounds which bind to the biological target.

A further aspect relates to a method for identifying a compound which binds to a biological target, the method comprising the steps of:
(a) contacting the biological target with the compound library disclosed herein under conditions suitable for at least one member of the compound library to bind to the target;
(b) removing library members that do not bind to the target;
(c) amplifying the encoding oligonucleotides of the at least one member of the compound library which binds to the target;
(d) sequencing the encoding oligonucleotides of step (c); and
(e) using the sequences determined in step (d) to determine the structure of the functional moieties of the members of the compound library which bind to the biological target;
thereby identifying one or more compounds which bind to the biological target.

In some embodiments, the library comprises at least about 10⁵ or at least about 10⁷ copies of each of the distinct compound species.

In another aspect, the disclosure provides a method of synthesizing a library of compounds, wherein the compounds comprise at least two functional moieties comprising two or more building blocks which are operably linked to an oligonucleotide which identifies the structure of the at least two functional moieties. The method utilizes a "split and pool" strategy in which a solution comprising an initiator, comprising a first building block linked to an encoding oligonucleotide, is divided ("split") into multiple fractions. In each fraction, the initiator is reacted with a second, unique, building block and a second, unique oligonucleotide which identifies the second building block. These reactions can be simultaneous or sequential and, if sequential, either reaction can precede the other. The dimeric molecules produced in each of the fractions are combined ("pooled") and then divided again into multiple fractions. Each of these fractions is then reacted with a third unique (fraction-specific) building block and a third unique oligonucleotide which encodes the building block. The number of unique molecules present in the product library is a function of (1) the number of different building blocks used at each step of the synthesis, and (2) the number of times the pooling and dividing process is repeated.

In one embodiment, the method comprises the steps of (a) providing a solution comprising m initiator compounds, wherein m is an integer of 1 or greater, wherein the m initiator compounds each comprise at least two initial functional moieties comprising n building blocks, where n is an integer of 1 or greater, which is operably linked to an initial oligonucleotide which identifies the n building blocks; (b) dividing the solution of step (a) into r reaction vessels, wherein r is an integer of 2 or greater, thereby producing r aliquots of the solution; (c) reacting the initiator compounds in each reaction vessel with one of r building blocks, thereby producing r aliquots comprising compounds comprising at least two functional moieties comprising n+1 building blocks operably linked to the initial oligonucleotide; and (d) reacting the initial oligonucleotide in each aliquot with one of a set of r distinct incoming oligonucleotides in the presence of an enzyme which catalyzes the ligation of the incoming oligonucleotide and the initial oligonucleotide, under conditions suitable for enzymatic ligation of the incoming oligonucleotide and the initial oligonucleotide, thereby producing r aliquots comprising compounds comprising at least two functional moieties operably linked to an elongated oligonucleotide which encodes the building blocks. Optionally, the method can further include the step of (e) combining two or more of the r aliquots, thereby producing a solution comprising compounds comprising at least two functional moieties which are operably linked to an elongated oligonucleotide which encodes the building blocks. Steps (a) to (e) can be conducted one or more times to yield cycles 1 to i, where i is an integer of 2 or greater. In cycle s+1, where s is an integer of i-1 or less, the solution comprising m initiator compounds of step (a) is the solution of step (e) of cycle s. Likewise, the initiator compounds of step (a) of cycle s+1 are the compounds of step (e) of cycle s.

In a preferred embodiment, the building blocks are coupled in each step using conventional chemical reactions. The building blocks can be coupled to produce linear or branched polymers or oligomers, such as peptides, peptidomimetics, and peptoids, or non-oligomeric molecules, such as molecules comprising a scaffold structure to which is attached one or more additional chemical moieties. For example, if the building blocks are amino acid residues, the building blocks can be coupled using standard peptide synthesis strategies, such as solution-phase or solid phase synthesis using suitable protection/deprotection strategies as are known in the field. Preferably, the building blocks are coupled using solution phase chemistry. The encoding oligonucleotides are single stranded or double stranded oligonucleotides, preferably double-stranded oligonucleotides. The encoding oligonucleotides are preferably oligonucleotides of 4 to 12 bases or base pairs per building block; the encoding oligonucleotides can be coupled using standard solution phase or solid phase oligonucleotide synthetic methodology, but are preferably coupled using a solution phase enzymatic process. For example, the oligonucleotides can be coupled using a topoisomerase, a ligase, or a DNA polymerase, if the sequence of the encoding oligonucleotides includes an initiation sequence for ligation by one of these enzymes. Enzymatic coupling of the encoding oligonucleotides offers the advantages of (1) greater accuracy of addition compared to standard synthetic (non-enzymatic) coupling; and (2) the use of a simpler protection/deprotection strategy.

In another aspect, the disclosure provides a method for identifying one or more compounds which bind to a biological target, the method comprising the steps of: (a) contacting the biological target with a compound library prepared by the method of Claim 55 under conditions suitable for at least one member of the compound library to bind to the target; (b) removing library members that do not bind to the target; (c) amplifying the encoding oligonucleotides of the at least one member of the compound library which binds to the target; (d) sequencing the encoding oligonucleotides of step (c); and (e) using the sequences determined in step (d) to determine the structure of the functional moieties of the members of the compound library which bind to the biological target, thereby identifying one or more compounds which bind to the biological target.

In yet another aspect, the present disclosure provides a method for identifying a compound which binds to a biological target. The method comprises the steps of (a) contacting the biological target with a compound library comprising at least about 10^2 distinct compounds, said compounds comprising at least two functional moieties comprising two or more building blocks which are operably linked to an oligonucleotide which identifies the structure of the at least two functional moieties under conditions suitable for at least one member of the compound library to bind to the target; (b) removing library members that do not bind to the target; (c) amplifying the encoding oligonucleotides of the at least one member of the compound library which binds to the target; (d) sequencing the encoding oligonucleotides of step (c); and (e) using the sequences determined in step (d) to determine the structure of the functional moieties of the members of the compound library which bind to the biological target, thereby identifying one or more compounds which bind to the biological target.

The present disclosure provides several advantages in the identification of molecules having a desired property. For example, the methods of the disclosure allow the use of a range of chemical reactions for constructing the molecules in the presence of the oligonucleotide tag. The methods of the disclosure also provide a high-fidelity means of incorporating oligonucleotide tags into the chemical structures so produced. Also, they enable the synthesis of libraries having a large number of copies of each member, thereby allowing multiple rounds of selection against a biological target while leaving a sufficient number of molecules following the final round for amplification and sequence of the oligonucleotide tags. Furthermore, the disclosure increase the ratio of chemicals to DNA sequences in the libraries before the screening, which could lead to better recognition of affinity binders by proteins during the screening and better signal/noise ratio after sequencing.

### BRIEF DESCRIPTION OF THE DRAWINGS

FIG. 1 is an exemplary synthesis scheme for a compound having 2 functional moieties.
FIG. 2 is an exemplary synthesis scheme for a compound having 4 functional moieties.
FIG. 3 shows the fold enrichment of divalent vs. monovalent conjugates spiked into ~2.9*10⁷-fold excess of background DEL0936 (3-Cycle DEL, size: 28.69 million) (2-round selection).
FIG.4 shows the fold enrichment of divalent vs. monovalent conjugates spiked into ~1.1*10⁹-fold excess of background DEL1019 (4-Cycle DEL, size: 1.108 billion) (3-round selection).
FIG. 5 is an exemplary synthesis scheme conjugating AOP-HUB with an exemplary compound 1.
FIG. 6 is an exemplary encoding procedure of conjugates.

### DETAILED DESCRIPTION

The present disclosure relates to methods and compositions for producing compounds and combinatorial compound libraries, the compounds and libraries produced therefrom, and methods of using the libraries to identify compounds having a desired property, such as a desired biological activity. The disclosure further relates to the compounds identified using these methods.

A variety of approaches have been taken to produce and screen combinatorial chemical libraries. Examples include methods in which the individual members of the library are physically separated from each other, such as when a single compound is synthesized in each of a multitude of reaction vessels. However, these libraries are typically screened one compound at a time, or at most, several compounds at a time and do not, therefore, result in the most efficient screening process. In other methods, compounds are synthesized on solid supports. Such solid supports include chips in which specific compounds occupy specific regions of the chip or membrane ("position addressable"). In other methods, compounds are synthesized on beads, with each bead containing a different chemical structure.

Two difficulties that arise in screening large libraries are (1) the number of distinct compounds that can be screened; and (2) the identification of compounds which are active in the screen. In one method, the compounds which are active in the screen are identified by narrowing the original library into ever smaller fractions and subfractions, in each case selecting the fraction or subfraction which contains active compounds and further subdividing until attaining an active subfraction which contains a set of compounds which is sufficiently small that all members of the subset can be individually synthesized and assessed for the desired activity. This is a tedious and time consuming activity.

Another method of deconvoluting the results of a combinatorial library screen is to utilize libraries in which the library members are tagged with an identifying label, that is, each label present in the library is associated with a discreet compound structure present in the library, such that identification of the label tells the structure of the tagged molecule. One approach to tagged libraries utilizes oligonucleotide tags, as described, for example, in U.S. Pat. Nos. 5,573,905; 5,708,153; 5,723,598, 6,060,596 published PCT applications WO 93/06121; WO 93/20242; WO 94/13623; WO 00/23458; WO 02/074929 and WO 02/103008, and by Brenner and Lerner (Proc. Natl. Acad. Sci. USA 89, 5381-5383 (1992); Nielsen and Janda (Methods: A Companion to Methods in Enzymology 6, 361-371 (1994); and Nielsen, Brenner and Janda (J. Am. Chem. Soc. 115, 9812-9813 (1 993)).

Such tags can be amplified, using for example, polymerase chain reaction, to produce many copies of the tag and identify the tag by sequencing. The sequence of the tag then identifies the structure of the binding molecule, which can be synthesized in pure form and tested. The present disclosure provides an improvement in methods to produce DNA-encoded libraries, as well as the examples of large (10⁵ members or greater) libraries of DNA-encoded molecules in which the functional moiety is synthesized using solution phase synthetic methods.

The present disclosure provides methods for synthesizing a plurality of distinct compound species that each comprise at least two functional moieties which are made up of building blocks, and a barcode (encoding oligonucleotide) operably linked to the at least two functional moieties. Each barcode can include an oligonucleotide tag which uniquely identifies the structure of at least two functional moieties. In some embodiments, the oligonucleotide tag indicates which building blocks were used in the construction of the at least two functional moieties, as well as the order in which the building blocks were linked. Generally, the information provided by the oligonucleotide tag is sufficient to determine the building blocks used to construct the active moieties. In certain embodiments, the sequence of the oligonucleotide tag is sufficient to determine the arrangement of the building blocks in the functional moieties, for example, for peptidic moieties, the amino acid sequence.

### Definitions

For convenience, certain terms employed in the specification, examples, and appended claims are collected here. Unless defined otherwise, all technical and scientific terms used herein have the same meaning as commonly understood by one of ordinary skill in the art to which this disclosure belongs.

The articles "a" and "an" are used herein to refer to one or to more than one (i.e., at least one) of the grammatical object of the article. By way of example, "an element" means one element or more than one element.

As used herein, the term "about" means within 20%, more preferably within 10% and most preferably within 5%. The term "substantially" means more than 50%, preferably more than 80%, and most preferably more than 90% or 95%.

As used herein, "a plurality of" means more than 1, e.g., 2, 3, 4, 5, 6, 7, 8, 9, 10, 11, 12, 13, 14, 15, 16, 17, 18, 19, 20, or more, e.g., 25, 30, 40, 50, 60, 70, 80, 90, 100, 200, 300, 400, 500, or more, or any integer therebetween.

As used herein, the term "oligonucleotide" refers to a polymeric form of nucleotides that may have various lengths (e.g., 5-500 bases), including deoxyribonucleotides and/or ribonucleotides, or analogs or modifications thereof. The term includes both single-stranded (ss) and double-stranded (ds) molecules, the latter containing two strands that are at least partially complementary to each other. Oligonucleotide sequences that are "complementary" are those that are capable of base-pairing according to the standard Watson-Crick complementarity rules (e.g., A-T and G-C base pairing).

"Functional group" as used herein, refers to a chemical group that is capable of reacting with another group, such as an electrophilic group, a nucleophilic group, a diene, a dienophile, etc. Examples of functional groups include, but are not limited to, -NH₂, -SH, -OH, -CO2H, halo, -N3, -CONH2, etc.

"Linker" as used herein, refers to any linking molecule which is operably linked to an oligonucleotide and which includes at least one functional group.

The term "halo" or "halogen" refers to any radical of fluorine, chlorine, bromine or iodine.

In general, and unless otherwise indicated, substituent (radical) prefix names are derived from the parent hydride by either (i) replacing the "ane" in the parent hydride with the suffixes "yl," "diyl," "triyl," "tetrayl," etc.; or (ii) replacing the "e" in the parent hydride with the suffixes "yl," "diyl," "triyl," "tetrayl," etc. (here the atom(s) with the free valence, when specified, is (are) given numbers as low as is consistent with any established numbering of the parent hydride). Accepted contracted names, e.g., adamantyl, naphthyl, anthryl, phenanthryl, furyl, pyridyl, isoquinolyl, quinolyl, and piperidyl, and trivial names, e.g., vinyl, allyl, phenyl, and thienyl are also used herein throughout. Conventional numbering/lettering systems are also adhered to for substituent numbering and the nomenclature of fused, bicyclic, tricyclic, polycyclic rings.

The term "alkyl" refers to a saturated hydrocarbon chain that may be a straight chain or branched chain, containing the indicated number of carbon atoms. For example, C₁-₆ alkyl indicates that the group may have 1 to 6 (inclusive) carbon atoms in it. Any atom can be optionally substituted, e.g., by one or more substituents. Examples of alkyl groups include without limitation methyl, ethyl, n-propyl, isopropyl, and tert-butyl.

The term "alkenyl" refers to a straight or branched hydrocarbon chain containing the indicated number of carbon atoms and having one or more carbon-carbon double bonds. Any atom can be optionally substituted, e.g., by one or more substituents. Alkenyl groups can include, e.g., vinyl, allyl, 1-butenyl, and 2-hexenyl. One of the double bond carbons can optionally be the point of attachment of the alkenyl substituent.

The term "alkynyl" refers to a straight or branched hydrocarbon chain containing the indicated number of carbon atoms and having one or more carbon-carbon triple bonds. Alkynyl groups can be optionally substituted, e.g., by one or more substituents. Alkynyl groups can include, e.g., ethynyl, propargyl, and 3-hexynyl. One of the triple bond carbons can optionally be the point of attachment of the alkynyl substituent.

The term "cycloalkyl" refers to a fully saturated monocyclic, bicyclic, tricyclic, or other polycyclic hydrocarbon groups. Any atom can be optionally substituted, e.g., by one or more substituents. A ring carbon serves as the point of attachment of a cycloalkyl group to another moiety. Cycloalkyl moieties can include, e.g., cyclopropyl, cyclobutyl, cyclopentyl, cyclohexyl, cycloheptyl, adamantyl, and norbornyl (bicycle[2.2.1]heptyl).

The term "cycloalkenyl" refers to a non-aromatic monocyclic, bicyclic, tricyclic, or other polycyclic hydrocarbon groups having one or more carbon-carbon double bonds. Any atom can be optionally substituted, e.g., by one or more substituents. A ring carbon serves as the point of attachment of a cycloalkenyl group to another moiety. Cycloalkenyl moieties can include, e.g., cyclopentenyl, cyclohexenyl and cycloheptenyl.

The term "heterocycloalkyl" refers to a fully saturated monocyclic, bicyclic, tricyclic or other polycyclic ring system having one or more constituent heteroatom ring atoms independently selected from O, N (it is understood that one or two additional groups may be present to complete the nitrogen valence and/or form a salt), or S. The heteroatom or ring carbon can be the point of attachment of the heterocycloalkyl substituent to another moiety. Any atom can be optionally substituted, e.g., by one or more substituents. Heterocycloalkyl groups can include, e.g., tetrahydrofuryl, tetrahydropyranyl, piperidyl (piperidino), piperazinyl, morpholinyl (morpholino), pyrrolinyl, and pyrrolidinyl.

The term "heterocycloalkenyl" refers to a non-aromatic monocyclic, bicyclic, tricyclic, or other polycyclic hydrocarbon groups having one or more carbon-carbon double bonds and one or more constituent heteroatom ring atoms independently selected from O, N (it is understood that one or two additional groups may be present to complete the nitrogen valence and/or form a salt), or S. The heteroatom or ring carbon can be the point of attachment of the heterocycloalkenyl substituent to another moiety. Any atom can be optionally substituted, e.g., by one or more substituents.

The term "aryl" refers to an aromatic monocyclic, bicyclic (2 fused rings), or tricyclic (3 fused rings), or polycyclic (>3 fused rings) hydrocarbon ring system. One or more ring atoms can be optionally substituted, e.g., by one or more substituents. Aryl moieties include, e.g., phenyl and naphthyl.

The term "heteroaryl" refers to an aromatic monocyclic, bicyclic (2 fused rings), tricyclic (3 fused rings), or polycyclic (>3 fused rings) hydrocarbon groups having one or more heteroatom ring atoms independently selected from O, N (it is understood that one or two additional groups may be present to complete the nitrogen valence and/or form a salt), or S. One or more ring atoms can be optionally substituted, e.g., by one or more substituents. Examples of heteroaryl groups include, but are not limited to, 2H-pyrrolyl, 3H-indolyl, 4H-quinolizinyl, acridinyl, benzo[b]thienyl, benzothiazolyl, P-carbolinyl, carbazolyl, coumarinyl, chromenyl, cinnolinyl, dibenzo[b,d]furanyl, furazanyl, furyl, imidazolyl, imidizolyl, indazolyl, indolyl, isobenzofuranyl, isoindolyl, isoquinolyl, isothiazolyl, isoxazolyl, naphthyridinyl, oxazolyl, perimidinyl, phenanthridinyl, phenanthrolinyl, phenarsazinyl, phenazinyl, phenothiazinyl, phenoxathiinyl, phenoxazinyl, phthalazinyl, pteridinyl, purinyl, pyranyl, pyrazinyl, pyrazolyl, pyridazinyl, pyridyl, pyrimidinyl, pyrrolyl, quinazolinyl, quinolyl, quinoxalinyl, thiadiazolyl, thianthrenyl, thiazolyl, thienyl, triazolyl, and xanthenyl.

The term "amine" includes primary (-NH₂), secondary (-NHR), tertiary (-NRR'), and quaternary (-N⁺RR'R") amine, wherein R, R' and R" are independently selected substituents such as straight chain or branched chain alkyl, alkenyl, alkynyl, cycloalkyl, aryl, heteroaryl, heterocycle, and the like.

The term "substituent" refers to a group "substituted" on, e.g., an alkyl, haloalkyl, cycloalkyl, heterocycloalkyl, heterocycloalkenyl, cycloalkenyl, aryl, or heteroaryl group at any atom of that group. In one aspect, the substituent(s) on a group are independently any one single, or any combination of two or more of the permissible atoms or groups of atoms delineated for that substituent.

The term "valence" of an element is a measure of its combining power with other atoms when it forms chemical compounds or molecules. An atom or group is bivalent when it is capable of forming two covalent bonds, and so on. For example, carbon is tetravalent.

The term "functional moiety" as used herein, refers to a chemical moiety comprising one or more building blocks. Preferably, the building blocks in the functional moiety are not nucleic acids. The functional moiety can be a linear or branched or cyclic polymer or oligomer or a small organic molecule.

The term "building block", as used herein, is used interchangeably with "synthon" and refers to a chemical structural unit which is linked to other chemical structural units or can be linked to other such units. When the functional moiety is polymeric or oligomeric, the building blocks are the monomeric units of the polymer or oligomer. Building blocks can also include a scaffold structure ("scaffold building block") to which is, or can be, attached one or more additional structures ("peripheral building blocks").

It is to be understood that the term "building block" is used herein to refer to a chemical structural unit as it exists in a functional moiety and also in the reactive form used for the synthesis of the functional moiety. Within the functional moiety, a building block will exist without any portion of the building block which is lost as a consequence of incorporating the building block into the functional moiety. For example, in cases in which the bond-forming reaction releases a small molecule, the building block as it exists in the functional moiety is a "building block residue", that is, the remainder of the building block used in the synthesis following loss of the atoms that it contributes to the released molecule.

The building blocks can be any chemical compounds which are complementary, that is the building blocks must be able to react together to form a structure comprising two or more building blocks. Typically, all of the building blocks used will have at least two reactive groups, although it is possible that some of the building blocks (for example the last building block in an oligomeric functional moiety) used will have only one reactive group each. Reactive groups on two different building blocks should be complementary, i.e., capable of reacting together to form a covalent bond, optionally with the concomitant loss of a small molecule, such as water, HCl, HF, and so forth.

For the present purposes, two reactive groups are complementary if they are capable of reacting together to form a covalent bond. In one embodiment, the bond forming reactions occur rapidly under ambient conditions without substantial formation of side products. Preferably, a given reactive group will react with a given complementary reactive group exactly once. In one embodiment, complementary reactive groups of two building blocks react, for example, via nucleophilic substitution, to form a covalent bond. In one embodiment, one member of a pair of complementary reactive groups is an electrophilic group and the other member of the pair is a nucleophilic group.

Complementary electrophilic and nucleophilic groups include any two groups which react via nucleophilic substitution under suitable conditions to form a covalent bond. A variety of suitable bond-forming reactions are known in the art. See, for example, March, Advanced Organic Chemistry, fourth edition, New York: John Wiley and Sons (1992), Chapters 10 to 16; Carey and Sundberg, Advanced Organic Chemistry, Part B, Plenum (1990), Chapters 1-11; and Collman et al., Principles and Applications of Organotransition Metal Chemistry, University Science Books, Mill Valley, Calif. (1987), Chapters 13 to 20.

Examples of suitable electrophilic groups include reactive carbonyl groups, such as acyl chloride groups, ester groups, including carbonyl pentafluorophenyl esters and succinimide esters, ketone groups and aldehyde groups; reactive sulfonyl groups, such as sulfonyl chloride groups, and reactive phosphonyl groups. Other electrophilic groups include terminal epoxide groups, isocyanate groups and alkyl halide groups. Suitable nucleophilic groups include primary and secondary amino groups and hydroxyl groups and carboxyl groups.

Suitable complementary reactive groups are set forth below. One of skill in the art can readily determine other reactive group pairs that can be used in the present method, and the examples provided herein are not intended to be limiting.

In a first embodiment, the complementary reactive groups include activated carboxyl groups, reactive sulfonyl groups or reactive phosphonyl groups, or a combination thereof, and primary or secondary amino groups. In this embodiment, the complementary reactive groups react under suitable conditions to form an amide, sulfonamide or phosphonamidate bond.

In a second embodiment, the complementary reactive groups include epoxide groups and primary or secondary amino groups. An epoxide-containing building block reacts with an amine-containing building block under suitable conditions to form a carbon-nitrogen bond, resulting in a β-amino alcohol.

In another embodiment, the complementary reactive groups include aziridine groups and primary or secondary amino groups. Under suitable conditions, an aziridine-containing building block reacts with an amine-containing building block to form a carbon-nitrogen bond, resulting in a 1,2-diamine. In a third embodiment, the complementary reactive groups include isocyanate groups and primary or secondary amino groups. An isocyanate-containing building block will react with an amino-containing building block under suitable conditions to form a carbon-nitrogen bond, resulting in a urea group.

In a fourth embodiment, the complementary reactive groups include isocyanate groups and hydroxyl groups. An isocyanate-containing building block will react with an hydroxyl-containing building block under suitable conditions to form a carbon-oxygen bond, resulting in a carbamate group.

In a fifth embodiment, the complementary reactive groups include amino groups and carbonyl-containing groups, such as aldehyde or ketone groups. Amines react with such groups via reductive amination to form a new carbon-nitrogen bond.

In a sixth embodiment, the complementary reactive groups include phosphorous ylide groups and aldehyde or ketone groups. A phosphorus-ylide-containing building block will react with an aldehyde or ketone-containing building block under suitable conditions to form a carbon-carbon double bond, resulting in an alkene.

In a seventh embodiment, the complementary reactive groups react via cycloaddition to form a cyclic structure. One example of such complementary reactive groups are alkynes and organic azides, which react under suitable conditions to form a triazole ring structure. Suitable conditions for such reactions are known in the art and include those disclosed in WO 2003/101972.

In an eighth embodiment, the complementary reactive groups are an alkyl halide and a nucleophile, such as an amino group, a hydroxyl group or a carboxyl group. Such groups react under suitable conditions to form a carbon-nitrogen (alkyl halide plus amine) or carbon oxygen (alkyl halide plus hydroxyl or carboxyl group).

In a ninth embodiment, the complementary functional groups are a halogenated heteroaromatic group and a nucleophile, and the building blocks are linked under suitable conditions via aromatic nucleophilic substitution. Suitable halogenated heteroaromatic groups include chlorinated pyrimidines, triazines and purines, which react with nucleophiles, such as amines, under mild conditions in aqueous solution.

It is to be understood that the synthesis of a functional moiety can proceed via one particular type of coupling reaction, such as, but not limited to, one of the reactions discussed above, or via a combination of two or more coupling reactions, such as two or more of the coupling reactions discussed above. For example, in one embodiment, the building blocks are joined by a combination of amide bond formation (amino and carboxylic acid complementary groups) and reductive amination (amino and aldehyde or ketone complementary groups). Any coupling chemistry can be used, provided that it is compatible with the presence of an oligonucleotide. Double stranded (duplex) oligonucleotide tags, as used in certain embodiments of the present disclosure, are chemically more robust than single stranded tags, and, therefore, tolerate a broader range of reaction conditions and enable the use of bond-forming reactions that would not be possible with single-stranded tags.

A building block can include one or more functional groups in addition to the reactive group or groups employed to form the functional moiety. One or more of these additional functional groups can be protected to prevent undesired reactions of these functional groups. Suitable protecting groups are known in the art for a variety of functional groups (Greene and Wuts, Protective Groups in Organic Synthesis, second edition, New York: John

Wiley and Sons (1991). Particularly useful protecting groups include t-butyl esters and ethers, acetals, trityl ethers and amines, acetyl esters, trimethylsilyl ethers, trichloroethyl ethers and esters and carbamates.

In one embodiment, each building block comprises two reactive groups, which can be the same or different. For example, each building block added in cycle s can comprise two reactive groups which are the same, but which are both complementary to the reactive groups of the building blocks added at steps s-1 and s+1. In another embodiment, each building block comprises two reactive groups which are themselves complementary. For example, a library comprising polyamide molecules can be produced via reactions between building blocks comprising two primary amino groups and building blocks comprising two activated carboxyl groups. In the resulting compounds there is no N- or C-terminus, as alternate amide groups have opposite directionality. Alternatively, a polyamide library can be produced using building blocks that each comprise an amino group and an activated carboxyl group. In this embodiment, the building blocks added in step n of the cycle will have a free reactive group which is complementary to the available reactive group on the n-1 building block, while, preferably, the other reactive group on the nth building block is protected. For example, if the members of the library are synthesized from the C to N direction, the building blocks added will comprise an activated carboxyl group and a protected amino group.

The functional moieties can be polymeric or oligomeric moieties, such as peptides, peptidomimetics, peptide nucleic acids or peptoids, or they can be small non-polymeric molecules, for example, molecules having a structure comprising a central scaffold and structures arranged about the periphery of the scaffold. Linear polymeric or oligomeric libraries will result from the use of building blocks having two reactive groups, while branched polymeric or oligomeric libraries will result from the use of building blocks having three or more reactive groups, optionally in combination with building blocks having only two reactive groups. Such molecules can be represented by the general formula X1X2 ... Xn, where each X is a monomeric unit of a polymer comprising n monomeric units, where n is an integer greater than 1. In the case of oligomeric or polymeric compounds, the terminal building blocks need not comprise two functional groups. For example, in the case of a polyamide library, the C-terminal building block can comprise an amino group, but the presence of a carboxyl group is optional. Similarly, the building block at the N-terminus can comprise a carboxyl group, but need not contain an amino group.

Branched oligomeric or polymeric compounds can also be synthesized provided that at least one building block comprises three functional groups which are reactive with other building blocks. A library of the disclosure can comprise linear molecules, branched molecules or a combination thereof.

Libraries can also be constructed using, for example, a scaffold building block having two or more reactive groups, in combination with other building blocks having only one available reactive group, for example, where any additional reactive groups are either protected or not reactive with the other reactive groups present in the scaffold building block.

In one embodiment, the libraries of the disclosure comprise polyamide compounds. The polyamide compounds can be composed of building blocks derived from any amino acids, including the twenty naturally occurring α-amino acids, such as alanine (Ala; A), glycine (Gly; G), asparagine (Asn; N), aspartic acid (Asp; D), glutamic acid (Glu; E), histidine (His; H), leucine (Leu; L), lysine (Lys; K), phenylalanine (Phe; F), tyrosine (Tyr; Y), threonine (Thr; T), serine (Ser; S), arginine (Arg; R), valine (Val; V), glutamine (Gln; Q), isoleucine (Ile; l), cysteine (Cys; C), methionine (Met; M), proline (Pro; P) and tryptophan (Trp; W), where the three-letter and one-letter codes for each amino acid are given. In their naturally occurring form, each of the foregoing amino acids exists in the L-configuration, which is to be assumed herein unless otherwise noted. In the present method, however, the D-configuration forms of these amino acids can also be used. These D-amino acids are indicated herein by lower case three- or one-letter code, i.e., ala (a), gly (g), leu (l), gln (q), thr (t), ser (s), and so forth. The building blocks can also be derived from other α-amino acids, including, but not limited to, 3-arylalanines, such as naphthylalanine, phenyl-substituted phenylalanines, including 4-fluoro-, 4-chloro, 4-bromo and 4-methylphenylalanine; 3-heteroarylalanines, such as 3-pyridylalanine, 3-thienylalanine, 3-quinolylalanine, and 3-imidazolylalanine; ornithine; citrulline; homocitrulline; sarcosine; homoproline; homocysteine; substituted proline, such as hydroxyproline and fluoroproline; dehydroproline; norleucine; O-methyltyrosine; O-methylserine; O-methylthreonine and 3-cyclohexylalanine. Each of the preceding amino acids can be utilized in either the D- or L-configuration.

The building blocks can also be amino acids which are not α-amino acids, such as α-azamino acids; β, γ, δ, ε-amino acids, and N-substituted amino acids, such as N-substituted glycine, where the N-substituent can be, for example, a substituted or unsubstituted alkyl, aryl, heteroaryl, arylalkyl or heteroarylalkyl group. In one embodiment, the N-substituent is a side chain from a naturally-occurring or non-naturally occurring α-amino acid. The building block can also be a peptidomimetic structure, such as a dipeptide, tripeptide, tetrapeptide or pentapeptide mimetic. Such peptidomimetic building blocks can be derived from amino acyl compounds, such that the chemistry of addition of these building blocks to the growing poly(aminoacyl) group is the same as, or similar to, the chemistry used for the other building blocks. The building blocks can also be molecules which are capable of forming bonds which are isosteric with a peptide bond, to form peptidomimetic functional moieties comprising a peptide backbone modification, such as ψ[CH2S], ψ[CH2NH], ψ[CSNH2], ψ[NHCO], ψ[COCH2], and ψ[(E) or (Z) CH=CH]. In the nomenclature used above, ψ indicates the absence of an amide bond. The structure that replaces the amide group is specified within the brackets.

### Methods and Compositions for Library Construction and Use thereof

In one embodiment, the disclosure provides a method of synthesizing a molecule comprising at least two functional moieties which are operably linked to an encoding oligonucleotide. The method includes the steps of: (a) providing an initiator compound comprising at least two initial functional moieties each comprising n building blocks, where n is an integer of 1 or greater, wherein the at least two initial functional moieties each comprise at least one reactive group, and is operably linked to an initial oligonucleotide; (b) reacting the initiator compound with a building block comprising at least one complementary reactive group, wherein the at least one complementary reactive group is complementary to the at least one reactive group of step (a), under conditions suitable for reaction of the complementary reactive group to form a covalent bond; (c) reacting the initial oligonucleotide with an incoming oligonucleotide which identifies the building block of step (b) in the presence of an enzyme which catalyzes ligation of the initial oligonucleotide and the incoming oligonucleotide, under conditions suitable for ligation of the incoming oligonucleotide and the initial oligonucleotide to form an encoding oligonucleotide, thereby producing a molecule which comprises at least two functional moieties operably linked to the encoding oligonucleotide. If the at least two functional moieties of step (c) comprise at least one reactive group, steps (a)-(c) can be repeated one or more times, thereby forming cycles 1 to i, where i is an integer of 2 or greater, with the product of step (c) of a cycle s, where s is an integer of i-1 or less, becoming the initiator compound of step (1) of cycle s + 1. In each cycle, one building block is added to the growing functional moiety and one oligonucleotide sequence, which encodes the new building block, is added to the growing encoding oligonucleotide.

In one embodiment, each individual building block is associated with a distinct oligonucleotide, such that the sequence of nucleotides in the oligonucleotide added in a given cycle identifies the building block added in the same cycle.

The coupling of building blocks and ligation of oligonucleotides will generally occur at similar concentrations of starting materials and reagents. For example, concentrations of reactants on the order of micromolar to millimolar, for example from about 10 µM to about 10 mM, can be used in order to have efficient coupling of building blocks.

In certain embodiments, the method further comprises, following step (b), the step of scavenging any unreacted initial functional moiety. Scavenging any unreacted initial functional moiety in a particular cycle prevents the initial functional moiety of the cycle from reacting with a building block added in a later cycle. Such reactions could lead to the generation of functional moieties missing one or more building blocks, potentially leading to a range of functional moiety structures which correspond to a particular oligonucleotide sequence. Such scavenging can be accomplished by reacting any remaining initial functional moiety with a compound which reacts with the reactive group of step (b). In some embodiments, the scavenger compound reacts rapidly with the reactive group of step (b) and includes no additional reactive groups that can react with building blocks added in later cycles. For example, in the synthesis of a compound where the reactive group of step (b) is an amino group, a suitable scavenger compound is an N-hydroxysuccinimide ester, such as acetic acid N-hydroxysuccinimide ester.

The disclosure further relates to the compounds which can be produced using the methods of the disclosure, and collections of such compounds, either as isolated species or pooled to form a library of chemical structures. In some embodiments, the compound can have formula (I): 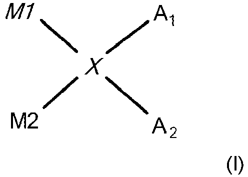 wherein X is an atom or a molecular scaffold; A1 is a moiety comprising a divalent linker and an oligonucleotide; A2 is a moiety comprising a divalent linker and an oligonucleotide; M1 is a moiety including a functional group; and M2 is a moiety including a functional group. In some embodiments, X is a tetravalent or higer polyvalent moiety comprising an atom or a group of atoms. In one embodiment, X is a carbon atom, a boron atom, a nitrogen atom, a phosphorus atom, or a polyatomic scaffold. In another embodiment, X is a phosphate group, a cyclic group or a polycyclic group. In yet another embodiment, X is a cycloalkyl, cycloalkenyl, heterocycloalkyl, heterocycloalkenyl, aryl or heteroaryl group.

In some embodiments, the compounds are of formula (II): 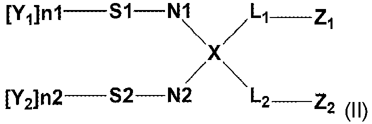 where L1 is a divalent linker; Z1 is an oligonucleotide attached at the 3' terminus to L1; L2 is a divalent linker; Z2 is an oligonucleotide attached at the 5' terminus to L2; N1 is a moiety including a functional group, which is attached to a poly-functional spacer S1, which in turn is attached to n1 copies of a moiety Y1, wherein each copy of Y1 independently comprises at least one synthon; N2 is a moiety including a functional group, which is attached to a poly-functional spacer S2, which in turn is attached to n2 copies of a moiety Y2, wherein each copy of Y2 independently comprises at least one synthon; and synthons are a set of chemically similar building blocks that can together comprise the molecular moieties Y1 and Y2. n1 copies of the moiety Y1 can be the same or different. n2 copies of the moiety Y2 can be the same or different. Y1 and Y2 can each independently comprise an amino group, a hydroxyl group, a carboxylic acid group, a thiol group, a halide, an azido group, an alkyne group, or an alkene group.

In various embodiments, Z1 and Z2 are substantially complementary and are oriented in the compound so as to enable Watson-Crick base pairing and duplex formation under suitable conditions. Z1 and Z2 are the same length or different lengths. Z1 and Z2 may be of substantially the same length, or one of Z1 and Z2 is from 1 to 10 bases longer than the other. In some embodiments, Z1 and Z2 are each 10 or more bases in length and have complementary regions often or more base pairs. In one embodiment, Z1 and Z2 are substantially complementary throughout their length, i.e., they have no more than one mismatch per every ten base pairs. Z1 and Z2 can also be complementary throughout their length, i.e., except for any overhang region on Z1 or Z2, the strands hybridize via Watson-Crick base pairing with no mismatches throughout their entire length.

S1 and S2 can each independently comprise an alkylene chain or an oligo(ethylene glycol)chain. L1 and L2 can each independently comprise a phosphate group.

In one embodiment, the L1 and L2 can have the structure

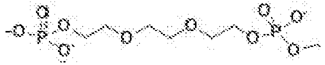

In another embodiment, the disclosure provides a method of producing a library of compounds, wherein each compound comprises at least two functional moieties comprising two or more building blocks which are operably linked to an oligonucleotide. In one embodiment, the oligonucleotide present in each molecule provides sufficient information to identify the building blocks within the molecule and, optionally, the order of addition of the building blocks. In this embodiment, the method of the disclosure comprises a method of synthesizing a library of compounds, wherein the compounds comprise at least two functional moieties comprising two or more building blocks which are operably linked to an oligonucleotide which identifies the structure of the at least two functional moieties. The method comprises the steps of (a) providing a solution comprising m initiator compounds, wherein m is an integer of 1 or greater, wherein the m initiator compounds each comprise at least two initial functional moieties comprising n building blocks, where n is an integer of 1 or greater, which is operably linked to an initial oligonucleotide which identifies the n building blocks; (b) dividing the solution of step (a) into r reaction vessels, wherein r is an integer of 2 or greater, thereby producing r aliquots of the solution; (c) reacting the initiator compounds in each reaction vessel with one of r building blocks, thereby producing r aliquots comprising compounds comprising at least two functional moieties comprising n+1 building blocks operably linked to the initial oligonucleotide; and (d) reacting the initial oligonucleotide in each aliquot with one of a set of r distinct incoming oligonucleotides in the presence of an enzyme which catalyzes the ligation of the incoming oligonucleotide and the initial oligonucleotide, under conditions suitable for enzymatic ligation of the incoming oligonucleotide and the initial oligonucleotide, thereby producing r aliquots comprising compounds comprising at least two functional moieties operably linked to an elongated oligonucleotide which encodes the building blocks. Optionally, the method can further include the step of (e) combining two or more of the r aliquots, thereby producing a solution comprising compounds comprising at least two functional moieties which are operably linked to an elongated oligonucleotide which encodes the building blocks. Steps (a) to (e) can be conducted one or more times to yield cycles 1 to i, where i is an integer of 2 or greater. In cycle s+1, where s is an integer of i-1 or less, the solution comprising m initiator compounds of step (a) is the solution of step (e) of cycle s. Likewise, the initiator compounds of step (a) of cycle s+1 are the products of step (e) in cycle s.

In some embodiments, the solution of step (b) is divided into r fractions in each cycle of the library synthesis. In this embodiment, each fraction is reacted with a unique building block.

In the methods of the disclosure, the order of addition of the building block and the incoming oligonucleotide is not critical, and steps (b) and (c) of the synthesis of a molecule, and steps (c) and (d) in the library synthesis can be reversed, i.e., the incoming oligonucleotide can be ligated to the initial oligonucleotide before the new building block is added. In certain embodiments, it may be possible to conduct these two steps simultaneously. In certain embodiments, the method further comprises, following step (b), the step of scavenging any unreacted initial functional moiety. Scavenging any unreacted initial functional moiety in a particular cycle prevents the initial functional moiety of the cycle from reacting with a building block added in a later cycle. Such reactions could lead to the generation of functional moieties missing one or more building blocks, potentially leading to a range of functional moiety structures which correspond to a particular oligonucleotide sequence. Such scavenging can be accomplished by reacting any remaining initial functional moiety with a compound which reacts with the reactive group of step (b). In some embodiments, the scavenger compound reacts rapidly with the reactive group of step (b) and includes no additional reactive groups that can react with building blocks added in later cycles. For example, in the synthesis of a compound where the reactive group of step (b) is an amino group, a suitable scavenger compound is an N-hydroxysuccinimide ester, such as acetic acid N-hydroxysuccinimide ester.

In one embodiment, the building blocks used in the library synthesis are selected from a set of candidate building blocks by evaluating the ability of the candidate building blocks to react with appropriate complementary functional groups under the conditions used for synthesis of the library. Building blocks which are shown to be suitably reactive under such conditions can then be selected for incorporation into the library. The products of a given cycle can, optionally, be purified. When the cycle is an intermediate cycle, i.e., any cycle prior to the final cycle, these products are intermediates and can be purified prior to initiation of the next cycle. If the cycle is the final cycle, the products of the cycle are the final products, and can be purified prior to any use of the compounds. This purification step can, for example, remove unreacted or excess reactants and the enzyme employed for oligonucleotide ligation. Any methods which are suitable for separating the products from other species present in solution can be used, including liquid chromatography, such as high performance liquid chromatography (HPLC) and precipitation with a suitable co-solvent, such as ethanol. Suitable methods for purification will depend upon the nature of the products and the solvent system used for synthesis.

The reactions are, in some embodiments, conducted in aqueous solution, such as a buffered aqueous solution, but can also be conducted in mixed aqueous/organic media consistent with the solubility properties of the building blocks, the oligonucleotides, the intermediates and final products and the enzyme used to catalyze the oligonucleotide ligation.

It is to be understood that the theoretical number of compounds produced by a given cycle in the method described above is the product of the number of different initiator compounds, m, used in the cycle and the number of distinct building blocks added in the cycle, r. The actual number of distinct compounds produced in the cycle can be as high as the product of r and m (r×m), but could be lower, given differences in reactivity of certain building blocks with certain other building blocks. For example, the kinetics of addition of a particular building block to a particular initiator compound may be such that on the time scale of the synthetic cycle, little to none of the product of that reaction may be produced.

In certain embodiments, a common building block is added prior to cycle 1, following the last cycle or in between any two cycles. For example, when the functional moiety is a polyamide, a common N-terminal capping building block can be added after the final cycle. A common building block can also be introduced between any two cycles, for example, to add a functional group, such as an alkyne or azide group, which can be utilized to modify the functional moieties, for example by cyclization, following library synthesis.

The term "operably linked", as used herein, means that two chemical structures are linked together in such a way as to remain linked through the various manipulations they are expected to undergo. Typically the functional moiety and the encoding oligonucleotide are linked covalently via an appropriate linking group. The linking group is a bivalent moiety with a site of attachment for the oligonucleotide and a site of attachment for the functional moiety. For example, when the functional moiety is a polyamide compound, the polyamide compound can be attached to the linking group at its N-terminus, its C-terminus or via a functional group on one of the side chains. The linking group is sufficient to separate the polyamide compound and the oligonucleotide by at least one atom, and in some embodiments, by more than one atom, such as at least two, at least three, at least four, at least five or at least six atoms. In certain embodiments, the linking group is sufficiently flexible to allow the polyamide compound to bind target molecules in a manner which is independent of the oligonucleotide.

In one embodiment, the linking group is attached to the N-terminus of the polyamide compound and the 5'-phosphate group of the oligonucleotide. For example, the linking group can be derived from a linking group precursor comprising an activated carboxyl group on one end and an activated ester on the other end. Reaction of the linking group precursor with the N-terminal nitrogen atom will form an amide bond connecting the linking group to the polyamide compound or N-terminal building block, while reaction of the linking group precursor with the 5'-hydroxy group of the oligonucleotide will result in attachment of the oligonucleotide to the linking group via an ester linkage. The linking group can comprise, for example, a polymethylene chain, such as a -(CH₂)n-chain or a polyethylene glycol) chain, such as a -(CH2CH2O)n chain, where in both cases n is an integer from 1 to about 20. Preferably, n is from 2 to about 12, more preferably from about 4 to about 10. In one embodiment, the linking group comprises a hexamethylene (-(CH₂)₆-) group.

When the building blocks are amino acid residues, the resulting functional moiety is a polyamide. The amino acids can be coupled using any suitable chemistry for the formation of amide bonds. In some embodiments, the coupling of the amino acid building blocks is conducted under conditions which are compatible with enzymatic ligation of oligonucleotides, for example, at neutral or near-neutral pH and in aqueous solution. In one embodiment, the polyamide compound is synthesized from the C-terminal to N-terminal direction. In this embodiment, the first, or C-terminal, building block is coupled at its carboxyl group to an oligonucleotide via a suitable linking group. The first building block is reacted with the second building block, which, in some embodiments, can have an activated carboxyl group and a protected amino group. Any activating/protecting group strategy which is suitable for solution phase amide bond formation can be used. For example, suitable activated carboxyl species include acyl fluorides (U.S. Pat. No. 5,360,928), symmetrical anhydrides and N-hydroxysuccinimide esters. The acyl groups can also be activated in situ, as is known in the art, by reaction with a suitable activating compound. Suitable activating compounds include dicyclohexylcarbodiimide (DCC), diisopropylcarbodiimide (DIC), 1-ethoxycarbonyl-2-ethoxy-1,2-dihydroquinoline (EEDQ), 1-ethyl-3-(3-dimethylaminopropyl)carbodiimide hydrochloride (EDC), n-propane-phosphonic anhydride (PPA), N,N-bis (2-oxo-3-oxazolidinyl)imido-phosphoryl chloride (BOP-CI), bromo-tris-pyrrolidinophosphonium hexafluorophosphate (PyBrop), diphenylphosphoryl azide (DPPA), Castro's reagent (BOP, PyBop), O-benzotriazolyl-N,N,N',N'-tetramethyluronium salts (HBTU), diethylphosphoryl cyanide (DEPCN), 2,5-diphenyl-2,3-dihydro-3-oxo-4-hydroxy-thiophene dioxide (Steglich's reagent; HOTDO), 1,1'-carbonyl-diimidazole (CDI), and 4-(4,6-dimethoxy-1 ,3,5-triazin-2-yl)-4-methylmorpholinium chloride (DMT-MM). The coupling reagents can be employed alone or in combination with additives such as N. N-dimethyl-4-aminopyridine (DMAP), N-hydroxy-benzotriazole (HOBt), N-hydroxybenzotriazine (HOOBt), N-hydroxysuccinimide (HOSu) N-hydroxyazabenzotriazole (HOAt), azabenzotriazolyl-tetramethyluronium salts (HATU, HAPyU) or 2-hydroxypyridine. In certain embodiments, synthesis of a library requires the use of two or more activation strategies, to enable the use of a structurally diverse set of building blocks. For each building block, one skilled in the art can determine the appropriate activation strategy.

The N-terminal protecting group can be any protecting group which is compatible with the conditions of the process, for example, protecting groups which are suitable for solution phase synthesis conditions. An exemplary protecting group is the fluorenylmethoxycarbonyl ("Fmoc") group. Any potentially reactive functional groups on the side chain of the aminoacyl building block may also need to be suitably protected. In some embodiments, the side chain protecting group is orthogonal to the N-terminal protecting group, that is, the side chain protecting group is removed under conditions which are different than those required for removal of the N-terminal protecting group. Suitable side chain protecting groups include the nitroveratryl group, which can be used to protect both side chain carboxyl groups and side chain amino groups. Another suitable side chain amine protecting group is the N-pent-4-enoyl group.

The building blocks can be modified following incorporation into the functional moiety, for example, by a suitable reaction involving a functional group on one or more of the building blocks. Building block modification can take place following addition of the final building block or at any intermediate point in the synthesis of the functional moiety, for example, after any cycle of the synthetic process. When a library of bifunctional molecules of the disclosure is synthesized, building block modification can be carried out on the entire library or on a portion of the library, thereby increasing the degree of complexity of the library. Suitable building block modifying reactions include those reactions that can be performed under conditions compatible with the functional moiety and the encoding oligonucleotide. Examples of such reactions include acylation and sulfonation of amino groups or hydroxyl groups, alkylation of amino groups, esterification or thioesterification of carboxyl groups, amidation of carboxyl groups, epoxidation of alkenes, and other reactions as are known the art. When the functional moiety includes a building block having an alkyne or an azide functional group, the azide/alkyne cycloaddition reaction can be used to derivatize the building block. For example, a building block including an alkyne can be reacted with an organic azide, or a building block including an azide can be reacted with an alkyne, in either case forming a triazole. Building block modification reactions can take place after addition of the final building block or at an intermediate point in the synthetic process, and can be used to append a variety of chemical structures to the functional moiety, including carbohydrates, metal binding moieties and structures for targeting certain biomolecules or tissue types.

In another embodiment, the functional moiety comprises a linear series of building blocks and this linear series is cyclized using a suitable reaction. For example, if at least two building blocks in the linear array include sulffiydryl groups, the sulfhydryl groups can be oxidized to form a disulfide linkage, thereby cyclizing the linear array. For example, the functional moieties can be oligopeptides which include two or more L or D-cysteine and/or L or D-homocysteine moieties. The building blocks can also include other functional groups capable of reacting together to cyclize the linear array, such as carboxyl groups and amino or hydroxyl groups.

In one embodiment, one of the building blocks in the linear array comprises an alkyne group and another building block in the linear array comprises an azide group. The azide and alkyne groups can be induced to react via cycloaddition, resulting in the formation of a macrocyclic structure. In a particle embodiment, the functional moiety is a polypeptide comprising a propargylglycine building block at its C-terminus and an azidoacetyl group at its N-terminus. Reaction of the alkyne and the azide group under suitable conditions results in formation of a cyclic compound, which includes a triazole structure within the macrocycle. In the case of a library, in one embodiment, each member of the library comprises alkyne- and azide-containing building blocks and can be cyclized in this way. In a second embodiment, all members of the library comprises alkyne- and azide-containing building blocks, but only a portion of the library is cyclized. In a third embodiment, only certain functional moieties include alkyne- and azide-containing building blocks, and only these molecules are cyclized. In the forgoing second and third embodiments, the library, following the cycloaddition reaction, will include both cyclic and linear functional moieties.

The oligonucleotides are ligated using enzymatic methods. In one embodiment, the initial building block is operably linked to an initial oligonucleotide. Prior to or following coupling of a second building block to the initial building block, a second oligonucleotide sequence which identifies the second building block is ligated to the initial oligonucleotide. In one embodiment, the initial oligonucleotide is double-stranded, and one strand includes an overhang sequence which is complementary to one end of the second oligonucleotide and brings the second oligonucleotide into contact with the initial oligonucleotide. In some embodiments, the overhanging sequence of the initial oligonucleotide and the complementary sequence of the second oligonucleotide are both at least about 4 bases; more preferably both sequences are both the same length. The initial oligonucleotide and the second oligonucleotide can be ligated using a suitable enzyme. If the initial oligonucleotide is linked to the first building block at the 5' end of one of the strands (the "top strand"), then the strand which is complementary to the top strand (the "bottom strand") will include the overhang sequence at its 5' end, and the second oligonucleotide will include a complementary sequence at its 5' end. Following ligation of the second oligonucleotide, a strand can be added which is complementary to the sequence of the second oligonucleotide which is 3'to the overhang complementary sequence, and which includes additional overhang sequence.

In one embodiment, the oligonucleotide bound to the growing functional moiety and the incoming oligonucleotide are positioned for ligation by the use of a "splint" sequence, which includes a region which is complementary to the 3' end of the initial oligonucleotide and a region which is complementary to the 5' end of the incoming oligonucleotide. The splint brings the 5' end of the oligonucleotide into proximity with the 3' end of the incoming oligo and ligation is accomplished using enzymatic ligation. In a particular embodiment, the initial oligonucleotide consists of 16 nucleobases and the splint is complementary to the 6 bases at the 3' end. The incoming oligonucleotide consists of 12 nucleobases, and the splint is complementary to the 6 bases at the 5' terminus. The length of the splint and the lengths of the complementary regions are not critical. However, the complementary regions should be sufficiently long to enable stable dimer formation under the conditions of the ligation, but not so long as to yield an excessively large encoding nucleotide in the final molecules. It is preferred that the complementary regions are from about 4 bases to about 12 bases, more preferably from about 5 bases to about 10 bases, and most preferably from about 5 bases to about 8 bases in length.

In one embodiment, the initial oligonucleotide is double-stranded and the two strands are covalently joined. A linking moiety is used to link the two strands and the functional moiety. The linking moiety can be any chemical structure which comprises a first functional group which is adapted to react with a building block, a second functional group which is adapted to react with the 3'-end of an oligonucleotide, and a third functional group which is adapted to react with the 5'-end of an oligonucleotide. Preferably, the second and third functional groups are oriented so as to position the two oligonucleotide strands in a relative orientation that permits hybridization of the two strands.

In embodiments in which the initial oligonucleotide is double-stranded, the incoming oligonucleotides are also double-stranded. In one embodiment, the initial oligonucleotide can have one strand which is longer than the other, providing an overhang sequence. In this embodiment, the incoming oligonucleotide includes an overhang sequence which is complementary to the overhang sequence of the initial oligonucleotide. Hybridization of the two complementary overhang sequences brings the incoming oligonucleotide into position for ligation to the initial oligonucleotide. This ligation can be performed enzymatically using a DNA or RNA ligase. The overhang sequences of the incoming oligonucleotide and the initial oligonucleotide can have the same length and consist of two or more nucleotides, e.g., from 2 to about 10 nucleotides, or from 2 to about 6 nucleotides. In one embodiment, the incoming oligonucleotide is a double-stranded oligonucleotide having an overhang sequence at each end. The overhang sequence at one end is complementary to the overhang sequence of the initial oligonucleotide, while, after ligation of the incoming oligonucleotide and the initial oligonucleotide, the overhang sequence at the other end becomes the overhang sequence of initial oligonucleotide of the next cycle. In one embodiment, the three overhang sequences are all 2 to 6 nucleotides in length, and the encoding sequence of the incoming oligonucleotide is from 3 to 10 nucleotides in length, preferably 3 to 6 nucleotides in length. In a particular embodiment, the overhang sequences are all 2 nucleotides in length and the encoding sequence is 5 nucleotides in length.

In another embodiment, the incoming strand has a region at its 3' end which is complementary to the 3' end of the initial oligonucleotide, leaving overhangs at the 5' ends of both strands. The 5' ends can be filled in using, for example, a DNA polymerase, such as vent polymerase, resulting in a double-stranded elongated oligonucleotide. The bottom strand of this oligonucleotide can be removed, and additional sequence added to the 3' end of the top strand using the same method.

The encoding oligonucleotide tag is formed as the result of the successive addition of oligonucleotides that identify each successive building block. In one embodiment of the methods of the disclosure, the successive oligonucleotide tags may be coupled by enzymatic ligation to produce an encoding oligonucleotide. Enzyme-catalyzed ligation of oligonucleotides can be performed using any enzyme that has the ability to ligate nucleic acid fragments. Exemplary enzymes include ligases, polymerases, and topoisomerases. In specific embodiments of the disclosure, DNA ligase (EC 6.5.1.1), DNA polymerase (EC 2.7.7.7), RNA polymerase (EC 2.7.7.6) or topoisomerase (EC 5.99.1.2) are used to ligate the oligonucleotides. Enzymes contained in each EC class can be found, for example, as described in Bairoch (2000) Nucleic Acids Research 28:304-5.

In one embodiment, the oligonucleotides used in the methods of the disclosure are oligodeoxynucleotides and the enzyme used to catalyze the oligonucleotide ligation is DNA ligase. In order for ligation to occur in the presence of the ligase, i.e., for a phosphodiester bond to be formed between two oligonucleotides, one oligonucleotide must have a free 5' phosphate group and the other oligonucleotide must have a free 3' hydroxyl group. Exemplary DNA ligases that may be used in the methods of the disclosure include T4 DNA ligase, Taq DNA ligase, T4 RNA ligase, DNA ligase (E. coli) (all available from, for example, New England Biolabs, MA). One of skill in the art will understand that each enzyme used for ligation has optimal activity under specific conditions, e.g., temperature, buffer concentration, pH and time. Each of these conditions can be adjusted, for example, according to the manufacturer's instructions, to obtain optimal ligation of the oligonucleotide tags. The incoming oligonucleotide can be of any desirable length, e.g., at least three nucleobases in lengthThe incoming oligonucleotide can be 4 or more nucleobases in length. In one embodiment, the incoming oligonucleotide is from 3 to about 12 nucleobases in length. It is preferred that the oligonucleotides of the molecules in the libraries of the disclosure have a common terminal sequence which can serve as a primer for PCR, as is known in the art. Such a common terminal sequence can be incorporated as the terminal end of the incoming oligonucleotide added in the final cycle of the library synthesis, or it can be added following library synthesis, for example, using the enzymatic ligation methods disclosed herein.

In an embodiment, the process begins with a synthesized DNA sequence which is attached at its 5' end to a linker which terminates in an amino group. In step 1, this starting DNA sequence is ligated to an incoming DNA sequence in the presence of a splint DNA strand, DNA ligase and dithiothreitol in Tris buffer. This yields a tagged DNA sequence which can then be used directly in the next step or purified, for example, using HPLC or ethanol precipitation, before proceeding to the next step. In step 2 the tagged DNA is reacted with a protected activated amino acid, in this example, an Fmoc-protected amino acid fluoride, yielding a protected amino acid-DNA conjugate. In step 3, the protected amino acid-DNA conjugate is deprotected, for example, in the presence of piperidine, and the resulting deprotected conjugate is, optionally, purified, for example, by HPLC or ethanol precipitation. The deprotected conjugate is the product of the first synthesis cycle, and becomes the starting material for the second cycle, which adds a second amino acid residue to the free amino group of the deprotected conjugate.

In embodiments in which PCR is to be used to amplify the encoding oligonucleotides of selected molecules, the encoding oligonucleotides can include PCR primer binding site sequences. For example, a PCR primer binding site sequence can be included in the initial oligonucleotide prior to the first cycle of synthesis, or it can be included with the first incoming oligonucleotide. The encoding oligonucleotide can also include a capping PCR primer binding site sequence that follows the encoding sequences. The capping sequence can be ligated to the encoding oligonucleotide following the final cycle of library synthesis or it can be included in the incoming oligonucleotide of the final cycle. In cases in which the PCR primer binding site sequences are included in an incoming oligonucleotide, these incoming oligonucleotides can be significantly longer than the incoming oligonucleotides added in the other cycles, because they will include both an encoding sequence and a PCR primer binding site sequence.

In cases in which the capping sequence is added after the addition of the final building block and final incoming oligonucleotide, the synthesis of a library as set forth herein will include the step of ligating the capping sequence to the encoding oligonucleotide, such that the oligonucleotide portion of substantially all of the library members terminates in a sequence that includes a PCR primer binding site sequence. PCR primer binding site sequences suitable for use in the libraries of the disclosure are known in the art; suitable primers and methods are set forth, for example, in Innis et al., eds., PCR Protocols: A Guide to Methods and Applications, San Diego: Academic Press (1990).

In some embodiments, the capping sequence is added by ligation to the pooled fractions which are products of the final synthetic cycle. The capping sequence can be added using the enzymatic process used in the construction of the library.

As indicated above, the nucleotide sequence of the oligonucleotide tag as part of the methods of this disclosure, may be determined by the use of the polymerase chain reaction (PCR).

The oligonucleotide tag is comprised of polynucleotides that identify the building blocks that make up the functional moiety as described herein. The nucleic acid sequence of the oligonucleotide tag is determined by subjecting the oligonucleotide tag to a PCR reaction as follows. The appropriate sample is contacted with a PCR primer pair, each member of the pair having a preselected nucleotide sequence. The PCR primer pair is capable of initiating primer extension reactions by hybridizing to a PCR primer binding site on the encoding oligonucleotide tag. The PCR primer binding site can be designed into the encoding oligonucleotide tag. For example, a PCR primer binding site may be incorporated into the initial oligonucleotide tag and the second PCR primer binding site may be in the final oligonucleotide tag. Alternatively, the second PCR primer binding site may be incorporated into the capping sequence as described herein. In certain embodiments, the PCR primer binding site is at least about 5, 7, 10, 13, 15, 17, 20, 22, or 25 nucleotides in length.

The PCR reaction is performed by mixing the PCR primer pair, e.g., a predetermined amount thereof, with the nucleic acids of the encoding oligonucleotide tag, e.g., a predetermined amount thereof, in a PCR buffer to form a PCR reaction admixture. The admixture is thermocycled for a number of cycles, which is typically predetermined, sufficient for the formation of a PCR reaction product. A sufficient amount of product is one that can be isolated in a sufficient amount to allow for DNA sequence determination.

PCR is typically carried out by thermocycling i.e., repeatedly increasing and decreasing the temperature of a PCR reaction admixture within a temperature range whose lower limit is about 30° C. to about 55° C. and whose upper limit is about 90° C. to about 100° C. The increasing and decreasing can be continuous, or can be phasic with time periods of relative temperature stability at each of temperatures favoring polynucleotide synthesis, denaturation and hybridization.

The PCR reaction is performed using any suitable method. Generally it occurs in a buffered aqueous solution, i.e., a PCR buffer, preferably at a pH of 7-9. In some embodiments, a molar excess of the primer is present. A large molar excess can help improve the efficiency of the process.

The PCR buffer also contains the deoxyribonucleotide triphosphates (polynucleotide synthesis substrates) dATP, dCTP, dGTP, and dTTP and a polymerase, typically thermostable, all in adequate amounts for primer extension (polynucleotide synthesis) reaction. The resulting solution (PCR admixture) is heated to about 90° C-100° C for about 1 to 10 minutes, preferably from 1 to 4 minutes. After this heating period the solution is allowed to cool to 54° C, which is preferable for primer hybridization. The synthesis reaction may occur at a temperature ranging from room temperature up to a temperature above which the polymerase (inducing agent) no longer functions efficiently. Thus, for example, if DNA polymerase is used, the temperature is generally no greater than about 40° C. The thermocycling is repeated until the desired amount of PCR product is produced. An exemplary PCR buffer comprises the following reagents: 50 mM KCI; 10 mM Tris-HCl at pH 8.3; 1.5 mM MgCl.sub.2; 0.001% (wt/vol) gelatin, 200 µM dATP; 200 pM dTTP; 200 µM dCTP; 200 µM dGTP; and 2.5 units Thermus aquaticus (Taq) DNA polymerase I per 100 microliters of buffer.

Suitable enzymes for elongating the primer sequences include, for example, *E*. *coli* DNA polymerase I, Taq DNA polymerase, Klenow fragment of *E. coli* DNA polymerase I, T4 DNA polymerase, other available DNA polymerases, reverse transcriptase, and other enzymes, including heat-stable enzymes, which will facilitate combination of the nucleotides in the proper manner to form the primer extension products which are complementary to each nucleic acid strand. Generally, the synthesis will be initiated at the 3' end of each primer and proceed in the 5' direction along the template strand, until synthesis terminates, producing molecules of different lengths.

The newly synthesized DNA strand and its complementary strand form a double-stranded molecule which can be used in the succeeding steps of the analysis process.

PCR amplification methods are described in detail in U.S. Pat. Nos. 4,683,192, 4,683,202, 4,800,159, and 4,965,188, and at least in PCR Technology: Principles and Applications for DNA Amplification, H. Erlich, ed., Stockton Press, New York (1989); and PCR Protocols: A Guide to Methods and Applications, Innis et al., eds., Academic Press, San Diego, Calif. (1990).

The term "polynucleotide" as used herein in reference to primers, probes and nucleic acid fragments or segments to be synthesized by primer extension is defined as a molecule comprised of two or more deoxyribonucleotides, preferably more than three.

The term "primer" as used herein refers to a polynucleotide whether purified from a nucleic acid restriction digest or produced synthetically, which is capable of acting as a point of initiation of nucleic acid synthesis when placed under conditions in which synthesis of a primer extension product which is complementary to a nucleic acid strand is induced, i.e., in the presence of nucleotides and an agent for polymerization such as DNA polymerase, reverse transcriptase and the like, and at a suitable temperature and pH. The primer is preferably single stranded for maximum efficiency, but may alternatively be in double stranded form. If double stranded, the primer is first treated to separate it from its complementary strand before being used to prepare extension products. Preferably, the primer is a polydeoxyribonucleotide. The primer must be sufficiently long to prime the synthesis of extension products in the presence of the agents for polymerization. The exact lengths of the primers will depend on many factors, including temperature and the source of primer.

The primers used herein are selected to be "substantially" complementary to the different strands of each specific sequence to be amplified. This means that the primer must be sufficiently complementary so as to non-randomly hybridize with its respective template strand. Therefore, the primer sequence may or may not reflect the exact sequence of the template.

The polynucleotide primers can be prepared using any suitable method, such as, for example, the phosphotriester or phosphodiester methods described in Narang et al., (1979) Meth. Enzymol., 68:90; U.S. Pat. No. 4,356,270, U.S. Pat. No. 4,458,066, U.S. Pat. No. 4,416,988, U.S. Pat. No. 4,293,652; and Brown et al., (1979) Meth. Enzymol., 68:109.

Once the encoding oligonucleotide tag has been amplified, the sequence of the tag, and ultimately the composition of the selected molecule, can be determined using nucleic acid sequence analysis, a well known procedure for determining the sequence of nucleotide sequences. Nucleic acid sequence analysis is approached by a combination of (a) physiochemical techniques, based on the hybridization or denaturation of a probe strand plus its complementary target, and (b) enzymatic reactions with polymerases.

In one embodiment, the libraries of the disclosure include molecules comprising at least two functional moieties composed of building blocks, where the at least two functional moieties are operably linked to an encoding oligonucleotide. The nucleotide sequence of the encoding oligonucleotide is indicative of the building blocks present in the functional moiety, and in some embodiments, the connectivity or arrangement of the building blocks. The disclosure provides the advantage that the methodology used to construct the functional moieties and that used to construct the oligonucleotide tag can be performed in the same reaction medium, e.g., an aqueous medium, thus simplifying the method of preparing the library compared to methods in the prior art. In certain embodiments in which the oligonucleotide ligation steps and the building block addition steps can both be conducted in aqueous media, each reaction will have a different pH optimum. In these embodiments, the building block addition reaction can be conducted at a suitable pH and temperature in a suitable aqueous buffer. The buffer can then be exchanged for an aqueous buffer which provides a suitable pH for oligonucleotide ligation.

One advantage of the methods of the disclosure is that they can be used to prepare libraries comprising vast numbers of compounds. The ability to amplify encoding oligonucleotide sequences using known methods such as polymerase chain reaction ("PCR") means that selected molecules can be identified even if relatively few copies are recovered. This allows the practical use of very large libraries, which, as a consequence of their high degree of complexity, either comprise relatively few copies of any given library member, or require the use of very large volumes. For example, a library consisting of 108 unique structures in which each structure has 1×10¹² copies (about 1 picomole), requires about 100 L of solution at 1 µM effective concentration. For the same library, if each member is represented by 1,000,000 copies, the volume required is 100 µL at 1 µM effective concentration.

In one embodiment, the library comprises from about 10² to about 10¹⁵ copies of each library member. Given differences in efficiency of synthesis among the library members, it is possible that different library members will have different numbers of copies in any given library. Therefore, although the number of copies of each member theoretically present in the library may be the same, the actual number of copies of any given library member is independent of the number of copies of any other member. In some embodiments, the compound libraries of the disclosure include at least about 10⁵, 10⁶ or 10⁷ copies of each library member, or of substantially all library members. By "substantially all" library members is meant at least about 85% of the members of the library, preferably at least about 90%, and more preferably at least about 95% of the members of the library.

In some embodiments, the library includes a sufficient number of copies of each member that multiple rounds (i.e., two or more) of selection against a biological target can be performed, with sufficient quantities of binding molecules remaining following the final round of selection to enable amplification of the oligonucleotide tags of the remaining molecules and, therefore, identification of the functional moieties of the binding molecules.

In one embodiment, there is no amplification (synthesis of more copies) of the compounds remaining after any of the rounds of selection. Such amplification can lead to a mixture of compounds which is not consistent with the relative amounts of the compounds remaining after the selection. This inconsistency is due to the fact that certain compounds may be more readily synthesized that other compounds, and thus may be amplified in a manner which is not proportional to their presence following selection. For example, if compound 2 is more readily synthesized than compound 1, the amplification of the molecules remaining after Round 2 would result in a disproportionate amplification of compound 2 relative to compound 1, and a resulting mixture of compounds with a much lower (if any) enrichment of compound 1 relative to compound 2.

In one embodiment, the target is immobilized on a solid support by any known immobilization technique. The solid support can be, for example, a water-insoluble matrix contained within a chromatography column or a membrane. The encoded library can be applied to a water-insoluble matrix contained within a chromatography column. The column is then washed to remove non-specific binders. Target-bound compounds can then be dissociated by changing the pH, salt concentration, organic solvent concentration, or other methods, such as competition with a known ligand to the target.

In another embodiment, the target is free in solution and is incubated with the encoded library. Compounds which bind to the target (also referred to herein as "ligands") are selectively isolated by a size separation step such as gel filtration or ultrafiltration. In one embodiment, the mixture of encoded compounds and the target biomolecule are passed through a size exclusion chromatography column (gel filtration), which separates any ligand-target complexes from the unbound compounds. The ligand-target complexes are transferred to a reverse-phase chromatography column, which dissociates the ligands from the target. The dissociated ligands are then analyzed by PCR amplification and sequence analysis of the encoding oligonucleotides. This approach is particularly advantageous in situations where immobilization of the target may result in a loss of activity.

Once single ligands are identified by the above-described process, various levels of analysis can be applied to yield structure-activity relationship information and to guide further optimization of the affinity, specificity and bioactivity of the ligand. For ligands derived from the same scaffold, three-dimensional molecular modeling can be employed to identify significant structural features common to the ligands, thereby generating families of small-molecule ligands that presumably bind at a common site on the target biomolecule.

A variety of screening approaches can be used to obtain ligands that possess high affinity for one target but significantly weaker affinity for another closely related target. One screening strategy is to identify ligands for both biomolecules in parallel experiments and to subsequently eliminate common ligands by a cross-referencing comparison. In this method, ligands for each biomolecule can be separately identified as disclosed above. This method is compatible with both immobilized target biomolecules and target biomolecules free in solution.

For immobilized target biomolecules, another strategy is to add a preselection step that eliminates all ligands that bind to the non-target biomolecule from the library. For example, a first biomolecule can be contacted with an encoded library as described above. Compounds which do not bind to the first biomolecule are then separated from any first biomolecule-ligand complexes which form. The second biomolecule is then contacted with the compounds which did not bind to the first biomolecule. Compounds which bind to the second biomolecule can be identified as described above and have significantly greater affinity for the second biomolecule than to the first biomolecule.

A ligand for a biomolecule of unknown function which is identified by the method disclosed above can also be used to determine the biological function of the biomolecule. This is advantageous because although new gene sequences continue to be identified, the functions of the proteins encoded by these sequences and the validity of these proteins as targets for new drug discovery and development are difficult to determine and represent perhaps the most significant obstacle to applying genomic information to the treatment of disease. Target-specific ligands obtained through the process described in this disclosure can be effectively employed in whole cell biological assays or in appropriate animal models to understand both the function of the target protein and the validity of the target protein for therapeutic intervention. This approach can also confirm that the target is specifically amenable to small molecule drug discovery.

In one embodiment, one or more compounds within a library of the disclosure are identified as ligands for a particular biomolecule. These compounds can then be assessed in an in vitro assay for the ability to bind to the biomolecule. In some embodiments, the functional moieties of the binding compounds are synthesized without the oligonucleotide tag or linker moiety, and these functional moieties are assessed for the ability to bind to the biomolecule.

The effect of the binding of the functional moieties to the biomolecule on the function of the biomolecule can also be assessed using in vitro cell-free or cell-based assays. For a biomolecule having a known function, the assay can include a comparison of the activity of the biomolecule in the presence and absence of the ligand, for example, by direct measurement of the activity, such as enzymatic activity, or by an indirect measure, such as a cellular function that is influenced by the biomolecule. If the biomolecule is of unknown function, a cell which expresses the biomolecule can be contacted with the ligand and the effect of the ligand on the viability, function, phenotype, and/or gene expression of the cell is assessed. The in vitro assay can be, for example, a cell death assay, a cell proliferation assay or a viral replication assay. For example, if the biomolecule is a protein expressed by a virus, a cell infected with the virus can be contacted with a ligand for the protein. The affect of the binding of the ligand to the protein on viral viability can then be assessed.

A ligand identified by the method of the disclosure can also be assessed in an in vivo model or in a human. For example, the ligand can be evaluated in an animal or organism which produces the biomolecule. Any resulting change in the health status (e.g., disease progression) of the animal or organism can be determined.

For a biomolecule, such as a protein or a nucleic acid molecule, of unknown function, the effect of a ligand which binds to the biomolecule on a cell or organism which produces the biomolecule can provide information regarding the biological function of the biomolecule. For example, the observation that a particular cellular process is inhibited in the presence of the ligand indicates that the process depends, at least in part, on the function of the biomolecule.

Ligands identified using the methods of the disclosure can also be used as affinity reagents for the biomolecule to which they bind. In one embodiment, such ligands are used to effect affinity purification of the biomolecule, for example, via chromatography of a solution comprising the biomolecule using a solid phase to which one or more such ligands are attached.

This disclosure is further illustrated by the following examples which should not be construed as limiting. The contents of all references, patents and published patent applications cited throughout this application, as well as the Figures and the Sequence Listing, are hereby incorporated in reference.

### EXAMPLES

### Example 1. Preparation of the HUB

### Phosphoramidites linker for Oligonucleotide Synthesis

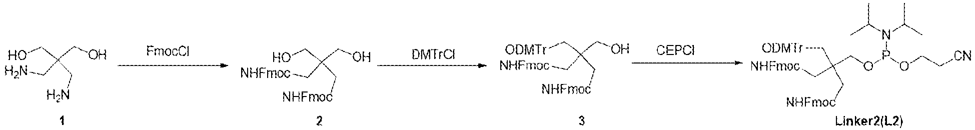

Preparation of (2): To the solution of 1 (13.4 g, 100 mmol, J. Org. Chem. 2004, 69, 2008-2016) in THF/sat.NaHCO₃(3:1, 800 mL) was added FmocCl (77.6 g, 300 mmol). The mixture was stirred at rom temperature for 3 h. The reaction mixture was diluted with H₂O, and then extracted with ethyl acetate. The combined organic phase was dried over anhydrous Na₂SO₄. After filtration, the filtrate was concentrated *in vacuo* to give crude product which was purified by silica gel column (PE:EA =3:1) to give **2** (46.3 g, 80.1% yield) as a white solid. ¹H-NMR (400 MHz, CDCl₃): δ ppm 7.78 (d, *J* = 7.52 Hz, 4H), 7.59 (d, *J* = 7.44 Hz, 4H), 7.42 (m, 4H), 7.33 (m, 4H), 5.79 (t, *J* = 6.72 Hz, 2 H), 4.45 (d, *J* = 6.76 Hz, 2H), 4.23 (t, *J* = 6.72 Hz, 2 H), 3.92 (t, *J* = 6.92 Hz, 2 H), 3.27 (d, *J* = 6.92 Hz, 4H), 3.02 (d, *J* = 6.80 Hz, 4H). ESI-LCMS: m/z 579 [M+H]⁺.

Preparation of (**3**): To a solution of **2** (50 g, 86.5 mmol) in dry DCM (500 mL) was added AgNOs (22 g, 129.4 mmol), Collidine (15 mL, 114 mmol) and DMTr-Cl (43.6 g, 128.6 mmol) at r.t.. The mixture was stirred at room temperature for overnight. The mixture was queeched with MeOH and diluted with DCM, washed with brine, dried over Na₂SO₄, the organic solvent was removed *in vacuo* to get crude product which was purified by a gel silica gel column (PE:EA = 5:1) to give the product as a white solid (54 g, 70.9% yield). ¹H-NMR (400 MHz, CDCl₃): δ ppm 7.84 (m, 4H), 7.49 (m, 20H), 7.22 (m, 2H), 6.89 (m, 4H), 5.11 (m, 2H), 4.39 (m, 4H), 4.22 (m, 2H), 3.94 (m, 1H), 3.75 (s, 6H), 3.72 (s, 3H), 3.45 (d, *J* = 5.42 Hz, 2H), 3.21 (m, 2H), 3.07 (s, 2H), 2.74 (m, 2H). ESI-LCMS: m/z 881 [M+H]⁺.

Preparation of (**Linker2**): To a solution of 3 (10 g, 11.4 mmol) in dry DCM (100 mL) was added DIEA(6.3 mL, 34.2), CEPCI(3.24 g, 13.7 mmol) at r.t.. The mixture was stirred at room temperature for 1 h. The mixture was queeched with sat.NaHCOs, washed with brine, dried over Na₂SO₄, the organic solvent was removed *in vacuo* to get crude product which was purified by a gel silica gel column (PE:EA = 8:1) to give the product as a white solid (9.5 g, 77% yield). ¹H-NMR (400 MHz, CDCl₃): δ ppm 7.78 (m, 4H), 7.59 (m, 4H), 7.36 (m, 17H), 6.84 (m, 4H), 5.47 (m, 2H), 4.38 (m, 4H), 4.22 (m, 2H), 4.00 (m, 1H), 3.81 (m, 1H), 3.58 (m, 11H), 3.28 (m, 3H), 2.48 (t, *J* = 6.40 Hz, 2H), 1.19 (m, 12H). ³¹P-NMR (400 MHz, CDCl₃): 148.3

Sequences of oligonucleotide (top strand: SEQ ID NO.: 1; bottom strand: SEQ ID NO.: 2) used are shown in formula (III) below. Oligonucleotide synthesis was performed on an ABI 394 synthesizer according to the protocol supplied by the manufacturer using a 0.02M iodine solution. The oligonucleotide was purified by reverse phase HPLC (theoretical mass: 4921.8; observed mass: 4921.6). 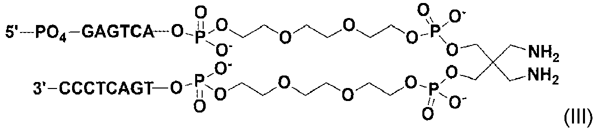 (HUB structure, abbreviated as:

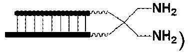

Where single letter codes (A, T, C, G) for deoxyribonucleotides:
A = adenosine
T = thymidine
C = cytidine
G = guanosine

**General Procedures for workup and reaction monitoring in on-DNA reactions and ligations:** The standard procedure for ethanol precipitations following enzymatic tag ligation or small molecule reactions involved addition of 10% by volume 5M NaCl (aq), 200-300% by volume cold ethanol, and then cooling to -20 °C for at least one hour. The precipitated material was then isolated as a pellet by centrifugation and subsequent removal of the supernatant. The concentration of all stock solutions containing HUB were determined by measuring OD absorbance at 260 nm. During library production, for quality control of tag ligations, two wells per plate (typically A1 and B2) were analyzed by gel electrophoresis on 3% agarose gel plates and HPLC/ESI-MS; for quality control of chemical reactions, two wells per plate (typically A1 and B2) were analyzed by LC-MS. For single chemical reaction development, the progress of reaction was monitored and analyzed by LC-MS.

### Example 2. Application of amide formation reactions to functional moiety

Installation of chemical spacer (Synthesis of AOP-HUB, the structure of AOP-HUB is shown in formula (IV) below):

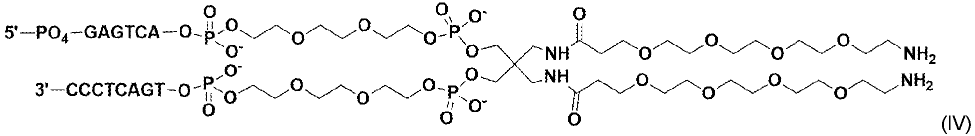

HUB (20 µmol) was dissolved in pH 9.4 borate buffer (250 mM, 20 mL) to make a 1 mM solution. Stocks of 80 equivalents of N-Fmoc-15-amino-4,7,10,13-tetraoxadodecanoic acid (AOP) in 0.4 M DMA stock (4.0 mL, 1.6 mmol), 80 equivalents of HATU in 0.4 M DMA stock (4.0 mL, 1.6 mmol), and 80 equivalents of DIPEA in 0.4 M DMA stock (4.0 mL, 1.6 mmol) were pre-cooled at 4 degree for 5 min, and then mixed together. After vortex, the "premix" stock was cooled at 4 degree for 5 min, then transferred to DNA solution, proceed at room temperature overnight. LCMS was used to show the progress of reactions.

Following acylation, a 0.1 × volume of 5 M aqueous NaCl (3.2 mL) and a 3× volume of ethanol (105.6 mL) was added and the mixture was allowed to stand at -20° C for at least one hour. The mixture was then subjected to centrifuge (12,000 rpm, 30 minutes) to give a white pellet. The solid was dissolved in 10 mL of water and purified by Reverse Phase HPLC with a Waters XBridge Prep Shield RP18 (5 µm, 10×150 mm) column. The purified material was concentrated by lyophilization and the resulting residue was then dissolved in 10 mL of water. A 0.1× volume of piperidine (1mL) was added to the solution and the mixture was reacted for 1 hour at room temperature. The product was then purified by ethanol precipitation as described above and isolated by centrifugation. The deFmoc reaction was clean and the product was ready for following use. The resulting pellet was dried by lyophilization to give purified AOP-HUB (9.3 **µ**mol as determined by OD, theoretical mass: 5416.1; observed mass: 5417.2).

### General procedure for amide formation of DNA-linker containing an amine with acid building blocks:

DNA (20 nmol) was dissolved in sodium borate buffer (250 mM, pH 9.4, 20 **µL**) to make 1 mM solution. 80 equivalents of acid building block (8.0 **µL**, 1.6 **µ**mοl) in 0.2 M DMA stock, 80 equivalents of HATU in 0.4 M DMA stock (4.0 **µL**, 1.6 **µ**mol), and 80 equivalents of DIPEA in 0.4 M DMA stock (4.0 **µL** 1.6 **µ**mol) were cooled at 4 degree for 5 min, and then mixed together. After vortex, the "premix" stock was cooled at 4 degree for 5 min, then transferred to DNA solution. After vortex, the reaction was allowed to proceed at room temperature overnight. After analyzing by LCMS, the product was then purified by ethanol precipitation as described above and isolated by centrifugation.

### Example 3. Application of reductive amination reactions to functional moiety

AOP-HUB was prepared following the same procedure as described above.

### General procedure for reductive amination of DNA-linker containing an secondary amine with aldehyde building blocks:

AOP-HUB (20 nmol) was dissolved in sodium borate buffer (250 mM, pH 9.4, 20 µL) to make 1 mM solution. 80 equivalents of amino acid containing Fmoc protected secondary amine (8.0 µL, 1.6 µmol) in 0.2 M DMA stock, 80 equivalents of HATU in 0.4 M DMA stock (4.0 µL, 1.6 and 80 equivalents of DIPEA in 0.4 M DMA stock (4.0 µL, 1.6 µmol) were cooled at 0 degree for 5 min, and then mixed together. After vortex, the "premix" stock was cooled at 4 degree for 5 min, then transferred to DNA solution, vortex again. The reaction was allowed to proceed at room temperature overnight. After analyzing by LCMS, the product was then purified by ethanol precipitation as described above and isolated by centrifugation. De-Fmoc procedure is the same as described above.

DNA-linker containing a secondary amine (20 nmol) was dissolved in borate buffer (250 mM, pH 9.4, 20 µL) to make 1 mM solution. 100 equivalents of aldehyde in 0.2 M DMA (10 uL, 2.0 µmol) was then added. The resulting mixture was allowed to sit for 30 min and then 300 equivalents of NaBH₄ (15 µL, 400 mM in H₂O, fresh-made stock) was added. The reaction was allowed to proceed at room temperature overnight. After analyzing by LCMS, the product was then purified by ethanol precipitation as described above and isolated by centrifugation.

### Example 4. Application of azide-alkyne cycloaddition reactions to functional moiety

AOP-HUB was prepared following the same procedure as described above.

### General procedure for azide-alkyne cycloaddition of DNA-linker containing an azide with alkyne building blocks:

AOP-HUB (20 nmol) was dissolved in pH 9.4 borate buffer (250 mM, 20 µL) to make 1 mM solution. 80 equivalents of azidoacetic acid in 0.2 M fresh made DMA solution (8.0 µL, 1.6 80 equivalents of HATU in 0.4 M DMA stock (4.0 µL, 1.6 and 80 equivalents of DIPEA in 0.4 M DMA stock (4.0 µL, 1.6 µmol) were cooled at 4 degree for 5 min, and then mixed together. After vortex, the "premix" stock was cooled at 4 degree for 5 min, then transferred to DNA solution. After vortex, the reaction was allowed to proceed at room temperature overnight. After completion of the material, the product was then purified by ethanol precipitation as described above and isolated by centrifugation. The resulting DNA pellet was dried by lyophilization.

To a 1 mM solution of the above product (20 nmol) in pH 9.4 borate buffer (20 µL) was added 4 equivalents of CuSO₄ in 0.2 M fresh made aqueous solution (0.4 µL, 80 nmol), 10 equivalents of ascorbic acid in 0.2 M fresh made aqueous solution (1 µL, 200 nmol), followed by 30 equivalents of TBTA in 0.2 M fresh made DMA solution (3 µL, 600 nmol). After vortex, the reaction mixture was allowed to proceed at room temperature overnight. After completion of the material monitored by LCMS, the product was then purified by ethanol precipitation as described above and isolated by centrifugation. The resulting DNA pellet was dried by lyophilization.

### Example 5. Application of Suzuki cross coupling reactions to functional moiety

AOP-HUB was prepared following the same procedure as described above.

### General procedure for Suzuki cross coupling of DNA-linker containing an aryl halide with borate ester or boronic acid building blocks:

AOP-HUB (20 nmol) was dissolved in pH 9.4 borate buffer (250 mM, 20 µL) to make 1 mM solution. 80 equivalents of aryl halide (normally aryl bromide or iodide) in 0.2 M DMA stock (8.0 µL, 1.6 80 equivalents of HATU in 0.4 M DMA stock (4.0 µL, 1.6 µmol) and 80 equivalents of DIPEA in 0.4 M DMA stock (4.0 µL, 1.6 µmol) were cooled at 4 degree for 5 min, and then mixed together. After vortex, the "premix" stock was cooled at 4 degree for 5 min, then transferred to DNA solution. After vortex, the reaction was allowed to proceed at room temperature overnight. After completion of the material monitored by LCMS, the product was then purified by ethanol precipitation as described above and isolated by centrifugation. The resulting DNA pellet was dried by lyophilization.

To a 1mM solution of the above product (20 nmol) in water (20 µL) was added 120 equivalents of borate ester or boronic acid building blocks in 0.6 M DMA stock (2.4 µmol, 4 uL), 150 equivalents of KOH (1 M in water, 3 uL), followed by 0.67 equivalents of POPd ([(t-Bu)2P-(OH)]2PdCl2, 20 mM in DMA, 0.67 µL) and 1.33 equivalents of ligand sodium 2'-(dicyclohexylphosphino)-2,6-dimethoxy-[1,1'-biphenyl]-3-sulfonate (20 mM in DMA, 1.33 µL). After vortex, the reaction was allowed to proceed at 95 degree for 2 hours. After completion of the material monitored by LCMS, the product was then purified by ethanol precipitation as described above and isolated by centrifugation. The resulting DNA pellet was dried by lyophilization.

### Example 6. Synthesis and characterization of a DNA-encoded library in the order of 10⁷ members

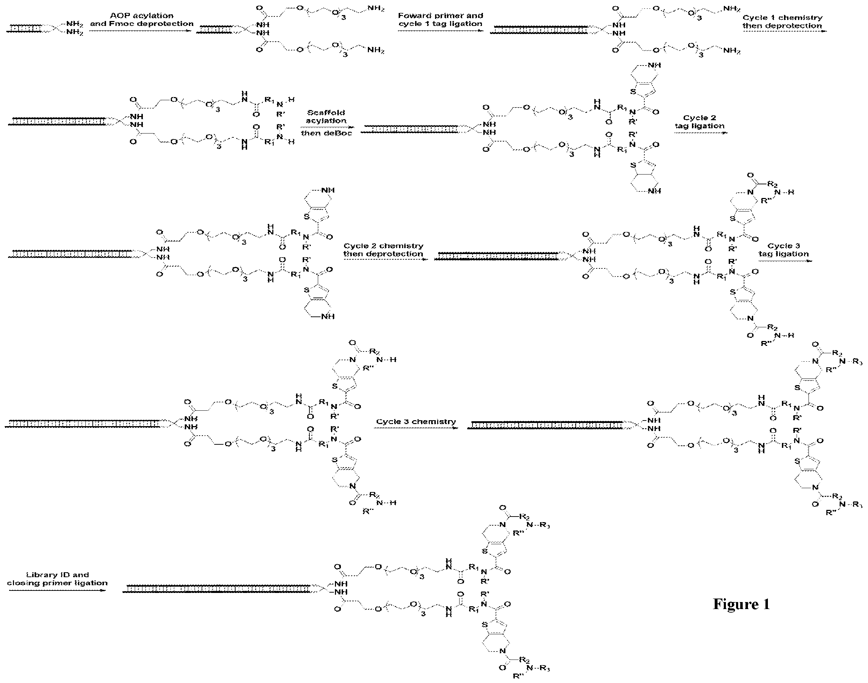

An exemplary synthesis scheme is shown in Figure 1, in which R1, R2 and R3 each can be any building block of interest. In one exemplary library, the following building blocks are used:
R1: 96 amino acids +1 Null
R2: 99 amino acids +1 Null
R3: 196 (acids, aldehyde, sulfonyl chlorides, isocyanates) + 4 Null

The synthesis of such library was accomplished using the following steps and reagents:
AOP-HUB was prepared following the same procedure as described above.
Building blocks, oligonucleotide tags and their correspondence were shown in the following each cycle.

The sequence of forward primer (with 5'-PHO) is shown below:
5'PHO-GACCGAAGGTTG-3' (SEQ ID NO.: 3)
3'-GGCTGGCTTCCA-5'-PHO (SEQ ID NO.: 4)

The 10× ligation buffer stock used in ligation reactions was composed as follows: 500 mM Tris pH 7.5, 500 mM NaCl, 100 mM MgCl2, 100 mM DTT, 25 mM ATP.

T4 DNA Ligase was obtained through escherichia coli expression, and the activity was tested before use (30 U/µL).

### Step 1: Cycle 1 synthesis:

### Procedures:

Cycle 1 incorporated 96 Fmoc-protected amino acids and 1 null control. Compound HUB-AOP (8.73 µmοl total) was split to 97 aliquots. To each PCR tube (2 mL) was added 90 µL of a 1 mM solution of HUB-AOP in water (90 nmol DNA in each tube), 90 µL of a 1 mM solution of forward primer, 90 µL of a 1 mM solution of cycle 1 tags, 72 µL of 10× ligase buffer, 8.2 uL of T4 DNA ligase and 9.8 µL of water. After vortex, the resulting solutions were incubated at 16° C for 16 hours. The reaction process was detected by gel electrophoresis.

After the ligation reaction, 36 µL of 5 M aqueous NaCl was added directly to each tube, followed by 1188 µL ethanol, and held at -20° C for 1 hour. The plates were centrifugated for 30 minutes at 12,000 rpm under centrifuge to give a white pellet, and the supernatant was removed. The isolated pellet was redissolved in 90 uL water and lyophilized.

Each of the DNA pellets in tubes was then dissolved in sodium borate buffer (90 µL, 150 mM, pH 9.4) to make a concentration of 1 mM solution. 80 equivalents of building block precursors in 0.2 M DMA stock (36 µL, 7.2 80 equivalents of the HATU in 0.4 M DMA stock (18 µL, 7.2 µmol) and 80 equivalents of the DIPEA in 0.4 M DMA stock (18 µL, 7.2 µmol) were cooled at 4 degree for 5 min, and then mixed together to react for 5 minutes at 4 °C. After that, the mixture was added to DNA samples and the solutions reacted for 16 hours at room temperature. In this cycle, 1 null control experiment was designed (addition of reagents but no addition of building block).

Following acylation, a 0.1 × volume of 5 M aqueous NaCl and a 3× volume of ethanol was added and the mixture was allowed to stand at -20° C for at least one hour. The mixture was then centrifuged for 30 minutes at 12,000 rpm. The supernatant was removed after centrifugation, and the pellet was redissolved in 90 uL H₂O. A 0.1× volume of piperidine was added to the solution and the mixture was reacted 1 h at room temperature. The product was then purified by ethanol precipitation as described above and isolated by centrifugation. The resulting DNA pellet was redissolved in H₂O and dried by lyophilization, yielding 8.7 µmol as determined by OD.

**Table 1. Cycle 1 building blocks and oligonucleotide tags:**

| Entry | Building blocks | Oligonucleotide tags (5' to 3', with 5'-PHO) | |
|---|---|---|---|
| | | Top strand sequence | Bottom strand sequence |
| 1 | 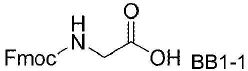 | ATCAGAACTAGAG (SEQ ID NO.: 5) | CTAGTTCTGATCA (SEQ ID NO.: 6) |
| 2 | 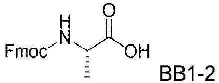 | GAGGAACGCTAAG (SEQ ID NO.: 7) | TAGCGTTCCTCCA (SEQ ID NO.: 8) |
| 3 | 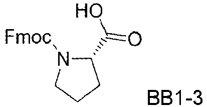 | CGCGAATGATGAG (SEQ ID NO.: 9) | CATCATTCGCGCA (SEQ ID NO.: 10) |
| 4 | 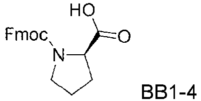 | TTAACGCGCTTAG (SEQ ID NO.: 11) | AAGCGCGTTAACA (SEQ ID NO.: 12) |
| 5 | 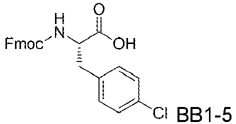 | TGTGCGTGTATAG (SEQ ID NO.: 13) | ATACACGCACACA (SEQ ID NO.: 14) |
| 6 | 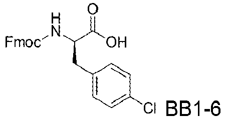 | ATGAACGGCGAAG (SEQ ID NO.: 15) | TCGCCGTTCATCA (SEQ ID NO.: 16) |
| 7 | 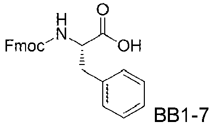 | CAATCTCAGAGAG (SEQ ID NO.: 17) | CTCTGAGATTGCA (SEQ ID NO.: 18) |
| 8 | 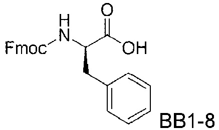 | CTGCCGATCGAAG (SEQ ID NO.: 19) | TCGATCGGCAGCA (SEQ ID NO.: 20) |
| 9 | 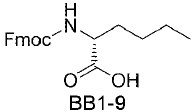 | GACACCTAGCGAG (SEQ ID NO.: 21) | CGCTAGGTGTCCA (SEQ ID NO.: 22) |
| 10 | 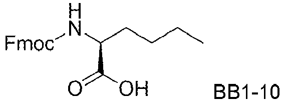 | TGACCGTTCCGAG (SEQ ID NO.: 23) | CGGAACGGTCACA (SEQ ID NO.: 24) |
| 11 | 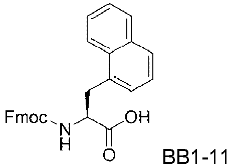 | TCCAGAGCAGTAG (SEQ ID NO.: 25) | ACTGCTCTGGACA (SEQ ID NO.: 26) |
| 12 | 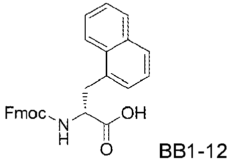 | CAGACGCCTCAAG (SEQ ID NO.: 27) | TGAGGCGTCTGCA (SEQ ID NO.: 28) |
| 13 | 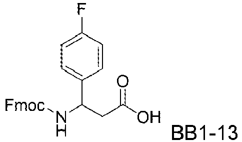 | AGGAGAAGTTCAG (SEQ ID NO.: 29) | GAACTTCTCCTCA (SEQ ID NO.: 30) |
| 14 | 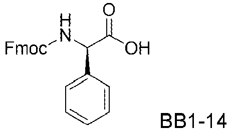 | TTCACCGTTCGAG (SEQ ID NO.: 31) | CGAACGGTGAACA (SEQ ID NO.: 32) |
| 15 | 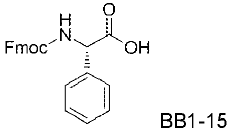 | ATGATTCGAGAAG (SEQ ID NO.: 33) | TCTCGAATCATCA (SEQ ID NO.: 34) |
| 16 | 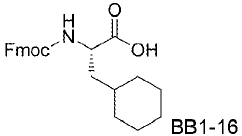 | AGATGGATGTTAG (SEQ ID NO.: 35) | AACATCCATCTCA (SEQ ID NO.: 36) |
| 17 | 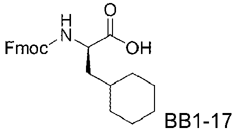 | ACAGTCACTACAG (SEQ ID NO.: 37) | GTAGTGACTGTCA (SEQ ID NO.: 38) |
| 18 | 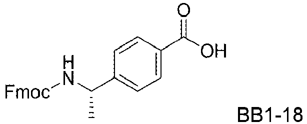 | ATCGGAATAGTAG (SEQ ID NO.: 39) | ACTATTCCGATCA (SEQ ID NO.: 40) |
| 19 | 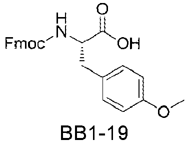 | AGTCGTATTATAG (SEQ ID NO.: 41) | ATAATACGACTCA (SEQ ID NO.: 42) |
| 20 | 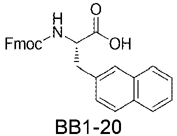 | GAGTGTGGTAGAG (SEQ ID NO.: 43) | CTACCACACTCCA (SEQ ID NO.: 44) |
| 21 | 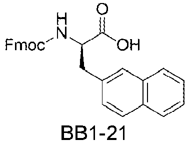 | CAGGTAATGTTAG (SEQ ID NO.: 45) | AACATTACCTGCA (SEQ ID NO.: 46) |
| 22 | 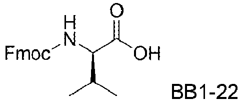 | AAGGTTACGTCAG (SEQ ID NO.: 47) | GACGTAACCTTCA (SEQ ID NO.: 48) |
| 23 | 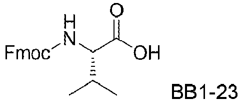 | TTGTATGAATAAG (SEQ ID NO.: 49) | TATTCATACAACA (SEQ ID NO.: 50) |
| 24 | 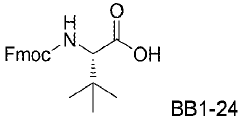 | ACCATAACCTTAG (SEQ ID NO.: 51) | AAGGTTATGGTCA (SEQ ID NO.: 52) |
| 25 | 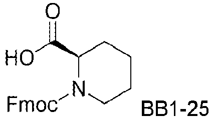 | CTACAGGTAATAG (SEQ ID NO.: 53) | ATTACCTGTAGCA (SEQ ID NO.: 54) |
| 26 | 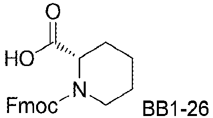 | AGGCTGCTAGAAG (SEQ ID NO.: 55) | TCTAGCAGCCTCA (SEQ ID NO.: 56) |
| 27 | 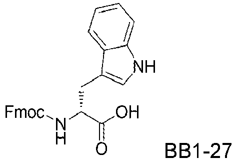 | CAATTACGATAAG (SEQ ID NO.: 57) | TATCGTAATTGCA (SEQ ID NO.: 58) |
| 28 | 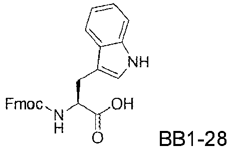 | ACGACGATCCAAG (SEQ ID NO.: 59) | TGGATCGTCGTCA (SEQ ID NO.: 60) |
| 29 | 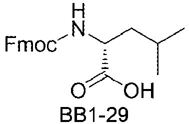 | TTAACAGCTTAAG (SEQ ID NO.: 61) | TAAGCTGTTAACA (SEQ ID NO.: 62) |
| 30 | 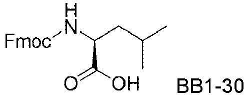 | ATGATACATGAAG (SEQ ID NO.: 63) | TCATGTATCATCA (SEQ ID NO.: 64) |
| 31 | 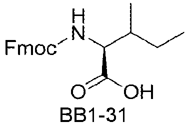 | AGCACTAACATAG (SEQ ID NO.: 65) | ATGTTAGTGCTCA (SEQ ID NO.: 66) |
| 32 | 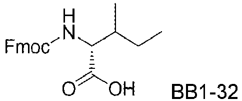 | TTCGCCTGCTTAG (SEQ ID NO.: 67) | AAGCAGGCGAACA (SEQ ID NO.: 68) |
| 33 | 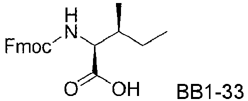 | ACATAGTCCGAAG (SEQ ID NO.: 69) | TCGGACTATGTCA (SEQ ID NO.: 70) |
| 34 | 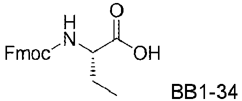 | TTACGAGAGCAAG (SEQ ID NO.: 71) | TGCTCTCGTAACA (SEQ ID NO.: 72) |
| 35 | 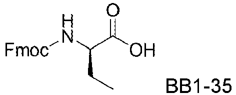 | TTCTGGCTCGTAG (SEQ ID NO.: 73) | ACGAGCCAGAACA (SEQ ID NO.: 74) |
| 36 | 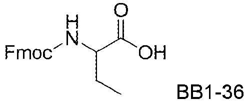 | TTAGACTCGAGAG (SEQ ID NO.: 75) | CTCGAGTCTAACA (SEQ ID NO.: 76) |
| 37 | 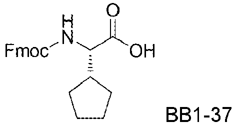 | GAAGGCATCACAG (SEQ ID NO.: 77) | GTGATGCCTTCCA (SEQ ID NO.: 78) |
| 38 | 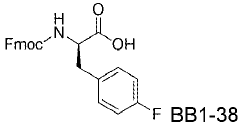 | GACCAACGAGTAG (SEQ ID NO.: 79) | ACTCGTTGGTCCA (SEQ ID NO.: 80) |
| 39 | 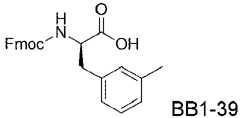 | CGGCGTTCCGCAG (SEQ ID NO.: 81) | GCGGAACGCCGCA (SEQ ID NO.: 82) |
| 40 | 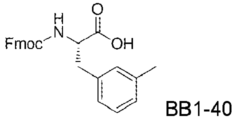 | AATAATCAACGAG (SEQ ID NO.: 83) | CGTTGATTATTCA (SEQ ID NO.: 84) |
| 41 | 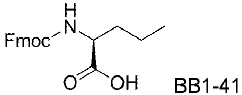 | AATGGTTAAGAAG (SEQ ID NO.: 85) | TCTTAACCATTCA (SEQ ID NO.: 86) |
| 42 | 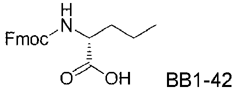 | TTACTCTAGGCAG (SEQ ID NO.: 87) | GCCTAGAGTAACA (SEQ ID NO.: 88) |
| 43 | 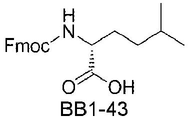 | AACAGCACGTGAG (SEQ ID NO.: 89) | CACGTGCTGTTCA (SEQ ID NO.: 90) |
| 44 | 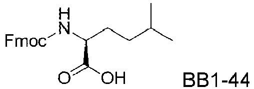 | GCACAACTGGTAG (SEQ ID NO.: 91) | ACCAGTTGTGCCA (SEQ ID NO.: 92) |
| 45 | 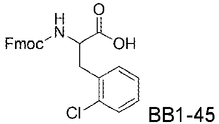 | GCTACAATTGAAG (SEQ ID NO.: 93) | TCAATTGTAGCCA (SEQ ID NO.: 94) |
| 46 | 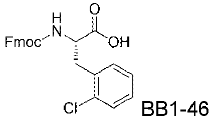 | CTCGTGTGCTAAG (SEQ ID NO.: 95) | TAGCACACGAGCA (SEQ ID NO.: 96) |
| 47 | 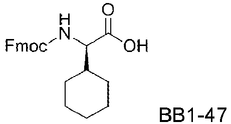 | ATTCCGTCCGCAG (SEQ ID NO.: 97) | GCGGACGGAATCA (SEQ ID NO.: 98) |
| 48 | 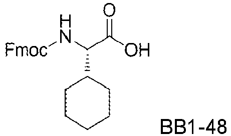 | GCCGTTGTCGCAG (SEQ ID NO.: 99) | GCGACAACGGCCA (SEQ ID NO.: 100) |
| 49 | 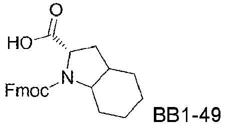 | GTACGAGCCGCAG (SEQ ID NO.: 101) | GCGGCTCGTACCA (SEQ ID NO.: 102) |
| 50 | 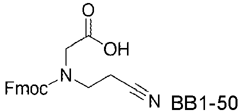 | TCAATCCACGTAG (SEQ ID NO.: 103) | ACGTGGATTGACA (SEQ ID NO.: 104) |
| 51 | 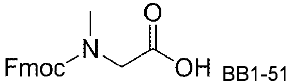 | AGTCACCTTGTAG (SEQ ID NO.: 105) | ACAAGGTGACTCA (SEQ ID NO.: 106) |
| 52 | 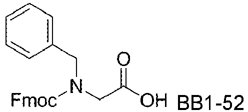 | TTAGTGGCTTAAG (SEQ ID NO.: 107) | TAAGCCACTAACA (SEQ ID NO.: 108) |
| 53 | 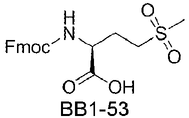 | TGGCAATGTATAG (SEQ ID NO.: 109) | ATACATTGCCACA (SEQ ID NO.: 110) |
| 54 | 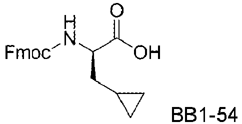 | AATTACGGCAGAG (SEQ ID NO.: 111) | CTGCCGTAATTCA (SEQ ID NO.: 112) |
| 55 | 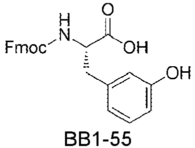 | TGGAGCGGAACAG (SEQ ID NO.: 113) | GTTCCGCTCCACA (SEQ ID NO.: 114) |
| 56 | 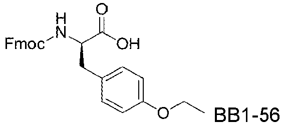 | GAGTCAGGACGAG (SEQ ID NO.: 115) | CGTCCTGACTCCA (SEQ ID NO.: 116) |
| 57 | 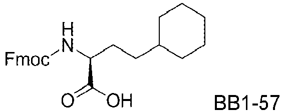 | ACAAGGTATGAAG (SEQ ID NO.: 117) | TCATACCTTGTCA (SEQ ID NO.: 118) |
| 58 | 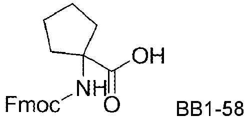 | TTGGAGATTAGAG (SEQ ID NO.: 119) | CTAATCTCCAACA (SEQ ID NO.: 120) |
| 59 | 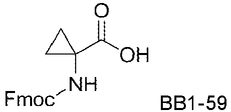 | TTAAGCGTAAGAG (SEQ ID NO.: 121) | CTTACGCTTAACA (SEQ ID NO.: 122) |
| 60 | 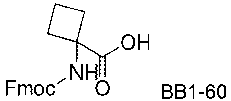 | GTAGTTGATTAAG (SEQ ID NO.: 123) | TAATCAACTACCA (SEQ ID NO.: 124) |
| 61 | 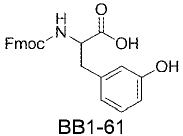 | AGTCCTATCACAG (SEQ ID NO.: 125) | GTGATAGGACTCA (SEQ ID NO.: 126) |
| 62 | 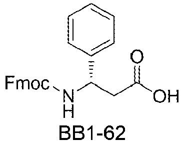 | GACCGTTCTGTAG (SEQ ID NO.: 127) | ACAGAACGGTCCA (SEQ ID NO.: 128) |
| 63 | 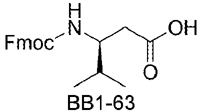 | GAAGGAACTAGAG (SEQ ID NO.: 129) | CTAGTTCCTTCCA (SEQ ID NO.: 130) |
| 64 | 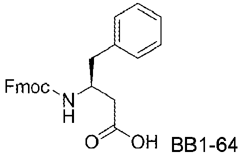 | GTTGTAATTGTAG (SEQ ID NO.: 131) | ACAATTACAACCA (SEQ ID NO.: 132) |
| 65 | 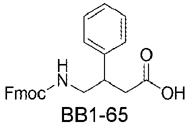 | CCGATGTCAGTAG (SEQ ID NO.: 133) | ACTGACATCGGCA (SEQ ID NO.: 134) |
| 66 | 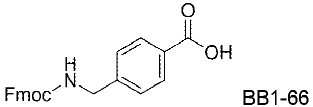 | ATCACTACCGAAG (SEQ ID NO.: 135) | TCGGTAGTGATCA (SEQ ID NO.: 136) |
| 67 | 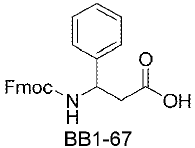 | CAACGCCGATTAG (SEQ ID NO.: 137) | AATCGGCGTTGCA (SEQ ID NO.: 138) |
| 68 | 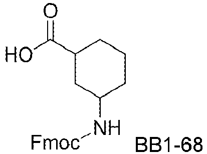 | GAACCGTCTAGAG (SEQ ID NO.: 139) | CTAGACGGTTCCA (SEQ ID NO.: 140) |
| 69 | 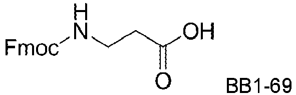 | GTAATCCGGCTAG (SEQ ID NO.: 141) | AGCCGGATTACCA (SEQ ID NO.: 142) |
| 70 | 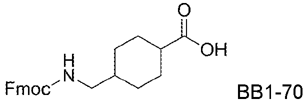 | TTCCGAGTTCGAG (SEQ ID NO.: 143) | CGAACTCGGAACA (SEQ ID NO.: 144) |
| 71 | 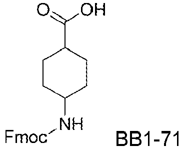 | CCTCACCAAGCAG (SEQ ID NO.: 145) | GCTTGGTGAGGCA (SEQ ID NO.: 146) |
| 72 | 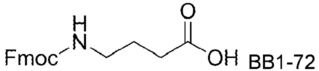 | AATCCACCATTAG (SEQ ID NO.: 147) | AATGGTGGATTCA (SEQ ID NO.: 148) |
| 73 | 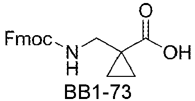 | TTCCACTAGTTAG (SEQ ID NO.: 149) | AACTAGTGGAACA (SEQ ID NO.: 150) |
| 74 | 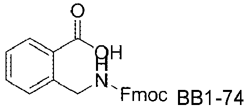 | TGGACTGCTTCAG (SEQ ID NO.: 151) | GAAGCAGTCCACA (SEQ ID NO.: 152) |
| 75 | 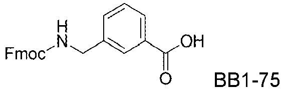 | CAAGTAAGTAGAG (SEQ ID NO.: 153) | CTACTTACTTGCA (SEQ ID NO.: 154) |
| 76 | 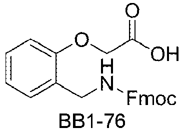 | GCGCACAGCTGAG (SEQ ID NO.: 155) | CAGCTGTGCGCCA (SEQ ID NO.: 156) |
| 77 | 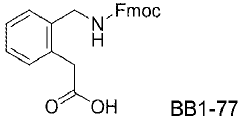 | CCTGTCACATTAG (SEQ ID NO.: 157) | AATGTGACAGGCA (SEQ ID NO.: 158) |
| 78 | 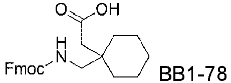 | CTCGGTCCGCGAG (SEQ ID NO.: 159) | CGCGGACCGAGCA (SEQ ID NO.: 160) |
| 79 | 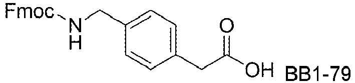 | GTATCCAGTTAAG (SEQ ID NO.: 161) | TAACTGGATACCA (SEQ ID NO.: 162) |
| 80 | 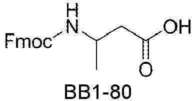 | CACCGTAAGACAG (SEQ ID NO.: 163) | GTCTTACGGTGCA (SEQ ID NO.: 164) |
| 81 | 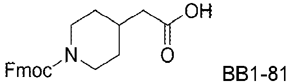 | CTACTGTCAATAG (SEQ ID NO.: 165) | ATTGACAGTAGCA (SEQ ID NO.: 166) |
| 82 | 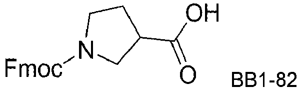 | GCTTCACCGTTAG (SEQ ID NO.: 167) | AACGGTGAAGCCA (SEQ ID NO.: 168) |
| 83 | 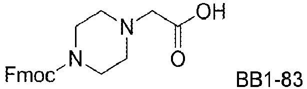 | TTATGGAATTCAG (SEQ ID NO.: 169) | GAATTCCATAACA (SEQ ID NO.: 170) |
| 84 | 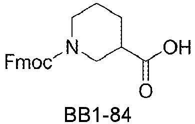 | GTAGCCGCCTCAG (SEQ ID NO.: 171) | GAGGCGGCTACCA (SEQ ID NO.: 172) |
| 85 | 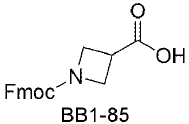 | TCTGTAGTGCTAG (SEQ ID NO.: 173) | AGCACTACAGACA (SEQ ID NO.: 174) |
| 86 | 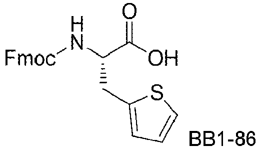 | AGTGATACTATAG (SEQ ID NO.: 175) | ATAGTATCACTCA (SEQ ID NO.: 176) |
| 87 | 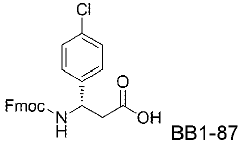 | TTGCTCCATCGAG (SEQ ID NO.: 177) | CGATGGAGCAACA (SEQ ID NO.: 178) |
| 88 | 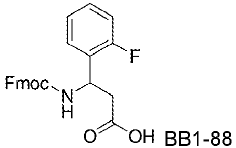 | AATCTCGGAGTAG (SEQ ID NO.: 179) | ACTCCGAGATTCA (SEQ ID NO.: 180) |
| 89 | 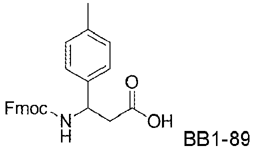 | TCACTTACCTCAG (SEQ ID NO.: 181) | GAGGTAAGTGACA (SEQ ID NO.: 182) |
| 90 | 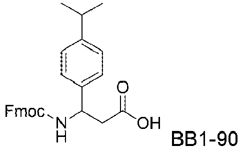 | CATGTCGGCGAAG (SEQ ID NO.: 183) | TCGCCGACATGCA (SEQ ID NO.: 184) |
| 91 | 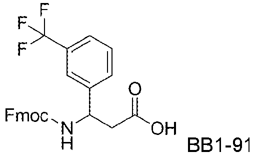 | CCTGGAGCCGCAG (SEQ ID NO.: 185) | GCGGCTCCAGGCA (SEQ ID NO.: 186) |
| 92 | 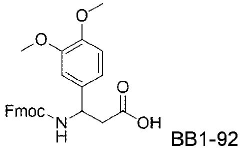 | AATGCGTGGTTAG (SEQ ID NO.: 187) | AACCACGCATTCA (SEQ ID NO.: 188) |
| 93 | 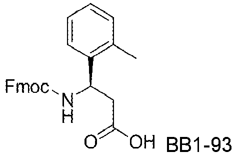 | GACACGGTCGTAG (SEQ ID NO.: 189) | ACGACCGTGTCCA (SEQ ID NO.: 190) |
| 94 | 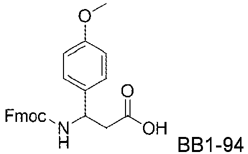 | TGTTCTCGAGCAG (SEQ ID NO.: 191) | GCTCGAGAACACA (SEQ ID NO.: 192) |
| 95 | 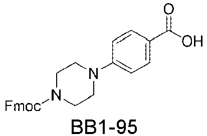 | CGAGTCCAGCAAG (SEQ ID NO.: 193) | TGCTGGACTCGCA (SEQ ID NO.: 194) |
| 96 | 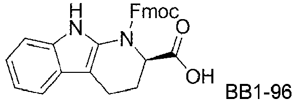 | TATACCAATACAG (SEQ ID NO.: 195) | GTATTGGTATACA (SEQ ID NO.: 196) |
| 97 | null | CCGCGTGGAAGAG (SEQ ID NO.: 197) | CTTCCACGCGGCA (SEQ ID NO.: 198) |

### Step 2: Installation of scaffold:

### Procedures:

Each of the DNA pellets (90 nmol for each tube) in tubes was then redissolved in sodium borate buffer (90 µL, 150 mM, pH 9.4) to make 1 mM stock. 80 equivalents of scaffold in 0.2 M DMA stock (36 µL, 7.2 80 equivalents of HATU in 0.4 M DMA stock (18 µL, 7.2 µmol), 80 equivalents of DIPEA in 0.4 M DMA stock (18 µL, 7.2 µmol ) were cooled at 4 degree for 30 minutes, and then mixed together to react for 5 minutes at 4 °C. After that, the mixture was added to each tube containing DNA samples and the solutions were incubated for 16 hours at room temperature. The reaction process was detected by LC-MS.

Following acylation, the 97 reaction mixtures were pooled, precipitated with ethanol, and purified by reverse-phase HPLC. The purified material was concentrated by lyophilization. The purified material was redissolved in sodium borate buffer (18 mL, 150 mM, pH 9.4) to a concentration of 0.25 M, and the mixture was heated to 90 °C for 16 h. After completion, the product was then purified by ethanol precipitation as described above and isolated by centrifugation. The samples were quantified by OD, yielding 5.3 µmol.

### Step 3: Cycle 2 synthesis:

### Procedures:

The above product (5 µmol) from step 2 was split into 100 aliquots. To each PCR tube (2 mL) was added 38.8 µL of a solution of Cycle 1 product (50 nmol) in water. To each tube was added 20 µL of 10× ligase buffer, 2.3 uL T4 DNA ligase and 88.9 µL of water. The resulting solutions were incubated at 16° C for 16 hours. The reaction process was detected by gel electrophoresis.

After the ligation reaction, 20 µL of 5 M aqueous NaCl was added directly to each tube, followed by 660 µL ethanol, and held at -20° C for 1 hour. The plates were centrifugated for 30 minutes at 12,000 rpm to give a white pellet, and the supernatant was removed. The isolated pellet was redissolved in 10 uL water and lyophilized again to white pellet.

Each of the DNA pellets (50 nmol) in tubes was then dissolved in sodium borate buffer (50 µL, 150 mM, pH 9.4) to make 1 mM stock. 80 equivalents of building block in 0.2 M DMA stock (20 µL, 4.0 80 equivalents of HATU in 0.4 M DMA stock (10 uL, 4.0 80 equivalents of DIPEA in 0.4 M DMA stock (10 µL, 4.0 µmol) were cooled at 4 degree for 5 min, and then mixed together to react for 5 minutes at 4 °C. After that, the mixture was added to each tube and the solutions reacted for 16 hours at room temperature. In this cycle, 1 null control experiment was designed (addition of reagents but no addition of building block).

Following acylation, a 0.1× volume of 5 M aqueous NaCl and a 3× volume of ethanol was added and the mixture was allowed to stand at -20° C for at least one hour. The mixture was then centrifuged for 30 minutes at 12,000 rpm. The supernatant was removed after centrifugation, and the pellet was pooled and redissolved in 50 uL H₂O. 0.1× volume of piperidine was added to the solution and the mixture was reacted 1 h at room temperature. The product was then purified by ethanol precipitation as described above and isolated by centrifugation. The isolated pellet was redissolved in 10 uL water and lyophilized again. After that, the DNA pellet was further purified by Millipore Amicon Ultra-15 Centrifugal Filter Units (3000-MW cutoff). The pooled DNA sample was further quantified by OD (4.6 µmοl).

**Table 2. Cycle 2 building blocks and oligonucleotide tags**

| Entry | Building blocks | Oligonucleotide tags (5' to 3', with 5'-PHO) | |
|---|---|---|---|
| | | Top strand sequence | Bottom strand sequence |
| 1 | 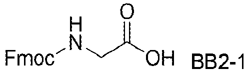 | ATAGAGCGCAGGT (SEQ ID NO.: 199) | CTGCGCTCTATCT (SEQ ID NO.: 200) |
| 2 | 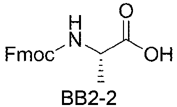 | GACACGCAGTCGT (SEQ ID NO.: 201) | GACTGCGTGTCCT (SEQ ID NO.: 202) |
| 3 | 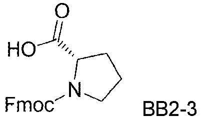 | GTATCCAGACCGT (SEQ ID NO.: 203) | GGTCTGGATACCT (SEQ ID NO.: 204) |
| 4 | 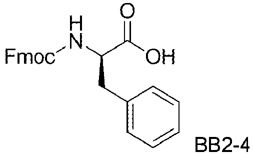 | AATACTCAGACGT (SEQ ID NO.: 205) | GTCTGAGTATTCT (SEQ ID NO.: 206) |
| 5 | 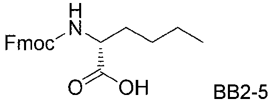 | GAGGACAACCAGT (SEQ ID NO.: 207) | TGGTTGTCCTCCT (SEQ ID NO.: 208) |
| 6 | 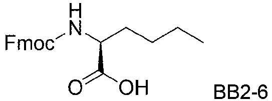 | TTACAATGGAGGT (SEQ ID NO.: 209) | CTCCATTGTAACT (SEQ ID NO.: 210) |
| 7 | 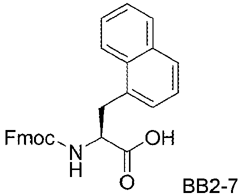 | TCGAGTTGGAGGT (SEQ ID NO.: 211) | CTCCAACTCGACT (SEQ ID NO.: 212) |
| 8 | 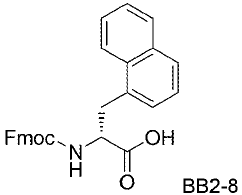 | TGCCTCACGGTGT (SEQ ID NO.: 213) | ACCGTGAGGCACT (SEQ ID NO.: 214) |
| 9 | 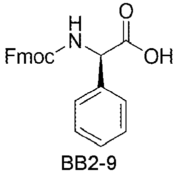 | TCTGGAGCTTCGT (SEQ ID NO.: 215) | GAAGCTCCAGACT (SEQ ID NO.: 216) |
| 10 | 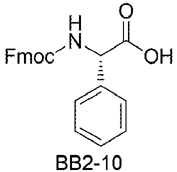 | CGGCGGCAACAGT (SEQ ID NO.: 217) | TGTTGCCGCCGCT (SEQ ID NO.: 218) |
| 11 | 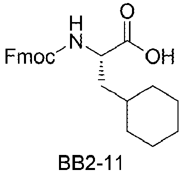 | GTCCGAAGACTGT (SEQ ID NO.: 219) | AGTCTTCGGACCT (SEQ ID NO.: 220) |
| 12 | 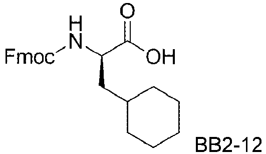 | TTACGCTAATGGT (SEQ ID NO.: 221) | CATTAGCGTAACT (SEQ ID NO.: 222) |
| 13 | 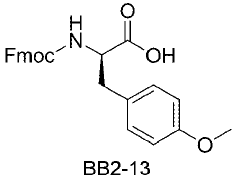 | ATCTTGGCAACGT (SEQ ID NO.: 223) | GTTGCCAAGATCT (SEQ ID NO.: 224) |
| 14 | 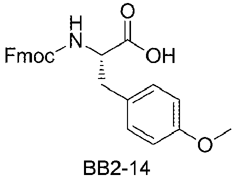 | CTGCGGTCTAAGT (SEQ ID NO.: 225) | TTAGACCGCAGCT (SEQ ID NO.: 226) |
| 15 | 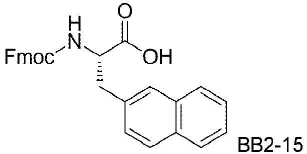 | TCCAAGCGATGGT (SEQ ID NO.: 227) | CATCGCTTGGACT (SEQ ID NO.: 228) |
| 16 | 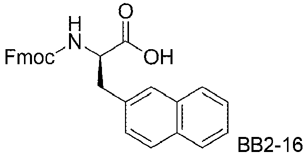 | AACACCAATCTGT (SEQ ID NO.: 229) | AGATTGGTGTTCT (SEQ ID NO.: 230) |
| 17 | 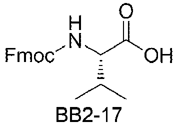 | TGTACCTTCATGT (SEQ ID NO.: 231) | ATGAAGGTACACT (SEQ ID NO.: 232) |
| 18 | 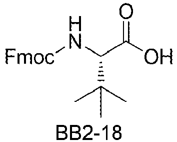 | CTTCCGACTCAGT (SEQ ID NO.: 233) | TGAGTCGGAAGCT (SEQ ID NO.: 234) |
| 19 | 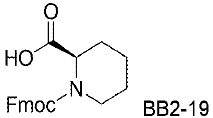 | CGTAGTGAGTCGT (SEQ ID NO.: 235) | GACTCACTACGCT (SEQ ID NO.: 236) |
| 20 | 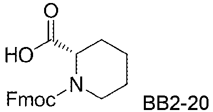 | CGGAGTGTAATGT (SEQ ID NO.: 237) | ATTACACTCCGCT (SEQ ID NO.: 238) |
| 21 | 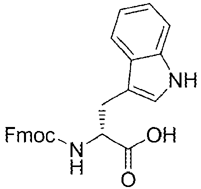 | TGCAGTAACTGGT (SEQ ID NO.: 239) | CAGTTACTGCACT (SEQ ID NO.: 240) |
| | 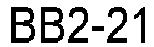 | | |
| 22 | 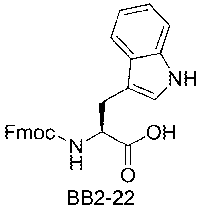 | ATACAACAGCCGT (SEQ ID NO.: 241) | GGCTGTTGTATCT (SEQ ID NO.: 242) |
| 23 | 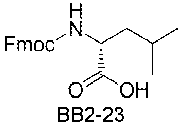 | TGTCATCATGAGT (SEQ ID NO.: 243) | TCATGATGACACT (SEQ ID NO.: 244) |
| 24 | 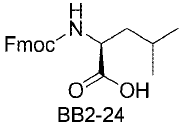 | GAACGATAGGCGT (SEQ ID NO.: 245) | GCCTATCGTTCCT (SEQ ID NO.: 246) |
| 25 | 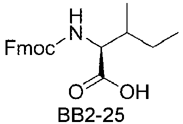 | TTCCGACGAGAGT (SEQ ID NO.: 247) | TCTCGTCGGAACT (SEQ ID NO.: 248) |
| 26 | 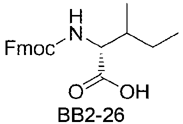 | ACAAGGCGGCAGT (SEQ ID NO.: 249) | TGCCGCCTTGTCT (SEQ ID NO.: 250) |
| 27 | 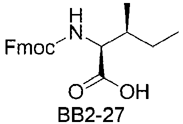 | AACTGTAGTGCGT (SEQ ID NO.: 251) | GCACTACAGTTCT (SEQ ID NO.: 252) |
| 28 | 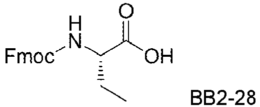 | TCCTCCTTGCGGT (SEQ ID NO.: 253) | CGCAAGGAGGACT (SEQ ID NO.: 254) |
| 29 | 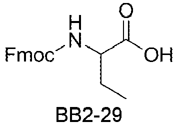 | CTGAGGTGTCGGT (SEQ ID NO.: 255) | CGACACCTCAGCT (SEQ ID NO.: 256) |
| 30 | 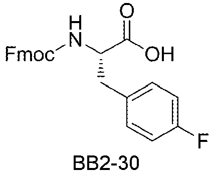 | ATCTTACTCTCGT (SEQ ID NO.: 257) | GAGAGTAAGATCT (SEQ ID NO.: 258) |
| 31 | 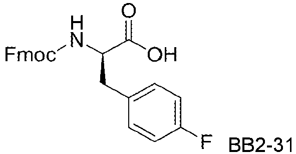 | GATTGCGATCTGT (SEQ ID NO.: 259) | AGATCGCAATCCT (SEQ ID NO.: 260) |
| 32 | 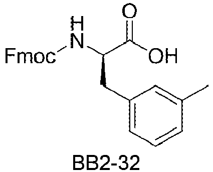 | CGTCAACTAACGT (SEQ ID NO.: 261) | GTTAGTTGACGCT (SEQ ID NO.: 262) |
| 33 | 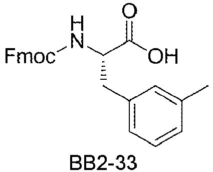 | GACGTGGAGACGT (SEQ ID NO.: 263) | GTCTCCACGTCCT (SEQ ID NO.: 264) |
| 34 | 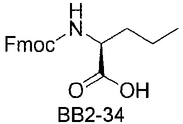 | TAGCCACAGCTGT (SEQ ID NO.: 265) | AGCTGTGGCTACT (SEQ ID NO.: 266) |
| 35 | 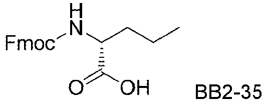 | TCCTATGCCTAGT (SEQ ID NO.: 267) | TAGGCATAGGACT (SEQ ID NO.: 268) |
| 36 | 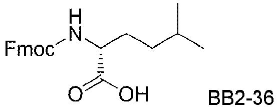 | GCGTAGACGCTGT (SEQ ID NO.: 269) | AGCGTCTACGCCT (SEQ ID NO.: 270) |
| 37 | 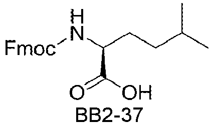 | CGCCTATCTTCGT (SEQ ID NO.: 271) | GAAGATAGGCGCT (SEQ ID NO.: 272) |
| 38 | 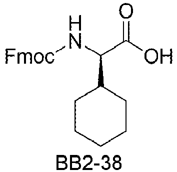 | ACAACGGTCTTGT (SEQ ID NO.: 273) | AAGACCGTTGTCT (SEQ ID NO.: 274) |
| 39 | 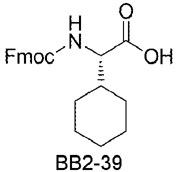 | CGTCCGATAGTGT (SEQ ID NO.: 275) | ACTATCGGACGCT (SEQ ID NO.: 276) |
| 40 | 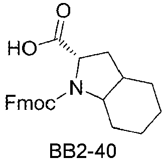 | ATGTGTCAGGAGT (SEQ ID NO.: 277) | TCCTGACACATCT (SEQ ID NO.: 278) |
| 41 | 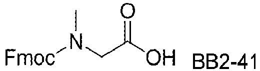 | TAGGCATTACGGT (SEQ ID NO.: 279) | CGTAATGCCTACT (SEQ ID NO.: 280) |
| 42 | 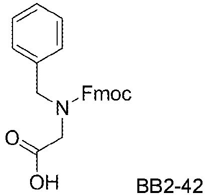 | TCACCAAGATTGT (SEQ ID NO.: 281) | AATCTTGGTGACT (SEQ ID NO.: 282) |
| 43 | 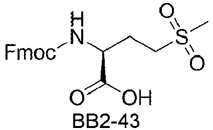 | ACAGCGTCATCGT (SEQ ID NO.: 283) | GATGACGCTGTCT (SEQ ID NO.: 284) |
| 44 | 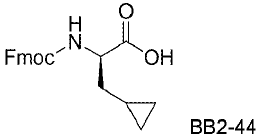 | CCTACTGTCGCGT (SEQ ID NO.: 285) | GCGACAGTAGGCT (SEQ ID NO.: 286) |
| 45 | 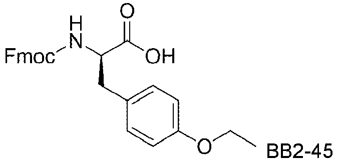 | GTCATCGTGATGT (SEQ ID NO.: 287) | ATCACGATGACCT (SEQ ID NO.: 288) |
| 46 | 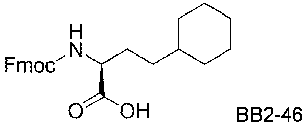 | CAGCCGTGCTCGT (SEQ ID NO.: 289) | GAGCACGGCTGCT (SEQ ID NO.: 290) |
| 47 | 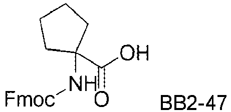 | GAGCTACACTTGT (SEQ ID NO.: 291) | AAGTGTAGCTCCT (SEQ ID NO.: 292) |
| 48 | 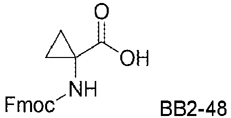 | CACCATCAACGGT (SEQ ID NO.: 293) | CGTTGATGGTGCT (SEQ ID NO.: 294) |
| 49 | 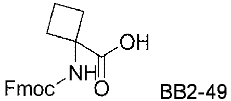 | TCTGCCGTAAGGT (SEQ ID NO.: 295) | CTTACGGCAGACT (SEQ ID NO.: 296) |
| 50 | 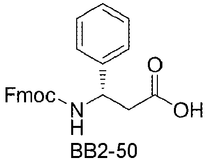 | GAGACTGAAGCGT (SEQ ID NO.: 297) | GCTTCAGTCTCCT (SEQ ID NO.: 298) |
| 51 | 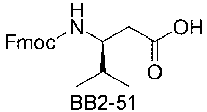 | GATGTTCACTGGT (SEQ ID NO.: 299) | CAGTGAACATCCT (SEQ ID NO.: 300) |
| 52 | 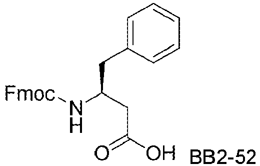 | AGTGCTAACAGGT (SEQ ID NO.: 301) | CTGTTAGCACTCT (SEQ ID NO.: 302) |
| 53 | 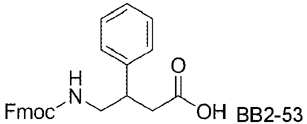 | AACGCAGCATAGT (SEQ ID NO.: 303) | TATGCTGCGTTCT (SEQ ID NO.: 304) |
| 54 | 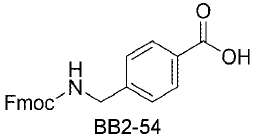 | AGTGAGTAGCAGT (SEQ ID NO.: 305) | TGCTACTCACTCT (SEQ ID NO.: 306) |
| 55 | 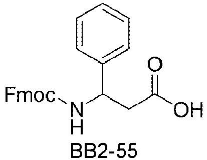 | TAGGCTCCTCGGT (SEQ ID NO.: 307) | CGAGGAGCCTACT (SEQ ID NO.: 308) |
| 56 | 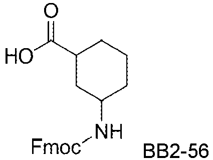 | TCAACGGCGCAGT (SEQ ID NO.: 309) | TGCGCCGTTGACT (SEQ ID NO.: 310) |
| 57 | 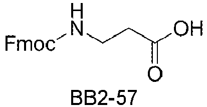 | ACTCTTATCGTGT (SEQ ID NO.: 311) | ACGATAAGAGTCT (SEQ ID NO.: 312) |
| 58 | 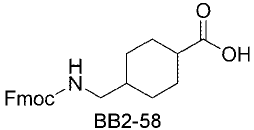 | GCATTAGGAGAGT (SEQ ID NO.: 313) | TCTCCTAATGCCT (SEQ ID NO.: 314) |
| 59 | 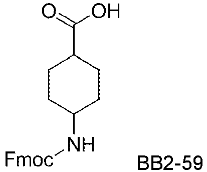 | TAAGAAGCCAAGT (SEQ ID NO.: 315) | TTGGCTTCTTACT (SEQ ID NO.: 316) |
| 60 | 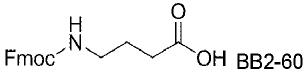 | ATATACTACATGT (SEQ ID NO.: 317) | ATGTAGTATATCT (SEQ ID NO.: 318) |
| 61 | 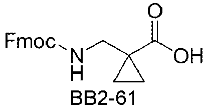 | TTACATCCGACGT (SEQ ID NO.: 319) | GTCGGATGTAACT (SEQ ID NO.: 320) |
| 62 | 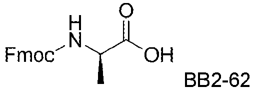 | TAGAGGTCGTTGT (SEQ ID NO.: 321) | AACGACCTCTACT (SEQ ID NO.: 322) |
| 63 | 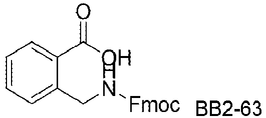 | TACCACCGTACGT (SEQ ID NO.: 323) | GTACGGTGGTACT (SEQ ID NO.: 324) |
| 64 | 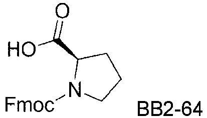 | GAGTTATGCTCGT (SEQ ID NO.: 325) | GAGCATAACTCCT (SEQ ID NO.: 326) |
| 65 | 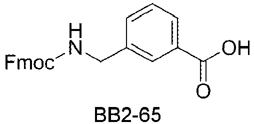 | GTCGTGAGTCCGT (SEQ ID NO.: 327) | GGACTCACGACCT (SEQ ID NO.: 328) |
| 66 | 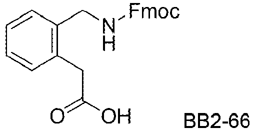 | CCACTGCCTACGT (SEQ ID NO.: 329) | GTAGGCAGTGGCT (SEQ ID NO.: 330) |
| 67 | 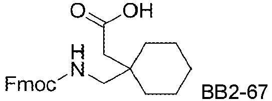 | TTCGATTGTGTGT (SEQ ID NO.: 331) | ACACAATCGAACT (SEQ ID NO.: 332) |
| 68 | 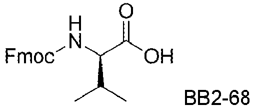 | CACAACTGGACGT (SEQ ID NO.: 333) | GTCCAGTTGTGCT (SEQ ID NO.: 334) |
| 69 | 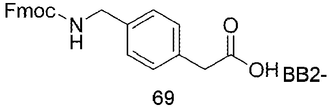 | GCTGATGTTCAGT (SEQ ID NO.: 335) | TGAACATCAGCCT (SEQ ID NO.: 336) |
| 70 | 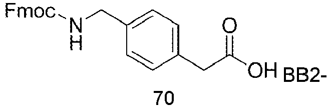 | GTGAGACCATCGT (SEQ ID NO.: 337) | GATGGTCTCACCT (SEQ ID NO.: 338) |
| 71 | 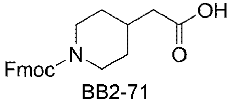 | ATTACAGACCGGT (SEQ ID NO.: 339) | CGGTCTGTAATCT (SEQ ID NO.: 340) |
| 72 | 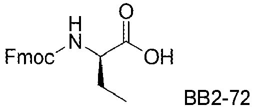 | GAACTCCTAGAGT (SEQ ID NO.: 341) | TCTAGGAGTTCCT (SEQ ID NO.: 342) |
| 73 | 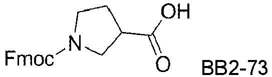 | TGGCCTTAGTCGT (SEQ ID NO.: 343) | GACTAAGGCCACT (SEQ ID NO.: 344) |
| 74 | 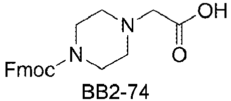 | ATATCAACGCGGT (SEQ ID NO.: 345) | CGCGTTGATATCT (SEQ ID NO.: 346) |
| 75 | Null | GCATGGTATCAGT (SEQ ID NO.: 347) | TGATACCATGCCT (SEQ ID NO.: 348) |
| 76 | 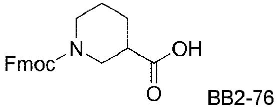 | CGAATAGAATGGT (SEQ ID NO.: 349) | CATTCTATTCGCT (SEQ ID NO.: 350) |
| 77 | 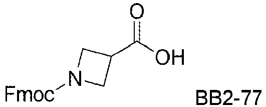 | GACAAGCATAGGT (SEQ ID NO.: 351) | CTATGCTTGTCCT (SEQ ID NO.: 352) |
| 78 | 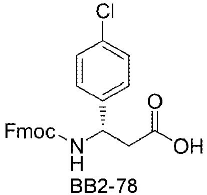 | AATACGAGACTGT (SEQ ID NO.: 353) | AGTCTCGTATTCT (SEQ ID NO.: 354) |
| 79 | 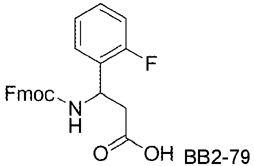 | AGCTTGTGCTAGT (SEQ ID NO.: 355) | TAGCACAAGCTCT (SEQ ID NO.: 356) |
| 80 | 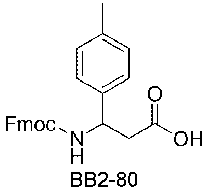 | CAGTCACGCGTGT (SEQ ID NO.: 357) | ACGCGTGACTGCT (SEQ ID NO.: 358) |
| 81 | 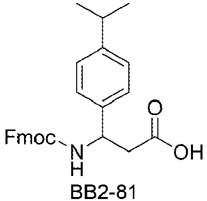 | GCAGGCGGAGTGT (SEQ ID NO.: 359) | ACTCCGCCTGCCT (SEQ ID NO.: 360) |
| 82 | 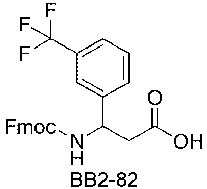 | AGAGTGGTCTGGT (SEQ ID NO.: 361) | CAGACCACTCTCT (SEQ ID NO.: 362) |
| 83 | 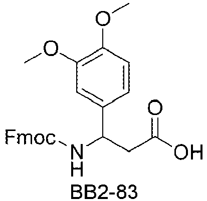 | ATATGAAGCAGGT (SEQ ID NO.: 363) | CTGCTTCATATCT (SEQ ID NO.: 364) |
| 84 | 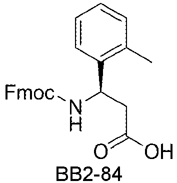 | CGGCTTCATCAGT (SEQ ID NO.: 365) | TGATGAAGCCGCT (SEQ ID NO.: 366) |
| 85 | 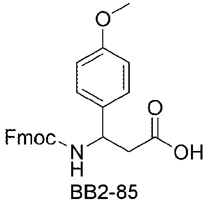 | TAGACAACCATGT (SEQ ID NO.: 367) | ATGGTTGTCTACT (SEQ ID NO.: 368) |
| 86 | 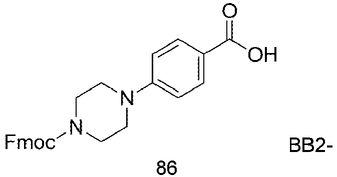 | AGCACAACTGTGT (SEQ ID NO.: 369) | ACAGTTGTGCTCT (SEQ ID NO.: 370) |
| 87 | 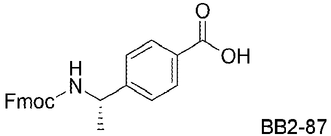 | AGATTATTAGCGT (SEQ ID NO.: 371) | GCTAATAATCTCT (SEQ ID NO.: 372) |
| 88 | 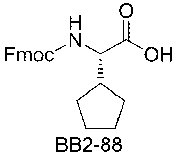 | TATTGAACTTGGT (SEQ ID NO.: 373) | CAAGTTCAATACT (SEQ ID NO.: 374) |
| 89 | 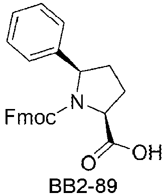 | CGTTCCAGACAGT (SEQ ID NO.: 375) | TGTCTGGAACGCT (SEQ ID NO.: 376) |
| 90 | 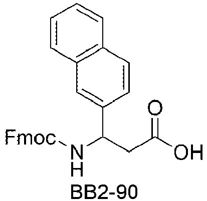 | CACTAAGAGCCGT (SEQ ID NO.: 377) | GGCTCTTAGTGCT (SEQ ID NO.: 378) |
| 91 | 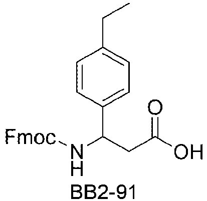 | GCCGCATAAGCGT (SEQ ID NO.: 379) | GCTTATGCGGCCT (SEQ ID NO.: 380) |
| 92 | 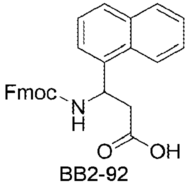 | GCCTTCAGGACGT (SEQ ID NO.: 381) | GTCCTGAAGGCCT (SEQ ID NO.: 382) |
| 93 | 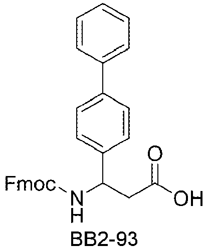 | GTCACGGTAATGT (SEQ ID NO.: 383) | ATTACCGTGACCT (SEQ ID NO.: 384) |
| 94 | 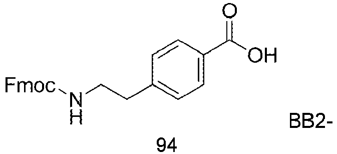 | ATGGCTGCTTGGT (SEQ ID NO.: 385) | CAAGCAGCCATCT (SEQ ID NO.: 386) |
| 95 | 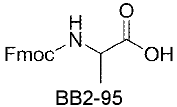 | CCTGTCTTGTGGT (SEQ ID NO.: 387) | CACAAGACAGGCT (SEQ ID NO.: 388) |
| 96 | 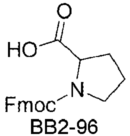 | CCGAACAGGCTGT (SEQ ID NO.: 389) | AGCCTGTTCGGCT (SEQ ID NO.: 390) |
| 97 | 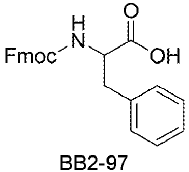 | TAGAGATAATAGT (SEQ ID NO.: 391) | TATTATCTCTACT (SEQ ID NO.: 392) |
| 98 | 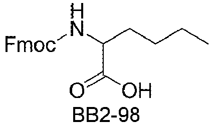 | AGCCATTAGTGGT (SEQ ID NO.: 393) | CACTAATGGCTCT (SEQ ID NO.: 394) |
| 99 | 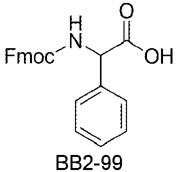 | CATGAACCTACGT (SEQ ID NO.: 395) | GTAGGTTCATGCT (SEQ ID NO.: 396) |
| 100 | 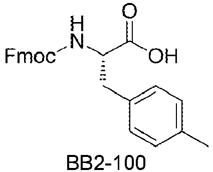 | AGCGAATCTACGT (SEQ ID NO.: 397) | GTAGATTCGCTCT (SEQ ID NO.: 398) |

### Step 4: Cycle 3 synthesis:

### Procedures:

The Cycle 2 product (1.0 mM stock solution in water, 4.5 µmol total) was split into 200 wells (22.5 uL, 22.5 nmol per well). To each well was then added 22.5 µL of a 1 mM solution of one of oligonucleotide tags (the molar ratio of Cycle 2 product to tags was 1:1), 9 µL of 10× ligase buffer, 2.1 uL T4 DNA ligase and 56.4 µL of water. The resulting solutions were incubated at 16° C for 16 hours. The reaction process was detected by gel electrophoresis.

After the ligation reaction, 9 µL of 5 M aqueous NaCl was added directly to each tube, followed by 297 µL ethanol, and held at -20° C. for 1 hour. The plates were centrifugated for 30 minutes at 12,000 rpm to give a white pellet, and the supernatant was removed. The isolated pellet was redissolved in 10 uL water and lyophilized again.

### Capping with acids R-COOH:

150 wells of the DNA pellets were reacted with acid. To each well was dissolved in sodium borate buffer (22.5 µL, 150 mM, pH 9.4) to 1 mM stock. 40 equivalents of acid in 200 mM DMA stock (4.5 µL 0.9 µmol) was added to each well, followed by 40 equivalents of DMT-MM in 200 mM water solution (4.5 uL 0.9 µmol). The acylation reactions were allowed to proceed at room temperature for 16 hours. Following acylation, the product was then purified by ethanol precipitation as described above and isolated by centrifugation. 1 null control experiment was designed (addition of reagents but no addition of building block).

### Capping with aldehyde R-CHO:

30 wells of the DNA pellets were reacted with aldehydes. To each well was dissolved in PBS buffer (22.5 µL, 150 mM, pH 5.5) to make 1 mM stock. 100 equivalents of aldehyde in 200 mM DMA stock (11.3 µL, 2.25 µmol) and 100 equivalents of NaCNBH₃ in 200 mM DMA solution (11.2 uL, 2.25µmol) was added to each well. The reaction was allowed to proceed at 60 degree overnight. After analyzing by LCMS, the product was then purified by ethanol precipitation as described above and isolated by centrifugation. 1 null control experiment was designed (addition of reagents but no addition of building block).

### Capping with sulfonyl chloride R-SO₂Cl:

10 wells of the DNA pellets were reacted with sulfonyl chloride. To each well (22.5 nmol) was dissolved in sodium borate buffer (22.5 µL, 150 mM, pH 9.4) to make 1 mM stock. 50 equivalents of sulfonyl chloride in 500 mM CH₃CN stock (2.3 µL, 1.13 µmol) was added to the each well. The reaction was allowed to proceed at room temperature overnight. After analyzing by LCMS, the product was then purified by ethanol precipitation as described above and isolated by centrifugation. 1 null control experiment was designed (addition of reagents but no addition of building block).

### Capping with isocyanate R-N=C=O:

10 wells of the DNA pellets were reacted with isocyanate. To each well (22.5 nmol) was dissolved in sodium borate buffer (22.5 µL, 150 mM, pH 9.4) to make 1 mM stock. 50 equivalents of isocyanate in 500 mM CH₃CN stock (2.3 µL, 1.13 µmol) was added to the each well. The reaction was allowed to proceed at room temperature overnight. After analyzing by LCMS, the product was then purified by ethanol precipitation as described above and isolated by centrifugation. 1 null control experiment was designed (addition of reagents but no addition of building block).

Finally, the above capping products were all pooled together and further purified by Millipore Amicon Ultra-15 Centrifugal Filter Units (3000-MW cutoff). The pooled DNA sample was further quantified by OD (4.1 µmol).

**Table 3. Cycle 3 building blocks and oligonucleotide tags**

| Entry | Building blocks | Oligonucleotide tags (5' to 3', with 5'-PHO) | |
|---|---|---|---|
| | | Top strand sequence | Bottom strand sequence |
| 1 | 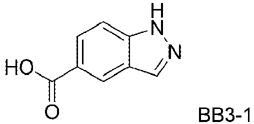 | ATACTAGTCGTGA (SEQ ID NO.: 399) | ACGACTAGTATAC (SEQ ID NO.: 400) |
| 2 | 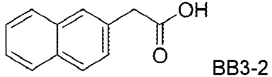 | CGAACGTAGCAGA (SEQ ID NO.: 401) | TGCTACGTTCGAC (SEQ ID NO.: 402) |
| 3 | 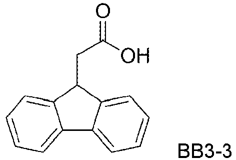 | TCGATATGTGAGA (SEQ ID NO.: 403) | TCACATATCGAAC (SEQ ID NO.: 404) |
| 4 | 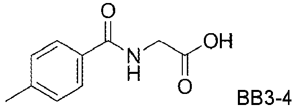 | GAGATATTGTAGA (SEQ ID NO.: 405) | TACAATATCTCAC (SEQ ID NO.: 406) |
| 5 | 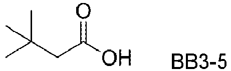 | AGATATCCTCAGA (SEQ ID NO.: 407) | TGAGGATATCTAC (SEQ ID NO.: 408) |
| 6 | 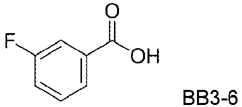 | CGAGGCCTACCGA (SEQ ID NO.: 409) | GGTAGGCCTCGAC (SEQ ID NO.: 410) |
| 7 | 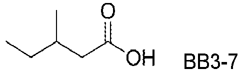 | TCGGACCGGAGGA (SEQ ID NO.: 411) | CTCCGGTCCGAAC (SEQ ID NO.: 412) |
| 8 | 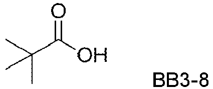 | ATACCGTATCCGA (SEQ ID NO.: 413) | GGATACGGTATAC (SEQ ID NO.: 414) |
| 9 | 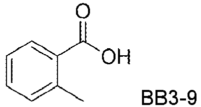 | GTGCGCAACCTGA (SEQ ID NO.: 415) | AGGTTGCGCACAC (SEQ ID NO.: 416) |
| 10 | 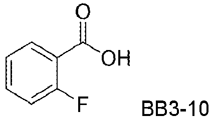 | CTAGCTATCGCGA (SEQ ID NO.: 417) | GCGATAGCTAGAC (SEQ ID NO.: 418) |
| 11 | 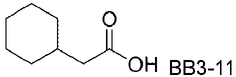 | TAGAGTCTCGTGA (SEQ ID NO.: 419) | ACGAGACTCTAAC (SEQ ID NO.: 420) |
| 12 | 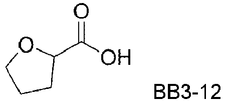 | CAGCGCATAAGGA (SEQ ID NO.: 421) | CTTATGCGCTGAC (SEQ ID NO.: 422) |
| 13 | 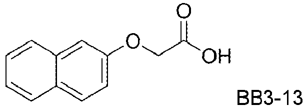 | ATCTACGTGGTGA (SEQ ID NO.: 423) | ACCACGTAGATAC (SEQ ID NO.: 424) |
| 14 | 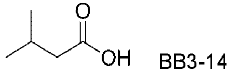 | AAGTACAATATGA (SEQ ID NO.: 425) | ATATTGTACTTAC (SEQ ID NO.: 426) |
| 15 | 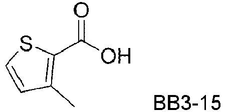 | TGCTTAGCACTGA (SEQ ID NO.: 427) | AGTGCTAAGCAAC (SEQ ID NO.: 428) |
| 16 | 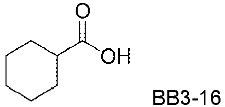 | TGCATGTCTCGGA (SEQ ID NO.: 429) | CGAGACATGCAAC (SEQ ID NO.: 430) |
| 17 | 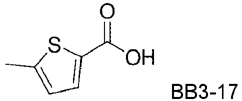 | ATAGGATCCGAGA (SEQ ID NO.: 431) | TCGGATCCTATAC (SEQ ID NO.: 432) |
| 18 | 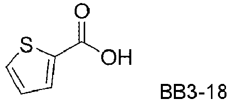 | GTTGGCCGGTGGA (SEQ ID NO.: 433) | CACCGGCCAACAC (SEQ ID NO.: 434) |
| 19 | 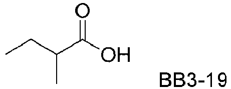 | AGTATTAAGTTGA (SEQ ID NO.: 435) | AACTTAATACTAC (SEQ ID NO.: 436) |
| 20 | 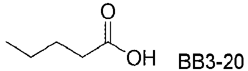 | AAGCGTACAGAGA (SEQ ID NO.: 437) | TCTGTACGCTTAC (SEQ ID NO.: 438) |
| 21 | 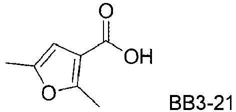 | CCACCTAGTAGGA (SEQ ID NO.: 439) | CTACTAGGTGGAC (SEQ ID NO.: 440) |
| 22 | 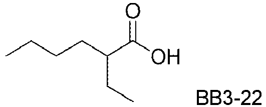 | CAACTAGCGTCGA (SEQ ID NO.: 441) | GACGCTAGTTGAC (SEQ ID NO.: 442) |
| 23 | 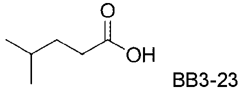 | TATCCGGTCTAGA (SEQ ID NO.: 443) | TAGACCGGATAAC (SEQ ID NO.: 444) |
| 24 | 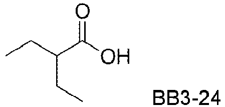 | CGGACATGCGCGA (SEQ ID NO.: 445) | GCGCATGTCCGAC (SEQ ID NO.: 446) |
| 25 | 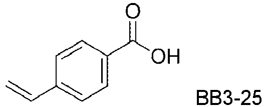 | TCTCTTAAGACGA (SEQ ID NO.: 447) | GTCTTAAGAGAAC (SEQ ID NO.: 448) |
| 26 | 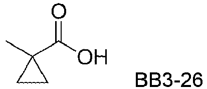 | CAGACATGTCAGA (SEQ ID NO.: 449) | TGACATGTCTGAC (SEQ ID NO.: 450) |
| 27 | 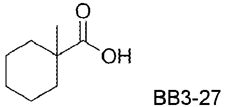 | CCAACATGATCGA (SEQ ID NO.: 451) | GATCATGTTGGAC (SEQ ID NO.: 452) |
| 28 | 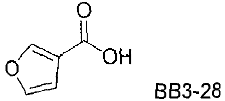 | GTGATCCAACAGA (SEQ ID NO.: 453) | TGTTGGATCACAC (SEQ ID NO.: 454) |
| 29 | 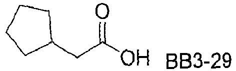 | GTCTCAGATTAGA (SEQ ID NO.: 455) | TAATCTGAGACAC (SEQ ID NO.: 456) |
| 30 | 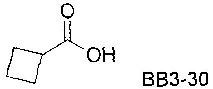 | GTCAGCTTGCCGA (SEQ ID NO.: 457) | GGCAAGCTGACAC (SEQ ID NO.: 458) |
| 31 | 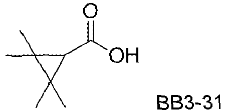 | AGCTGCAAGCTGA (SEQ ID NO.: 459) | AGCTTGCAGCTAC (SEQ ID NO.: 460) |
| 32 | 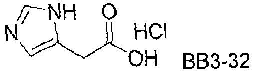 | CTCTTAAGGCCGA (SEQ ID NO.: 461) | GGCCTTAAGAGAC (SEQ ID NO.: 462) |
| 33 | 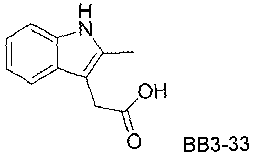 | GAGCGCAGCTTGA (SEQ ID NO.: 463) | AAGCTGCGCTCAC (SEQ ID NO.: 464) |
| 34 | 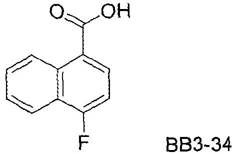 | GAAGCGCCGTCGA (SEQ ID NO.: 465) | GACGGCGCTTCAC (SEQ ID NO.: 466) |
| 35 | 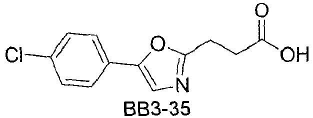 | CTCTAGTCGACGA (SEQ ID NO.: 467) | GTCGACTAGAGAC (SEQ ID NO.: 468) |
| 36 | 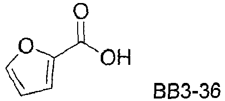 | AGGACGTTGTCGA (SEQ ID NO.: 469) | GACAACGTCCTAC (SEQ ID NO.: 470) |
| 37 | 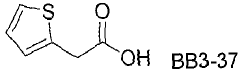 | GAGATCCTAAGGA (SEQ ID NO.: 471) | CTTAGGATCTCAC (SEQ ID NO.: 472) |
| 38 | 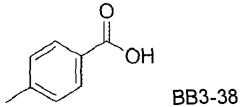 | GCTATACCTATGA (SEQ ID NO.: 473) | ATAGGTATAGCAC (SEQ ID NO.: 474) |
| 39 | 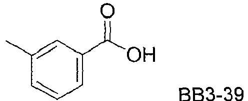 | GATTAATGCGCGA (SEQ ID NO.: 475) | GCGCATTAATCAC (SEQ ID NO.: 476) |
| 40 | 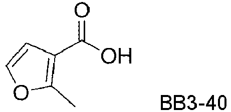 | CTGATCCGATGGA (SEQ ID NO.: 477) | CATCGGATCAGAC (SEQ ID NO.: 478) |
| 41 | 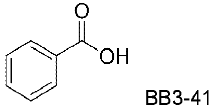 | CTCAGCTGGTGGA (SEQ ID NO.: 479) | CACCAGCTGAGAC (SEQ ID NO.: 480) |
| 42 | 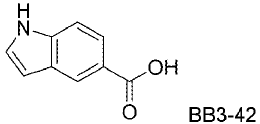 | CATATAGCAAGGA (SEQ ID NO.: 481) | CTTGCTATATGAC (SEQ ID NO.: 482) |
| 43 | 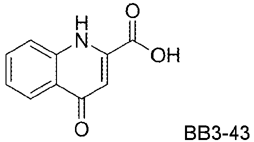 | CAGCGCTTGTTGA (SEQ ID NO.: 483) | AACAAGCGCTGAC (SEQ ID NO.: 484) |
| 44 | 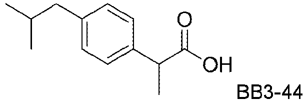 | TCCATGGCGGCGA (SEQ ID NO.: 485) | GCCGCCATGGAAC (SEQ ID NO.: 486) |
| 45 | 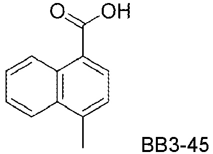 | TAAGCTGCGCTGA (SEQ ID NO.: 487) | AGCGCAGCTTAAC (SEQ ID NO.: 488) |
| 46 | 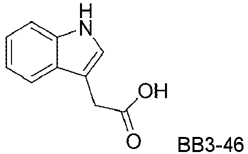 | TAATCGACATGGA (SEQ ID NO.: 489) | CATGTCGATTAAC (SEQ ID NO.: 490) |
| 47 | 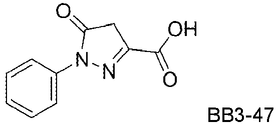 | TCCTGGCCTCGGA (SEQ ID NO.: 491) | CGAGGCCAGGAAC (SEQ ID NO.: 492) |
| 48 | 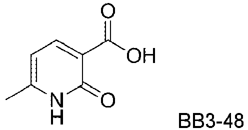 | ACACGTTCTTAGA (SEQ ID NO.: 493) | TAAGAACGTGTAC (SEQ ID NO.: 494) |
| 49 | 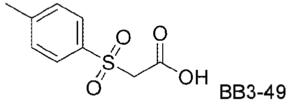 | TCGGAATTCAAGA (SEQ ID NO.: 495) | TTGAATTCCGAAC (SEQ ID NO.: 496) |
| 50 | 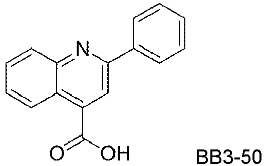 | ACGTACACTCGGA (SEQ ID NO.: 497) | CGAGTGTACGTAC (SEQ ID NO.: 498) |
| 51 | 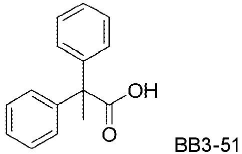 | TGCCGGCTGAAGA (SEQ ID NO.: 499) | TTCAGCCGGCAAC (SEQ ID NO.: 500) |
| 52 | 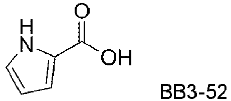 | GAAGTACGACCGA (SEQ ID NO.: 501) | GGTCGTACTTCAC (SEQ ID NO.: 502) |
| 53 | 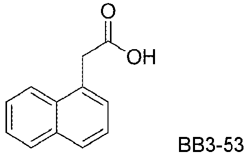 | AACATATTAGTGA (SEQ ID NO.: 503) | ACTAATATGTTAC (SEQ ID NO.: 504) |
| 54 | 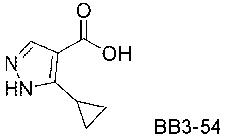 | GTACATGAGGAGA (SEQ ID NO.: 505) | TCCTCATGTACAC (SEQ ID NO.: 506) |
| 55 | 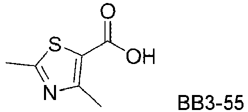 | ATGGTCCAGCCGA (SEQ ID NO.: 507) | GGCTGGACCATAC (SEQ ID NO.: 508) |
| 56 | 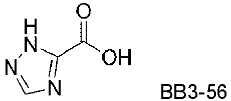 | CATTAAGTATCGA (SEQ ID NO.: 509) | GATACTTAATGAC (SEQ ID NO.: 510) |
| 57 | 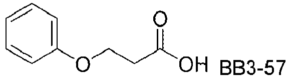 | AAGCTATAGTTGA (SEQ ID NO.: 511) | AACTATAGCTTAC (SEQ ID NO.: 512) |
| 58 | 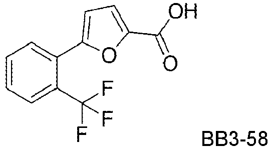 | TACTAGGCGCGGA (SEQ ID NO.: 513) | CGCGCCTAGTAAC (SEQ ID NO.: 514) |
| 59 | 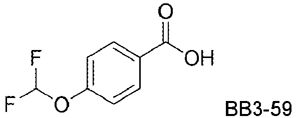 | GATCGAGTGGCGA (SEQ ID NO.: 515) | GCCACTCGATCAC (SEQ ID NO.: 516) |
| 60 | 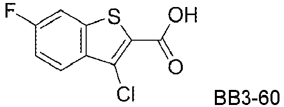 | TGAATTGAAGCGA (SEQ ID NO.: 517) | GCTTCAATTCAAC (SEQ ID NO.: 518) |
| 61 | 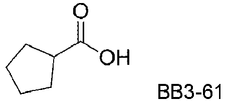 | GTGGCGCACATGA (SEQ ID NO.: 519) | ATGTGCGCCACAC (SEQ ID NO.: 520) |
| 62 | 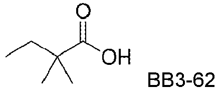 | AATTACGTTACGA (SEQ ID NO.: 521) | GTAACGTAATTAC (SEQ ID NO.: 522) |
| 63 | 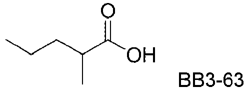 | TCCTTAAGTCTGA (SEQ ID NO.: 523) | AGACTTAAGGAAC (SEQ ID NO.: 524) |
| 64 | 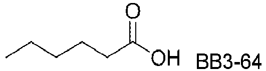 | CCTCGACGGATGA (SEQ ID NO.: 525) | ATCCGTCGAGGAC (SEQ ID NO.: 526) |
| 65 | 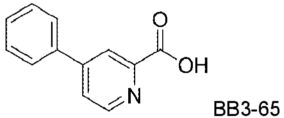 | TAGATCCAACCGA (SEQ ID NO.: 527) | GGTTGGATCTAAC (SEQ ID NO.: 528) |
| 66 | 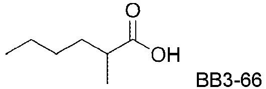 | TCAGCTCATGAGA (SEQ ID NO.: 529) | TCATGAGCTGAAC (SEQ ID NO.: 530) |
| 67 | 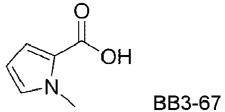 | GCAGAGCTAGCGA (SEQ ID NO.: 531) | GCTAGCTCTGCAC (SEQ ID NO.: 532) |
| 68 | 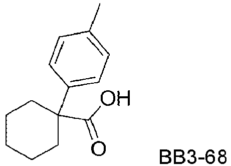 | TCAACTAGAGTGA (SEQ ID NO.: 533) | ACTCTAGTTGAAC (SEQ ID NO.: 534) |
| 69 | 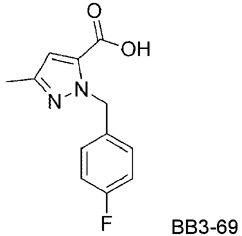 | ACCGCTAGTCAGA (SEQ ID NO.: 535) | TGACTAGCGGTAC (SEQ ID NO.: 536) |
| 70 | 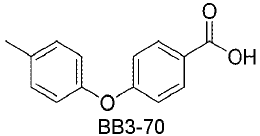 | ACGTACTATTGGA (SEQ ID NO.: 537) | CAATAGTACGTAC (SEQ ID NO.: 538) |
| 71 | 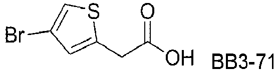 | AGAGCGCCGTTGA (SEQ ID NO.: 539) | AACGGCGCTCTAC (SEQ ID NO.: 540) |
| 72 | 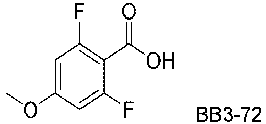 | GACTCAGTATGGA (SEQ ID NO.: 541) | CATACTGAGTCAC (SEQ ID NO.: 542) |
| 73 | 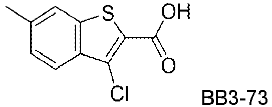 | TGCGTACCGTTGA (SEQ ID NO.: 543) | AACGGTACGCAAC (SEQ ID NO.: 544) |
| 74 | 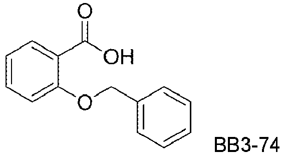 | ATACATGCTAAGA (SEQ ID NO.: 545) | TTAGCATGTATAC (SEQ ID NO.: 546) |
| 75 | 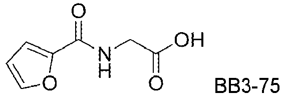 | TGCTAGAATAGGA (SEQ ID NO.: 547) | CTATTCTAGCAAC (SEQ ID NO.: 548) |
| 76 | 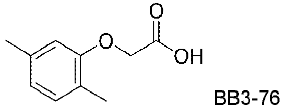 | TCATATAGGTAGA (SEQ ID NO.: 549) | TACCTATATGAAC (SEQ ID NO.: 550) |
| 77 | 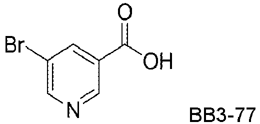 | TATCCTAGCAGGA (SEQ ID NO.: 551) | CTGCTAGGATAAC (SEQ ID NO.: 552) |
| 78 | 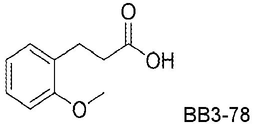 | ACGGTGCAACAGA (SEQ ID NO.: 553) | TGTTGCACCGTAC (SEQ ID NO.: 554) |
| 79 | 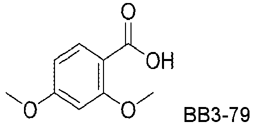 | CACAATGTCTGGA (SEQ ID NO.: 555) | CAGACATTGTGAC (SEQ ID NO.: 556) |
| 80 | 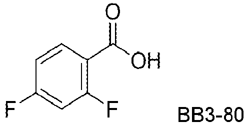 | TGAGACGTACAGA (SEQ ID NO.: 557) | TGTACGTCTCAAC (SEQ ID NO.: 558) |
| 81 | 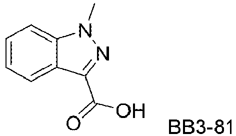 | TACCATGCCATGA (SEQ ID NO.: 559) | ATGGCATGGTAAC (SEQ ID NO.: 560) |
| 82 | 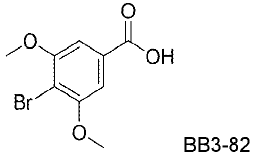 | TCATATTGGACGA (SEQ ID NO.: 561) | GTCCAATATGAAC (SEQ ID NO.: 562) |
| 83 | 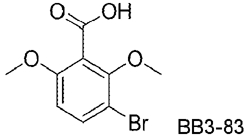 | GACATGGTATTGA (SEQ ID NO.: 563) | AATACCATGTCAC (SEQ ID NO.: 564) |
| 84 | 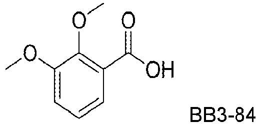 | CGTTCTAGAATGA (SEQ ID NO.: 565) | ATTCTAGAACGAC (SEQ ID NO.: 566) |
| 85 | 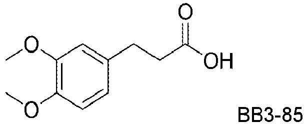 | ATACCATGTCGGA (SEQ ID NO.: 567) | CGACATGGTATAC (SEQ ID NO.: 568) |
| 86 | 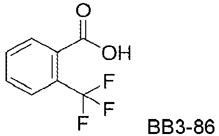 | TCGAATTGTCGGA (SEQ ID NO.: 569) | CGACAATTCGAAC (SEQ ID NO.: 570) |
| 87 | 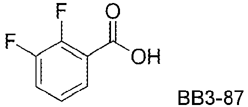 | ACTCCGGTTCAGA (SEQ ID NO.: 571) | TGAACCGGAGTAC (SEQ ID NO.: 572) |
| 88 | 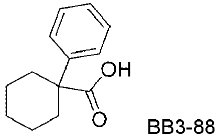 | ACAGTACCAGCGA (SEQ ID NO.: 573) | GCTGGTACTGTAC (SEQ ID NO.: 574) |
| 89 | 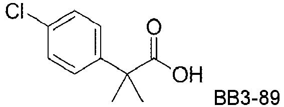 | ATCTAGGCGCAGA (SEQ ID NO.: 575) | TGCGCCTAGATAC (SEQ ID NO.: 576) |
| 90 | 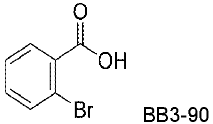 | GTGAATTGTAGGA (SEQ ID NO.: 577) | CTACAATTCACAC (SEQ ID NO.: 578) |
| 91 | 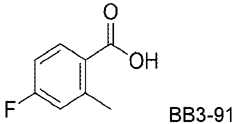 | CGATCCGGTCAGA (SEQ ID NO.: 579) | TGACCGGATCGAC (SEQ ID NO.: 580) |
| 92 | 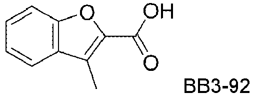 | CCTTACGTAATGA (SEQ ID NO.: 581) | ATTACGTAAGGAC (SEQ ID NO.: 582) |
| 93 | 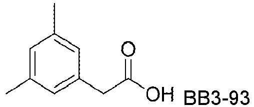 | AGCTCGAAGCGGA (SEQ ID NO.: 583) | CGCTTCGAGCTAC (SEQ ID NO.: 584) |
| 94 | 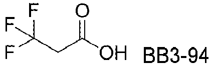 | TCTGCCGGTCGGA (SEQ ID NO.: 585) | CGACCGGCAGAAC (SEQ ID NO.: 586) |
| 95 | 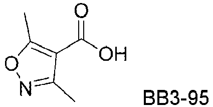 | TCTAGCTGAGCGA (SEQ ID NO.: 587) | GCTCAGCTAGAAC (SEQ ID NO.: 588) |
| 96 | 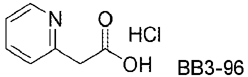 | AGCGGCGCGAAGA (SEQ ID NO.: 589) | TTCGCGCCGCTAC (SEQ ID NO.: 590) |
| 97 | 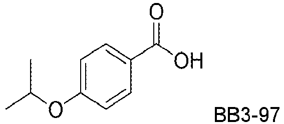 | GTAACATGGTAGA (SEQ ID NO.: 591) | TACCATGTTACAC (SEQ ID NO.: 592) |
| 98 | 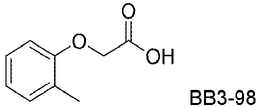 | AGAACGTATGCGA (SEQ ID NO.: 593) | GCATACGTTCTAC (SEQ ID NO.: 594) |
| 99 | 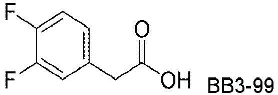 | AGACATATCTAGA (SEQ ID NO.: 595) | TAGATATGTCTAC (SEQ ID NO.: 596) |
| 100 | 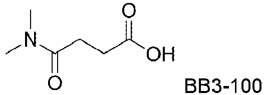 | GAGTAGCTTAGGA (SEQ ID NO.: 597) | CTAAGCTACTCAC (SEQ ID NO.: 598) |
| 101 | 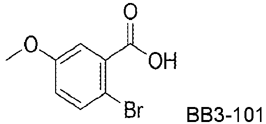 | TCTCGAGCAACGA (SEQ ID NO.: 599) | GTTGCTCGAGAAC (SEQ ID NO.: 600) |
| 102 | 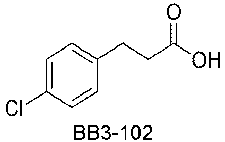 | GAGCAGCTCGAGA (SEQ ID NO.: 601) | TCGAGCTGCTCAC (SEQ ID NO.: 602) |
| 103 | 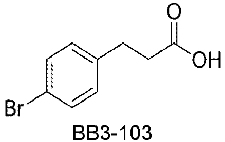 | GTGGCTAGGCCGA (SEQ ID NO.: 603) | GGCCTAGCCACAC (SEQ ID NO.: 604) |
| 104 | 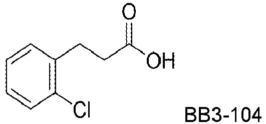 | AAGTACAGAGAGA (SEQ ID NO.: 605) | TCTCTGTACTTAC (SEQ ID NO.: 606) |
| 105 | 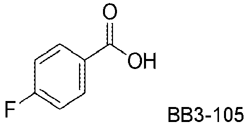 | GACATGGTGCCGA (SEQ ID NO.: 607) | GGCACCATGTCAC (SEQ ID NO.: 608) |
| 106 | 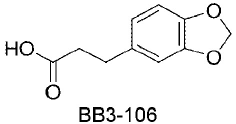 | ACATCGACACAGA (SEQ ID NO.: 609) | TGTGTCGATGTAC (SEQ ID NO.: 610) |
| 107 | 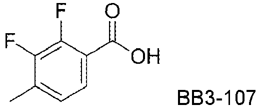 | GTTACTAACGTGA (SEQ ID NO.: 611) | ACGTTAGTAACAC (SEQ ID NO.: 612) |
| 108 | 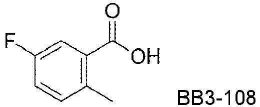 | CTGATCCTAGTGA (SEQ ID NO.: 613) | ACTAGGATCAGAC (SEQ ID NO.: 614) |
| 109 | 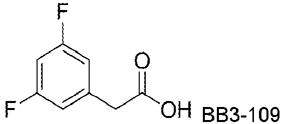 | TAATGGCCGTAGA (SEQ ID NO.: 615) | TACGGCCATTAAC (SEQ ID NO.: 616) |
| 110 | 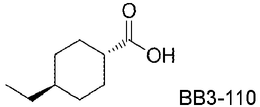 | TCTCGACATGAGA (SEQ ID NO.: 617) | TCATGTCGAGAAC (SEQ ID NO.: 618) |
| 111 | 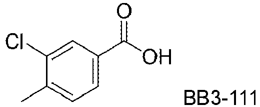 | CCAATTCTGAGGA (SEQ ID NO.: 619) | CTCAGAATTGGAC (SEQ ID NO.: 620) |
| 112 | 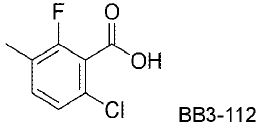 | CGCTAGGACATGA (SEQ ID NO.: 621) | ATGTCCTAGCGAC (SEQ ID NO.: 622) |
| 113 | 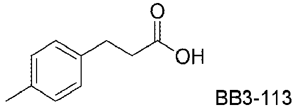 | CGGATTCCTGTGA (SEQ ID NO.: 623) | ACAGGAATCCGAC (SEQ ID NO.: 624) |
| 114 | 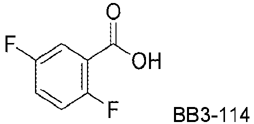 | GTTAGTAATGCGA (SEQ ID NO.: 625) | GCATTACTAACAC (SEQ ID NO.: 626) |
| 115 | 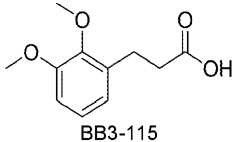 | CTACCATGACCGA (SEQ ID NO.: 627) | GGTCATGGTAGAC (SEQ ID NO.: 628) |
| 116 | 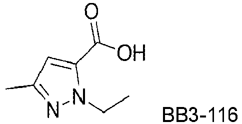 | GTGTCGCGGTCGA (SEQ ID NO.: 629) | GACCGCGACACAC (SEQ ID NO.: 630) |
| 117 | 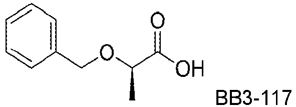 | AGGTACATTCAGA (SEQ ID NO.: 631) | TGAATGTACCTAC (SEQ ID NO.: 632) |
| 118 | 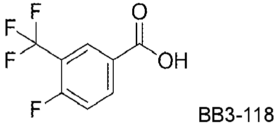 | TCCATATTGTTGA (SEQ ID NO.: 633) | AACAATATGGAAC (SEQ ID NO.: 634) |
| 119 | 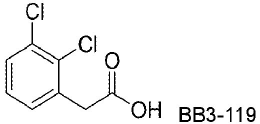 | TAGTACAATTCGA (SEQ ID NO.: 635) | GAATTGTACTAAC (SEQ ID NO.: 636) |
| 120 | 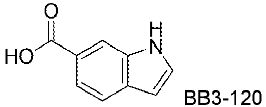 | CCAGCTAGGACGA (SEQ ID NO.: 637) | GTCCTAGCTGGAC (SEQ ID NO.: 638) |
| 121 | 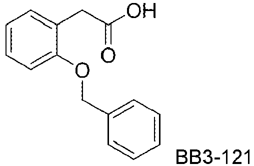 | TGAATATGCTTGA (SEQ ID NO.: 639) | AAGCATATTCAAC (SEQ ID NO.: 640) |
| 122 | 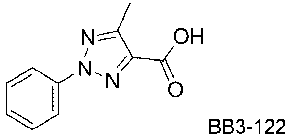 | ACGCTAGGATTGA (SEQ ID NO.: 641) | AATCCTAGCGTAC (SEQ ID NO.: 642) |
| 123 | 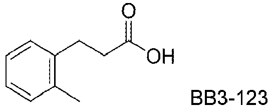 | CCTATATTAATGA (SEQ ID NO.: 643) | ATTAATATAGGAC (SEQ ID NO.: 644) |
| 124 | 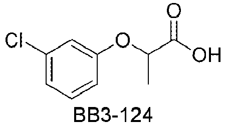 | TAGATCAGCCAGA (SEQ ID NO.: 645) | TGGCTGATCTAAC (SEQ ID NO.: 646) |
| 125 | 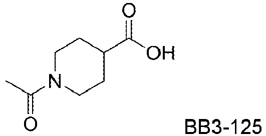 | TCGCATGGCTCGA (SEQ ID NO.: 647) | GAGCCATGCGAAC (SEQ ID NO.: 648) |
| 126 | 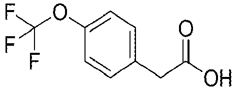 | AATATAACTCTGA (SEQ ID NO.: 649) | AGAGTTATATTAC (SEQ ID NO.: 650) |
| | 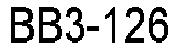 | | |
| 127 | 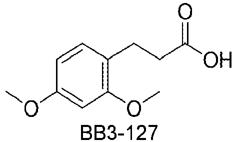 | GAACGCGCACCGA (SEQ ID NO.: 651) | GGTGCGCGTTCAC (SEQ ID NO.: 652) |
| 128 | 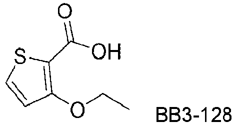 | CGACGTGTCCGGA (SEQ ID NO.: 653) | CGGACACGTCGAC (SEQ ID NO.: 654) |
| 129 | 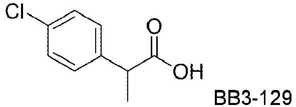 | TCCGCGATCATGA (SEQ ID NO.: 655) | ATGATCGCGGAAC (SEQ ID NO.: 656) |
| 130 | 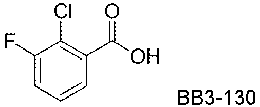 | CGTATAATATAGA (SEQ ID NO.: 657) | TATATTATACGAC (SEQ ID NO.: 658) |
| 131 | 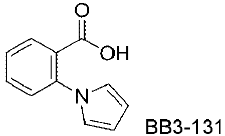 | GATATACAGATGA (SEQ ID NO.: 659) | ATCTGTATATCAC (SEQ ID NO.: 660) |
| 132 | 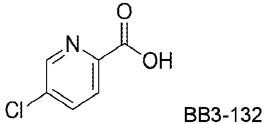 | CCGTTATACTAGA (SEQ ID NO.: 661) | TAGTATAACGGAC (SEQ ID NO.: 662) |
| 133 | 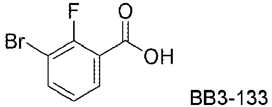 | CCACGTGTGAAGA (SEQ ID NO.: 663) | TTCACACGTGGAC (SEQ ID NO.: 664) |
| 134 | 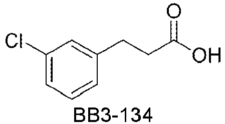 | GAGTTGCAGGCGA (SEQ ID NO.: 665) | GCCTGCAACTCAC (SEQ ID NO.: 666) |
| 135 | 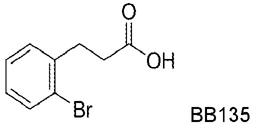 | TATGTCGAATTGA (SEQ ID NO.: 667) | AATTCGACATAAC (SEQ ID NO.: 668) |
| 136 | 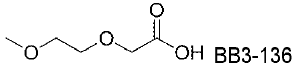 | CCAATTGCGACGA (SEQ ID NO.: 669) | GTCGCAATTGGAC (SEQ ID NO.: 670) |
| 137 | 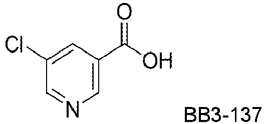 | GAATATAGATAGA (SEQ ID NO.: 671) | TATCTATATTCAC (SEQ ID NO.: 672) |
| 138 | 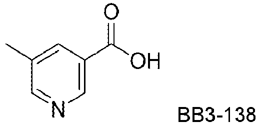 | TACAGCTTAGTGA (SEQ ID NO.: 673) | ACTAAGCTGTAAC (SEQ ID NO.: 674) |
| 139 | 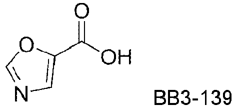 | TACACGCGTCTGA (SEQ ID NO.: 675) | AGACGCGTGTAAC (SEQ ID NO.: 676) |
| 140 | 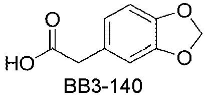 | ACCGTACTCGTGA (SEQ ID NO.: 677) | ACGAGTACGGTAC (SEQ ID NO.: 678) |
| 141 | 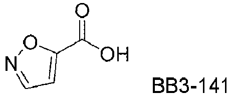 | TACATTGTCGTGA (SEQ ID NO.: 679) | ACGACAATGTAAC (SEQ ID NO.: 680) |
| 142 | 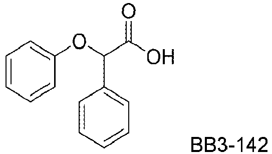 | ATCCTAGTAGAGA (SEQ ID NO.: 681) | TCTACTAGGATAC (SEQ ID NO.: 682) |
| 143 | 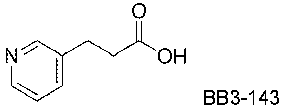 | GAGATCACAGTGA (SEQ ID NO.: 683) | ACTGTGATCTCAC (SEQ ID NO.: 684) |
| 144 | 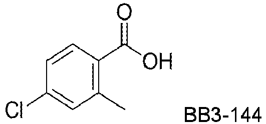 | CACATGGATACGA (SEQ ID NO.: 685) | GTATCCATGTGAC (SEQ ID NO.: 686) |
| 145 | 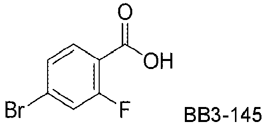 | GACAAGCTAGAGA (SEQ ID NO.: 687) | TCTAGCTTGTCAC (SEQ ID NO.: 688) |
| 146 | 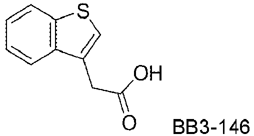 | TACGGCGCTCGGA (SEQ ID NO.: 689) | CGAGCGCCGTAAC (SEQ ID NO.: 690) |
| 147 | 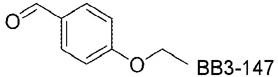 | AAGCTGCAGGAGA (SEQ ID NO.: 691) | TCCTGCAGCTTAC (SEQ ID NO.: 692) |
| 148 | 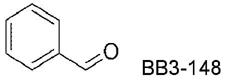 | TCCAGCTGGCTGA (SEQ ID NO.: 693) | AGCCAGCTGGAAC (SEQ ID NO.: 694) |
| 149 | 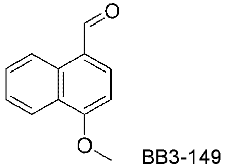 | ATACTGCAACGGA (SEQ ID NO.: 695) | CGTTGCAGTATAC (SEQ ID NO.: 696) |
| 150 | 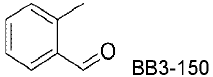 | TGACTGCACCAGA (SEQ ID NO.: 697) | TGGTGCAGTCAAC (SEQ ID NO.: 698) |
| 151 | 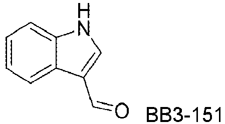 | AGCCGGCGCGTGA (SEQ ID NO.: 699) | ACGCGCCGGCTAC (SEQ ID NO.: 700) |
| 152 | 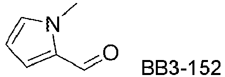 | CTACGTCGGTGGA (SEQ ID NO.: 701) | CACCGACGTAGAC (SEQ ID NO.: 702) |
| 153 | 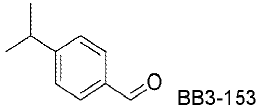 | ATGCTCGACCGGA (SEQ ID NO.: 703) | CGGTCGAGCATAC (SEQ ID NO.: 704) |
| 154 | 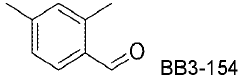 | CTCTATAATGTGA (SEQ ID NO.: 705) | ACATTATAGAGAC (SEQ ID NO.: 706) |
| 155 | 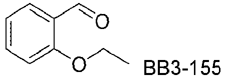 | CATAATTAACAGA (SEQ ID NO.: 707) | TGTTAATTATGAC (SEQ ID NO.: 708) |
| 156 | 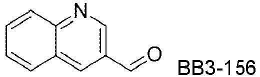 | GTGTACGGTCGGA (SEQ ID NO.: 709) | CGACCGTACACAC (SEQ ID NO.: 710) |
| 157 | 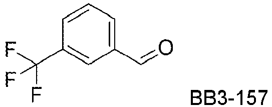 | TGAGTTAAGAAGA (SEQ ID NO.: 711) | TTCTTAACTCAAC (SEQ ID NO.: 712) |
| 158 | 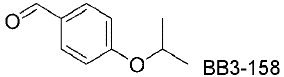 | GAGGATCAAGAGA (SEQ ID NO.: 713) | TCTTGATCCTCAC (SEQ ID NO.: 714) |
| 159 | 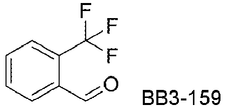 | TGGTGCGCTCAGA (SEQ ID NO.: 715) | TGAGCGCACCAAC (SEQ ID NO.: 716) |
| 160 | 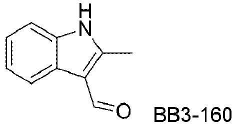 | AAGGACCTCAGGA (SEQ ID NO.: 717) | CTGAGGTCCTTAC (SEQ ID NO.: 718) |
| 161 | 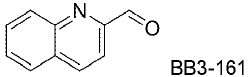 | GTGTACCGAGAGA (SEQ ID NO.: 719) | TCTCGGTACACAC (SEQ ID NO.: 720) |
| 162 | 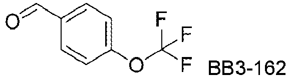 | GCAGCTCACCTGA (SEQ ID NO.: 721) | AGGTGAGCTGCAC (SEQ ID NO.: 722) |
| 163 | 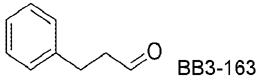 | GACGGATCGGTGA (SEQ ID NO.: 723) | ACCGATCCGTCAC (SEQ ID NO.: 724) |
| 164 | 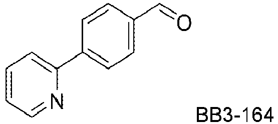 | CGCCGGACTACGA (SEQ ID NO.: 725) | GTAGTCCGGCGAC (SEQ ID NO.: 726) |
| 165 | 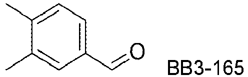 | CAGGCTAGCTCGA (SEQ ID NO.: 727) | GAGCTAGCCTGAC (SEQ ID NO.: 728) |
| 166 | 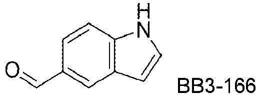 | CCATTAAGTTCGA (SEQ ID NO.: 729) | GAACTTAATGGAC (SEQ ID NO.: 730) |
| 167 | 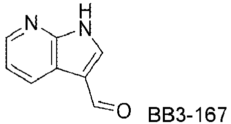 | CGTTGTACGGAGA (SEQ ID NO.: 731) | TCCGTACAACGAC (SEQ ID NO.: 732) |
| 168 | 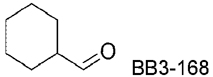 | CCTTAACCTCAGA (SEQ ID NO.: 733) | TGAGGTTAAGGAC (SEQ ID NO.: 734) |
| 169 | 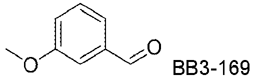 | CGGTACGTCATGA (SEQ ID NO.: 735) | ATGACGTACCGAC (SEQ ID NO.: 736) |
| 170 | 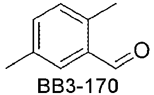 | TCTGCGCACGCGA (SEQ ID NO.: 737) | GCGTGCGCAGAAC (SEQ ID NO.: 738) |
| 171 | 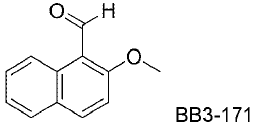 | CGTCGATTCACGA (SEQ ID NO.: 739) | GTGAATCGACGAC (SEQ ID NO.: 740) |
| 172 | 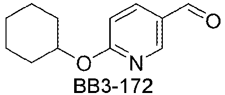 | ACGGCCTCCACGA (SEQ ID NO.: 741) | GTGGAGGCCGTAC (SEQ ID NO.: 742) |
| 173 | 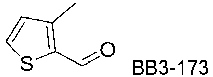 | TAGTGCATAACGA (SEQ ID NO.: 743) | GTTATGCACTAAC (SEQ ID NO.: 744) |
| 174 | 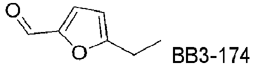 | TGATATACGGTGA (SEQ ID NO.: 745) | ACCGTATATCAAC (SEQ ID NO.: 746) |
| 175 | 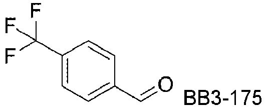 | TCATAGCTGGCGA (SEQ ID NO.: 747) | GCCAGCTATGAAC (SEQ ID NO.: 748) |
| 176 | 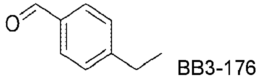 | ATGGCCAATAGGA (SEQ ID NO.: 749) | CTATTGGCCATAC (SEQ ID NO.: 750) |
| 177 | 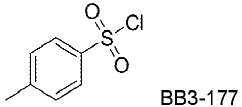 | ACTTACGTATCGA (SEQ ID NO.: 751) | GATACGTAAGTAC (SEQ ID NO.: 752) |
| 178 | 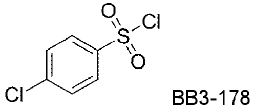 | TGAGCTGGAAGGA (SEQ ID NO.: 753) | CTTCCAGCTCAAC (SEQ ID NO.: 754) |
| 179 | 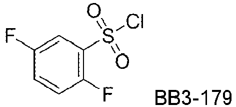 | CAAGCTAAGAAGA (SEQ ID NO.: 755) | TTCTTAGCTTGAC (SEQ ID NO.: 756) |
| 180 | 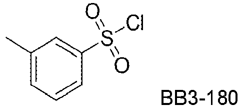 | AGTTAACAAGTGA (SEQ ID NO.: 757) | ACTTGTTAACTAC (SEQ ID NO.: 758) |
| 181 | 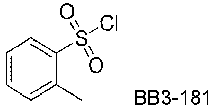 | ATTCCGCGTCGGA (SEQ ID NO.: 759) | CGACGCGGAATAC (SEQ ID NO.: 760) |
| 182 | 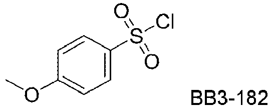 | ATAACTAGCTTGA (SEQ ID NO.: 761) | AAGCTAGTTATAC (SEQ ID NO.: 762) |
| 183 | 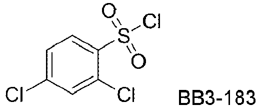 | GACAGATCGCGGA (SEQ ID NO.: 763) | CGCGATCTGTCAC (SEQ ID NO.: 764) |
| 184 | 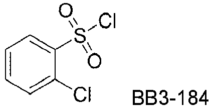 | CTAGTACGTAAGA (SEQ ID NO.: 765) | TTACGTACTAGAC (SEQ ID NO.: 766) |
| 185 | 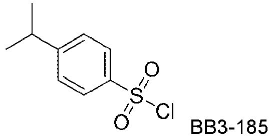 | CTTCAATTGCGGA (SEQ ID NO.: 767) | CGCAATTGAAGAC (SEQ ID NO.: 768) |
| 186 | 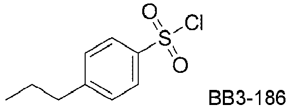 | TAGCCGCTCAAGA (SEQ ID NO.: 769) | TTGAGCGGCTAAC (SEQ ID NO.: 770) |
| 187 | 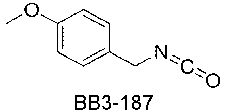 | GCGTCATGACTGA (SEQ ID NO.: 771) | AGTCATGACGCAC (SEQ ID NO.: 772) |
| 188 | 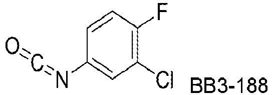 | GCGCCGGAGTGGA (SEQ ID NO.: 773) | CACTCCGGCGCAC (SEQ ID NO.: 774) |
| 189 | 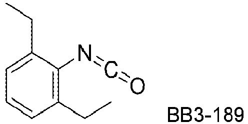 | CTTCCTAGATTGA (SEQ ID NO.: 775) | AATCTAGGAAGAC (SEQ ID NO.: 776) |
| 190 | 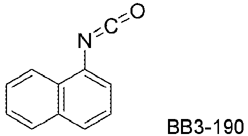 | GACAATTCTCAGA (SEQ ID NO.: 777) | TGAGAATTGTCAC (SEQ ID NO.: 778) |
| 191 | 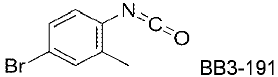 | CCTGCGCTAGAGA (SEQ ID NO.: 779) | TCTAGCGCAGGAC (SEQ ID NO.: 780) |
| 192 | 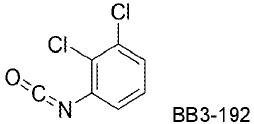 | ACGGCATGTGAGA (SEQ ID NO.: 781) | TCACATGCCGTAC (SEQ ID NO.: 782) |
| 193 | 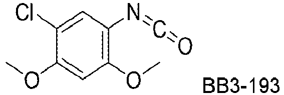 | TCTGCGCTCTGGA (SEQ ID NO.: 783) | CAGAGCGCAGAAC (SEQ ID NO.: 784) |
| 194 | 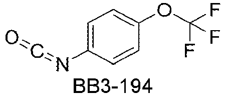 | TCAGCTAGCTTGA (SEQ ID NO.: 785) | AAGCTAGCTGAAC (SEQ ID NO.: 786) |
| 195 | 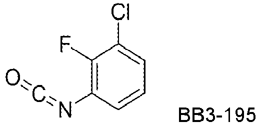 | ATGCAATTATAGA (SEQ ID NO.: 787) | TATAATTGCATAC (SEQ ID NO.: 788) |
| 196 | 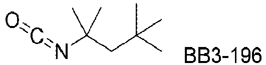 | GTCCGGTCTCGGA (SEQ ID NO.: 789) | CGAGACCGGACAC (SEQ ID NO.: 790) |
| 197 | Null for acid | TAGATCAAGTCGA (SEQ ID NO.: 791) | GACTTGATCTAAC (SEQ ID NO.: 792) |
| 198 | Null for aldehyde | GAGGTTAACAAGA (SEQ ID NO.: 793) | TTGTTAACCTCAC (SEQ ID NO.: 794) |
| 199 | Null for sulfonyl chloride | TGCTAGTGGATGA (SEQ ID NO.: 795) | ATCCACTAGCAAC (SEQ ID NO.: 796) |
| 200 | Null for isocyanate | AATCGACCTTAGA (SEQ ID NO.: 797) | TAAGGTCGATTAC (SEQ ID NO.: 798) |

### Step 5: Closing primer ligation:

Prior to selection, 100 nmol aliquots of library were ligated with closing primer using T4 DNA ligase. The primer duplex was then filled by Klenow polymerization. The generic sequence of the closing primer was:
5' X1X2X3...Xn 3'
3' CTX1'X2'X3'...Xn'N1N2N3...NmAGATCTGATGGCGCGAGGG 5' (SEQ ID NO.: 799)
5' end of top strand was phosphorylated. The variable region X1X2X3...Xn (n is an integer of 5 or greater) (X1'X2'X3'...Xn' is reverse complementary to X1X2X3...Xn) was unique to each closing primer and served as an identifier for the particular library and selection experiment. The variable region N1N2N3... Nm (m is an integer of 5 or greater) was a randomized sequence used to identify PCR duplication in the sequence data.

Each of X and N is independently selected from A, T, G or C.

### Example 7. Application of reverse condensation reactions to functional moiety

### General procedure for reverse condensation of DNA-linker containing an acid with amine building blocks:

HUB (20 nmol) was dissolved in pH 9.4 borate buffer (250 mM, 20 µL) to make 1 mM solution. 80 equivalents of diacid in 0.2 M DMA stock (8.0 µL, 1.6 µmol), 80 equivalents of HATU in 0.4 M DMA stock (4.0 µL, 1.6 µmol) and 80 equivalents of DIPEA in 0.4 M DMA stock (4.0 µL, 1.6 µmol) were cooled at 4 degree for 5 min, and then mixed together. After vortex, the "premix" stock was cooled at 4 degree for 5 min, then transferred to DNA solution. After vortex, the reaction was allowed to proceed at room temperature overnight. After completion of the material monitored by LCMS, the product was then purified by ethanol precipitation as described above and isolated by centrifugation. The resulting DNA pellet was dried by lyophilization.

To a 1mM solution of the above product (20 nmol) in pH 5.5 PBS buffer (250 mM, 20 µL)was added 300 equivalents of amine in 0.6 M MeCN stock (10 µL, 6 µmol), followed by adding 900 equivalents of DMTMM in 0.9 M water solution (20 µL, 18 µmol). After mixing the reaction, the mixture was allowed to proceed at room temperature overnight. After completion of the material monitored by LCMS, the product was then purified by ethanol precipitation as described above and isolated by centrifugation. The resulting DNA pellet was dried by lyophilization.

### Example 8. Application of azide reduction reactions to functional moiety

AOP-HUB was prepared following the same procedure as described above.

### General procedure for azide reduction of DNA-linker containing an azide with TPPTS:

AOP-HUB (20 nmol) was dissolved in pH 9.4 borate buffer (250 mM, 20 µL) to make 1 mM solution. 80 equivalents of azidoacetic acid in 0.2 M fresh made DMA solution (8.0 µL, 1.6 µmol), 80 equivalents of HATU in 0.4 M DMA stock (4.0 µL, 1.6 µmol), and 80 equivalents of DIPEA in 0.4 M DMA stock (4.0 µL, 1.6 µmol) were cooled at 4 degree for 5 min, and then mixed together. After vortex, the "premix" stock was cooled at 4 degree for 5 min, then transferred to DNA solution. After vortex, the reaction was allowed to proceed at room temperature overnight. After completion of the material, the product was then purified by ethanol precipitation as described above and isolated by centrifugation. The resulting DNA pellet was dried by lyophilization.

The above product (20 nmol) was dissolved in water (20 µL) to make 1 mM solution, and then 40 equivalents of TPPTS in 0.2 M water solution (4 µL, 0.8 µmol) was added. After mixing the reaction, the mixture was allowed to proceed at room temperature overnight. Then adjust pH to 5-6 with 0.5M HCl aqueous solution. After mixing the reaction, the mixture was allowed to proceed at room temperature for 4h. After completion of the material monitored by LCMS, the product was then purified by ethanol precipitation as described above and isolated by centrifugation. The resulting DNA pellet was dried by lyophilization.

### Example 9. Application of in-situ carbamylation reactions to functional moiety

AOP-HUB was prepared following the same procedure as described above.

### General procedure for in-situ carbamylation of DNA-linker containing an isocyanate with amide:

A 1 mM solution of AOP-HUB (20 nmol) was dissolved in pH 9.4 borate buffer (250 mM, 20 µL) was added 40 equivalents of 4-Nitrophenyl chloroformate in 0.2 M fresh made CH₃CN solution (4 µL, 0.8 µmol). Then followed by 40 equivalents of amine in 0.2 M CH₃CN/H₂O (1:1) stock (4 µL, 0.8 µmol). The reaction was allowed to proceed at room temperature overnight. After analyzing by LCMS, the product was then purified by ethanol precipitation as described above and isolated by centrifugation. The resulting DNA pellet was dried by lyophilization.

### Example 10. Application of in-situ thiourea reactions to functional moiety

AOP-HUB was prepared following the same procedure as described above.

### General procedure for in-situ thiourea of DNA-linker containing an isothiocyanates with amide:

A 1mM solution of AOP-HUB (20 nmol) was dissolved in pH 9.4 borate buffer (250 mM, 20 µL) was added 20 equivalents of di-2-pyridyl thionacarbonate in 0.2 M fresh made CH₃CN solution (2 µL, 0.4 µmol). The mixture was allowed to sit at r.t. for 10-15 min. Then followed by 200 equivalents of amine in 0.2 M CH₃CN/H₂O (1:1) stock (8 µL, 4 µmol). The reaction was allowed to proceed at 60 degree overnight. After analyzing by LCMS, the product was then purified by ethanol precipitation as described above and isolated by centrifugation. The resulting DNA pellet was dried by lyophilization.

### Example 11. Application of SNAr reactions to functional moiety

AOP-HUB was prepared following the same procedure as described above.

### General procedure for SNAr of DNA-linker containing an amide with Arylhalide

A 1 mM solution of AOP-HUB (20 nmol) was dissolved in pH 9.4 borate buffer (250 mM, 20 µL) was added 100 equivalents of Arylhalide in 0.2 M DMA solution (10 µL, 2 µmol). The reaction was sealed and heated at 60 degree for overnight. After completion of the material monitored by LCMS, the product was then purified by ethanol precipitation as described above and isolated by centrifugation. The resulting DNA pellet was dried by lyophilization.

### Example 12. Application of Buchwald reactions to functional moiety

AOP-HUB was prepared following the same procedure as described above.

### General procedure for Buchwald of DNA-linker containing an Arylhalide with amide

AOP-HUB (20 nmol) was dissolved in pH 9.4 borate buffer (250 mM, 20 µL) to make 1 mM solution. 80 equivalents of aryl halide (normally aryl bromide or iodide) in 0.2 M DMA stock (8.0 µL, 1.6 µmol), 80 equivalents of HATU in 0.4 M DMA stock (4.0 µL, 1.6 µmol) and 80 equivalents of DIPEA in 0.4 M DMA stock (4.0 µL, 1.6 µmol) were cooled at 4 degree for 5 min, and then mixed together. After vortex, the "premix" stock was cooled at 4 degree for 5 min, then transferred to DNA solution. After vortex, the reaction was allowed to proceed at room temperature overnight. After completion of the material monitored by LCMS, the product was then purified by ethanol precipitation as described above and isolated by centrifugation. The resulting DNA pellet was dried by lyophilization.

The above product (20 nmol) was dissolved in water (13.5 µL) to make 1.5 mM solution, and then was added. 80 equivalents of amine in 0.4 M DMA stock (4.0 µL, 1.6 µmol), 300 equivalents of NaOH in 1M water solution (6 µL, 6 µmol), 1.25 equivalents of Pdt-BuphosG3 in 12.5 mM DMA (2 µL, 25 nmol) were added. The reaction was allowed to proceed at 80 degree for 1h. After completion of the material monitored by LCMS, the product was then purified by ethanol precipitation as described above and isolated by centrifugation. The resulting DNA pellet was dried by lyophilization.

### Example 13. Application of nitro reduction reactions to functional moiety

AOP-HUB was prepared following the same procedure as described above.

### General procedure for nitro reduction of DNA-linker containing a nitro

A product of DNA-linker containing a nitro (20 nmol) was dissolved in pH 9.4 borate buffer (250 mM, 20 µL) to make 1 mM solution. 50 equivalents of FeSO₄ in 0.2 M fresh made aqueous solution (5 µL, 1 µmol) and NaOH in 1 M fresh made aqueous solution (10%v/v, 2.5 µL) were added. The reaction was conducted at 80°C for 2h. After completion of the material monitored by LCMS, the product was then purified by ethanol precipitation as described above and isolated by centrifugation. The resulting DNA pellet was dried by lyophilization.

### Example 14. Expansion to 4 or more functional moieties tethered on a single compound

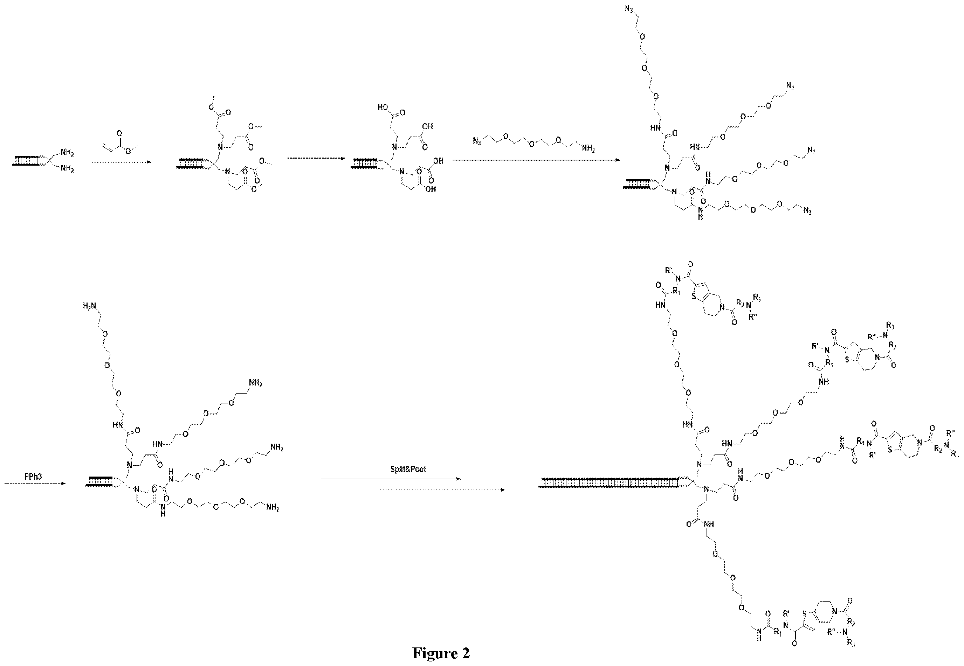

An exemplary scheme for synthesis of a compound having 4 functional moieties attached thereto is shown in Figure 2.

Similarly to Example 6, starting with Michael addition on the HUB, followed by hydrolysis and amide coupling would afford the azide terminus oligonucleotide which can be used directly as the starting oligonucleotide of the synthesis of DNA-encoded libraries. Here we use Staudinger reduction to furnish the amine terminus oligonucleotide. By following the exact procedure in Example 6, a new 107 membered DNA-encoded library can be synthesized in which each four identical molecules are attached to one DNA sequence and sharing one set of codes.
R1: 96 amino acids +1 Null
R2: 99 amino acids +1 Null
R3: 196 (acids, aldehyde, sulfonyl chlorides, isocyanates) + 4 Null

The synthesis of a library was accomplished using the following reagents:
Building blocks, oligonucleotide tags and their correspondence were shown in the following each cycle.

The same forward primer (with 5'-PHO) as Example 6 was used.

### Example 15. Synthesis of tool compound conjugates for HUB based selection study

The following tool compound and conjugates were used for HUB based selection study using Rock2 protein:

| Type | Reagent Name | Description |
|---|---|---|
| Compound | Compound 1 | Positive Rock2 inhibitor selected from HitGen DEL screening. The IC₅₀ of the inhibitor is ∼50nM. |
| Monovalent | Compound 1-HP-Conjugate | AOP-HP conjugate with Compound 1 |

| | | |
|---|---|---|
| conjugate | AOP-HP-Conjugate | AOP-HP conjugate without Compound 1, used as control |
| Divalent conjugate | Compound 1-HUB-Conjugate | AOP-HUB conjugate with Compound 1 |
| | AOP-HUB-Conjugate | AOP-HUB conjugate without Compound 1, used as control |

### Synthesis of conjugation of AOP-HP with compound 1 (Rock2 inhibitor)

200 nmol AOP-HP was dissolved in pH 9.4 borate buffer (250 mM) to make 1 mM solution. Stocks of 40 eq. compound 1 (200 mM in DMA), 40 eq. HATU (200 mM in DMA), and 40 eq. DIPEA **(200** mM in DMA) were cooled at 0°C for 5 min, and then mixed together. After vortex, the "premix" stock was cooled at 4°C for 5 min, then transferred to DNA solution, vortex again. The reaction was allowed to proceed at 30°C overnight. After monitoring by LCMS, the reaction was precipitated by adding 10% 5M NaCl aqueous 32 µl and 3 times volume of absolute EtOH 1056 µL, and then cooled in dry ice for 30 min. The oligo was isolated as a pellet by centrifugation for 30 min, and the supernatant was removed to give the product.

### Synthesis of conjugation of AOP-HUB with compound 1 (Rock2 inhibitor)

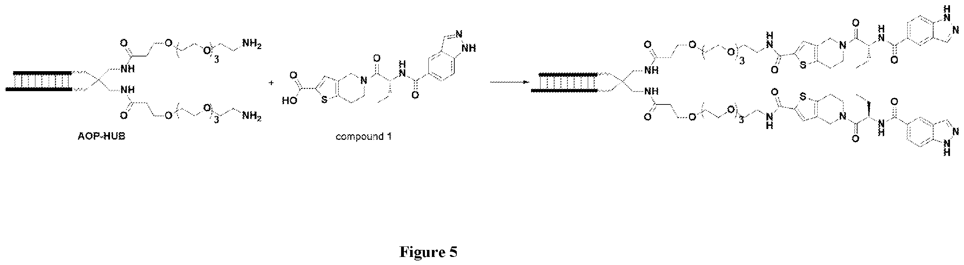

An exemplary synthesis scheme is shown in Figure 5.

700 nmol AOP-HUB was dissolved in pH 9.4 borate buffer (250 mM) to make 1 mM solution. Stocks of 80 equivalent compound 1 (200 mM in DMA), 80 equivalent HATU (200 mM in DMA), and 80 equivalent DIPEA (200 mM in DMA) were cooled at 0°C for 5 min, and then mixed together. After vortex, the "premix" stock was cooled at 4°C for 5 min, then transferred to DNA solution, vortex again. The reaction was allowed to proceed at 30°C overnight. After monitoring by LCMS, the reaction was precipitated by adding 10 vol% 5M NaCl aqueous 154 ul and 3 times volume of absolute EtOH 5.08 mL, and then cooled in dry ice for 30 min. The oligo was isolated as a pellet by centrifugation for 30 min, and the supernatant was removed. The isolated pellet was redissolved in 700 µL water, purified by prep-HPLC and dried by lyophilization to give the product.

### Encoding procedure of conjugates

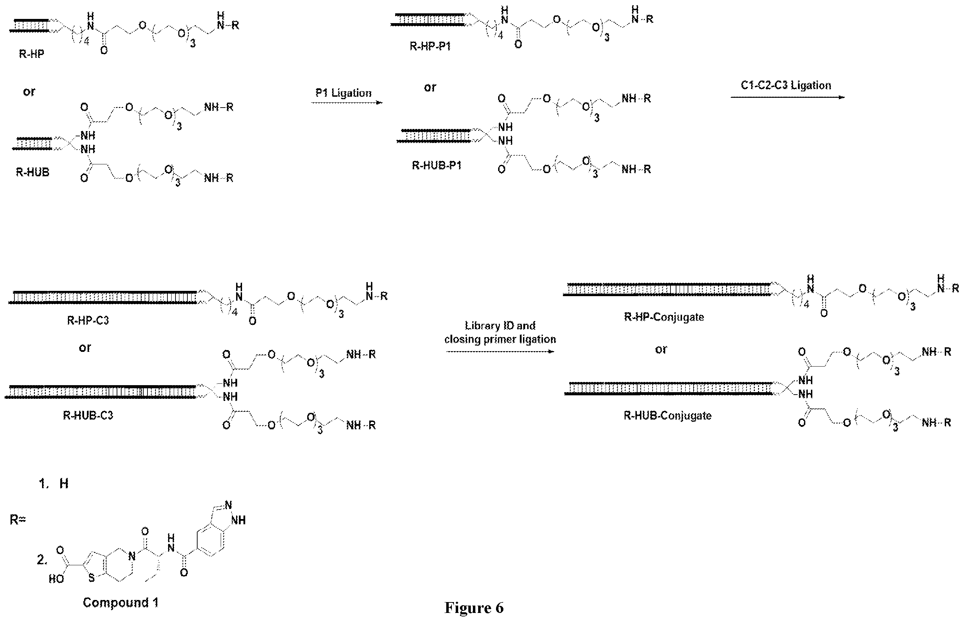

An exemplary encoding procedure of conjugates is shown in Figure 6. Note that P1 is the same as the forward primer of Example 6. "Library ID" has the following sequence:

Library ID= 5' AGGCTTACGNNNNNNNNNNNNNAGATCTGATGGCGCGAGGG 3' (SEQ ID NO.: 800)
3' CTTCCGAATGC 5' (SEQ ID NO.: 801)

| **Conjugate Name** | **C1-C2-C3 Sequence** |
|---|---|
| Compound 1-HP-Conjugate | 5' TGTCGCAGCATAGACCGTCGTCAAGTGCTTAGTGCCTGA 3' (SEQ ID NO.: 802) |
| | 3'ACACAGCGTCGTATCTGGCAGCAGTTCACGAATCACGGA 5' (SEQ ID NO.: 803) |
| AOP-HP-Conjugate | 5' CGTATCAGCACAGTTACTCCGTCTGTGGTAGAACGGTGA 3' (SEQ ID NO.: 804) |
| | 3'ACGCATAGTCGTGTCAATGAGGCAGACACCATGTTGCCA 5' (SEQ ID NO.: 805) |
| Compound 1-HUB-Conjugate | 5' TGTCGCAGCATAGCAGAGTCAGAAGTGCTTAGTGCCTGA 3' (SEQ ID NO.: 806) |
| | 3'ACACAGCGTCGTATCGTCTCAGTCTTCACGAATCACGGA 5' (SEQ ID NO.: 807) |
| AOP-HUB-Conjugate | 5' CGTATCAGCACAGATAGCCACTCTGTGGTAGAACGGTGA 3' (SEQ ID NO.: 808) |
| | 3'ACGCATAGTCGTGTCTATCGGTGAGACACCATGTTGCCA 5' (SEQ ID NO.: 809) |

Encoding strategy and DNA sequence used for AOP-HP with/without compound 1 or AOP-HUB with/without compound 1 is shown above. Firstly, P1 sequence (double stranded DNA sequence with 2bp overhang) was ligated into starting material R-HP or R-HUB to generate R-HP-P1 or R-HUB-P1. Then, encoding sequence C1-C2-C3 (double stranded DNA sequence with 2bp overhang and with the same encoding length as that of DNA encoded library) was ligated into R-HP-P1 or R-HUB-P1 to generate R-HP-C3 or P-HUB-C3. Finally, Library ID and closing primer sequence (double stranded DNA sequence with 2bp overhang) was ligated into R-HP-C3 or P-HUB-C3 to obtain the desired conjugate R-HP-Conjugate or R-HUB-Conjugate. The detailed ligation procedure can refer to the previous application **Example 6** (Synthesis and characterization of a DNA-encoded library in the order of 107 members).

### Example 16. Target protein Rock2 based selection of divalent vs. monovalent conjugates spiked into DEL

### Conjugates spike into DEL procedure

Two DEL libraries of different compound size were selected as background DEL for divalent vs. monovalent conjugates to spike into and use 10⁵ copy of each compound in the DEL quantified by qPCR. The size of DEL0936 is 28.69 million and the size of DEL1019 is 1108 million. For each Spike-into DEL, two sets of samples are prepared with the ratio of HUB containing components: HP containing components: DEL component of 1:1:1 or 0.5:1:1. In theory, divalent HUB conjugate should use half of DNA copy quantified by qPCR than that of monovalent HP conjugate to reach the same level of compound. Specifically, the detailed information of the four samples with five components each is listed in the table below.

| **Spike-into DEL** | **DEL size (Million)** | **Sample Name** | **Component** | **Copy number per selection** |
|---|---|---|---|---|
| DEL0936 | 28.7 | HUB+HP+DEL0936 (1:1:1) | Compound 1-HUB | 10⁵ |
| | | | AOP-HUB | 10⁵ |
| | | | Compound 1-HP | 10⁵ |
| | | | AOP-HP | 10⁵ |
| | | | DEL0936 | 10⁵ |
| | | HUB+HP+DEL0936 (0.5:1:1) | Compound 1-HUB | 0.5x10⁵ |
| | | | AOP-HUB | 0.5x10⁵ |
| | | | Compound 1-HP | 10⁵ |
| | | | AOP-HP | 10⁵ |
| | | | DEL0936 | 10⁵ |
| DEL1019 | 1108 | HUB+HP+DEL1019 (1:1:1)** | Compound 1-HUB | 10⁵ |
| | | | AOP-HUB | 10⁵ |
| | | | Compound 1-HP | 10⁵ |
| | | | AOP-HP | 10⁵ |
| | | | DEL1019 | 10⁵ |
| | | HUB+HP+DEL1019 (0.5:1:1)** | Compound 1-HUB | 0.5x10⁵ |
| | | | AOP-HUB | 0.5x10⁵ |
| | | | Compound 1-HP | 10⁵ |
| | | | AOP-HP | 10⁵ |
| | | | DEL1019 | 10⁵ |

### Selections

Rock2 protein used in the selection was expressed in insect SF9 cell with N-(His)6 tag. The protein was purified by Ni-NTA affinity column followed by size exclusion column. Automated selection was carried out using a KingFisher Duo Prime Purification System in 96-well plate. After 20 µL Ni-charged MagBeads was equilibrated with 150 µL selection buffer (40 mM Tris, 20 mM MgCl2, 50 µM DTT, 0.1% Tween-20, 0.3mg/mL shared salmon sperm DNA, 10mM Imidazole, pH 7.5), beads were transferred to a new row and incubated with 300 pmol Histagged Rock2 at RT for 30min in a 60 µL volume in selection buffer. Then the immobilized protein along with the beads was incubated with conjugates spiked-into DEL sample in 75 µL selection buffer at RT for 1h followed by 1 min wash at RT in 50 µL selection buffer. Retained DEL members were recovered by heat elution in 60 µL of elution buffer (40 mM Tris, 20 mM MgCl2, pH 7.5) at 75 °C for 15 min. After the first round of selection, another one or two rounds of selection were repeated. The heat eluted portion of each round was used as the input to the successive round with fresh protein. Two rounds of selection are used for DEL0936 containing sample enrichment, and three round selection was used for DEL1019 containing sample enrichment. After each round, the output was quantified by qPCR. When total DNA output was brought down to 10⁷ to 10⁸ copies, the selection was done and the output was amplified by PCR and further purified by Qiagen PCR purification kit according to the manufacture's instruction. Then sequencing was performed for PCR amplified samples on an Illumina HiSeq 2500.

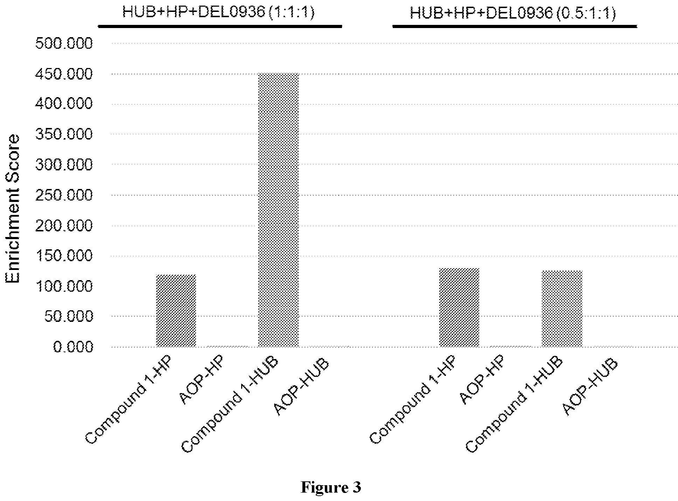

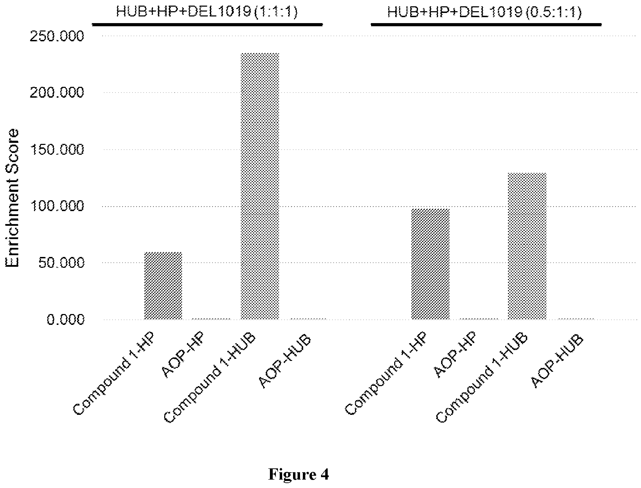

The enrichment score is calculated using Poisson distribution with -log10 transformation. As shown in Figure 3, the Compound1-HUB conjugate showed about 3-fold higher enrichment compared to the Compound1-HP conjugate in the HUB+HP+DEL0936 (1:1:1) selection condition, and can reach the same level of enrichment in the HUB+HP+DEL0936 (0.5:1:1) selection condition. The enrichment was Compound1-dependent because all the AOP conjugates showed no enrichment in the same selection condition. Similar results can also be observed in the HUB+HP+DEL1019 (1:1:1) selection condition (Figure 4), where a much higher background noise exist in the selection condition compare with the DEL0936 selection samples.

### Abbreviations

THF: Tetrahydrofuran
FmocCI: 9-Fluorenylmethyl chloroformate
PE: Petroleum ether
EA: ethyl acetate
DCM: dichloromethane
DMTr-Cl: 4,4'-Dimethoxytrityl Chloride
DIEA; DIPEA: N,N-Diisopropylethylamine
CEPCI: 2-Cyanoethyl N,N-diisopropylchlorophosphoramidite
DMA: N,N-Dimethylaniline
HATU: O-(7-Aza-1H-benzotriazol-1-yl)-N,N,N',N'-tetramethyluronium hexafluorophosphate
TBTA: tert-Butyl 2,2,2-trichloroacetimidate
POPd ([(t-Bu)2P-(OH)]2PdCl2: DIHYDROGEN DICHLOROBIS(DI-T-BUTYLPHOSPHINITO-KP)PALLADATE(2-)
DTT: Dithiothreitol
DMT-MM: 4-(4,6-Dimethoxy-1,3,5-triazin-2-yl)-4-methylmorpholinium chloride

### EQUIVALENTS

The present disclosure provides among other things DNA-encoded libraries and how to make and use them. While specific embodiments of the subject disclosure have been discussed, the above specification is illustrative and not restrictive. Many variations of the disclosure will become apparent to those skilled in the art upon review of this specification. The full scope of the disclosure should be determined by reference to the claims, and the specification, along with such variations.

## Claims

1. A compound of Formula II: 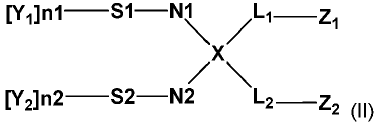 wherein:
X is an atom having a valence of at least 4;
L1 is a first linker having a valence of at least 2, optionally comprising a phosphate group;
Z1 is a first single-stranded oligonucleotide attached at its 3' terminus to the first linker, optionally comprising a phosphate group;
L2 is a second linker having a valence of at least 2;
Z2 is a second single-stranded oligonucleotide attached at its 5' terminus to the second linker;
N1 comprises a first functional group capable of forming at least one covalent bond with S1;
N2 comprises a second functional group capable of forming at least one covalent bond with S2;
S1 and S2 are each independently a spacer having a valence of at least 2;
[Y1]n1 comprises the first functional moiety and [Y2]n2 comprises the second functional moiety, wherein:
Y1 and Y2, at each occurrence, are each independently a functional moiety comprising one or more synthons;
n1 and n2 are each independently an integer of at least 1;
Y1 and Y2 are the same functional moiety; and
Z1 and Z2 are substantially complementary.

2. The compound of claim 1, wherein X is a carbon atom or a phosphorus atom.

3. The compound of claim 1, wherein the first and/or second functional groups are amine groups; wherein optionally the first and/or second functional groups are capable of forming two covalent bonds.

4. The compound of claim 1, wherein S1 and S2 each independently comprises an alkylene chain (such as -(CH₂)ₙ-) or an polyethylene glycol) chain (such as-(CH₂CH₂O)ₙ-), wherein n is an integer from 1 to 20, preferably from 2 to about 12, more preferably from 4 to 10.

5. The compound of claim 1, wherein Z1 and Z2 each further comprise a PCR primer binding site sequence.

6. The compound of claim 1, wherein Y1 and Y2 each independently comprise a functional group, preferably selected from an amino group, a hydroxyl group, a carboxylic acid group, a thiol group, a halide, an azido group, an alkyne group, or an alkene group.

7. The compound of claim 1, wherein L1 and L2 each are -OPO⁻₃-(CH₂CH₂O)ₙ-PO⁻₃-, wherein n is an integer selected from 1 to 10, preferably from 2 to about 6, more preferably 3.

8. A compound library comprising at least about 10² distinct compound species, each species having the compound of any one of claims 1-7, wherein each species comprises at least two functional moieties that are operably linked to a double-stranded oligonucleotide which uniquely identifies the at least two functional moieties.

9. The compound library of claim 8, wherein the library comprises at least about 10⁵ or at least about 10⁷ copies of each of the distinct compound species.

10. A method of synthesizing the compound of any one of claims 1-7, comprising:
(a) providing an initiator compound comprising at least two reactive groups operably linked to an initial oligonucleotide;
(b) reacting the initiator compound with a first synthon comprising a complementary reactive group that is complementary to the at least two reactive groups, thereby producing a cycle 1 product have two copies of the first synthon attached thereto;
(c) joining the initial oligonucleotide with a first incoming oligonucleotide which identifies the first synthon, thereby forming a first encoding oligonucleotide that encodes the cycle 1 product;
(d) optionally, reacting the cycle 1 product with a second synthon and joining the first encoding oligonucleotide with a second incoming oligonucleotide, thereby producing a cycle 2 product encoded by a second encoding oligonucleotide; and
(e) optionally, repeating step (d) i-1 more times using an ith synthon and an ith incoming oligonucleotide, wherein i is an integer of 2 or greater, thereby forming the compound of any one of claims 4-10 having the first and second functional moieties.

11. The method of claim 10, wherein step (c) precedes step (b) or step (b) precedes step (c).

12. The method of claim 10 or 11, wherein each synthon is an amino acid or an activated amino acid.

13. The method of claim 10 or 11, wherein the reactive groups and the complementary reactive group are each independently selected from the group consisting of an amino group, an aldehyde group, a ketone group, a hydroxyl group, an alkyne group, an azide group, a carboxyl group, a sulfonyl group, a phosphonyl group, an epoxide group, an aziridine group, a phosphorous ylide group, an isocyanate group, a halogenated heteroaromatic group, and a nucleophile; wherein preferably the halogenated heteroaromatic group is selected from the group consisting of chlorinated pyrimidines, chlorinated triazines and chlorinated purines, and/or wherein preferably the nucleophile comprises an amino group.

14. The method of claim 10 or 11, wherein step (b) is conducted under reducing conditions.

15. The method of claim 10 or 11, wherein step (b) comprises reacting via cycloaddition to form a cyclic structure.

16. The method of claim 10 or 11, wherein step (c) is conducted in the presence of an enzyme that is preferably selected from the group consisting of a DNA ligase, an RNA ligase, a DNA polymerase, an RNA polymerase and a topoisomerase.

17. The method of claim 10 or 11, wherein the initial oligonucleotide comprises a PCR primer binding site sequence.

18. The method of claim 10 or 11, wherein each incoming oligonucleotide is from 3 to 30 nucleotides in length.

19. The method of claim 10, wherein the ith incoming oligonucleotide comprises a PCR closing primer.

20. The method of any one of claims 10, 11 and 19, further comprising after step (e), cyclizing the first and/or second functional moiety.

21. The method of claim 20, wherein the first and/or second functional moiety each comprises an alkynyl group and an azido group, and wherein the compound is subjected to conditions suitable for cycloaddition of the alkynyl group and the azido group to form a triazole group, thereby cyclizing the first and/or second functional moiety.

22. A method of synthesizing a library of compounds of any one of claim 8-9, wherein the compounds comprise at least two functional moieties comprising two or more building blocks which are operably linked to an oligonucleotide which identifies the structure of the at least two functional moieties, the method comprising the steps of:
(a) providing a solution comprising m initiator compounds, wherein m is an integer of 1 or greater, wherein the m initiator compounds each comprise at least two initial functional moieties comprising n building blocks, where n is an integer of 1 or greater, which is operably linked to an initial oligonucleotide which identifies the n building blocks;
(b) dividing the solution of step (a) into r reaction vessels, wherein r is an integer of 2 or greater, thereby producing r aliquots of the solution;
(c) reacting the initiator compounds in each reaction vessel with one of r building blocks, thereby producing r aliquots comprising compounds comprising at least two functional moieties comprising n+1 building blocks operably linked to the initial oligonucleotide; and
(d) reacting the initial oligonucleotide in each aliquot with one of a set of r distinct incoming oligonucleotides in the presence of an enzyme which catalyzes the ligation of the incoming oligonucleotide and the initial oligonucleotide, under conditions suitable for enzymatic ligation of the incoming oligonucleotide and the initial oligonucleotide;
thereby producing r aliquots comprising compounds comprising at least two functional moieties operably linked to an elongated oligonucleotide which encodes the building blocks.

23. The method of Claim 22, further comprising the step of (e) combining two or more of the r aliquots, thereby producing a solution comprising compounds comprising at least two functional moieties which are operably linked to an elongated oligonucleotide which encodes the building blocks.

24. The method of Claim 23, wherein the steps (a) to (e) are conducted one or more times to yield cycles 1 to i, where i is an integer of 2 or greater, wherein in cycle s+1, where s is an integer of i-1 or less, the solution comprising m initiator compounds of step (a) is the solution of step (e) of cycle s.

25. The method of Claim 24, wherein in at least one of cycles 1 to i step (d) precedes step (c).

26. A method for identifying one or more compounds which bind to a biological target, the method comprising the steps of:
(a) contacting the biological target with a compound library prepared by the method of any one of Claims 22-25 under conditions suitable for at least one member of the compound library to bind to the target;
(b) removing library members that do not bind to the target;
(c) amplifying the encoding oligonucleotides of the at least one member of the compound library which binds to the target;
(d) sequencing the encoding oligonucleotides of step (c); and
(e) using the sequences determined in step (d) to determine the structure of the functional moieties of the members of the compound library which bind to the biological target;
thereby identifying one or more compounds which bind to the biological target.

27. A method for identifying a compound which binds to a biological target, the method comprising the steps of:
(a) contacting the biological target with the compound library of claim 8 under conditions suitable for at least one member of the compound library to bind to the target;
(b) removing library members that do not bind to the target;
(c) amplifying the encoding oligonucleotides of the at least one member of the compound library which binds to the target;
(d) sequencing the encoding oligonucleotides of step (c); and
(e) using the sequences determined in step (d) to determine the structure of the functional moieties of the members of the compound library which bind to the biological target;
thereby identifying one or more compounds which bind to the biological target.

28. The method of Claim 27, wherein the library comprises at least about 10⁵ or at least about 10⁷ copies of each of the distinct compound species.

## Patentansprüche

1. Verbindung der Formel II: 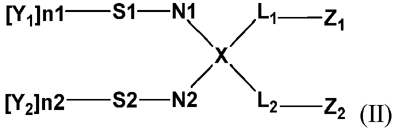 worin:
X ein Atom mit einer Wertigkeit von mindestens 4 ist,
L1 ein erster Linker mit einer Wertigkeit von mindestens 2 ist, der gegebenenfalls eine Phosphatgruppe umfasst,
Z1 ein erstes einzelsträngiges Oligonukleotid ist, das an seinem 3'-Terminus an den ersten Linker gebunden ist und gegebenenfalls eine Phosphatgruppe umfasst;
L2 ist ein zweiter Linker mit einer Wertigkeit von mindestens 2;
Z2 ein zweites einzelsträngiges Oligonukleotid ist, das an seinem 5'-Terminus an den zweiten Linker gebunden ist,
N1 eine erste funktionelle Gruppe umfasst, die in der Lage ist, mindestens eine kovalente Bindung mit S1 zu bilden;
N2 eine zweite funktionelle Gruppe umfasst, die in der Lage ist, mindestens eine kovalente Bindung mit S2 zu bilden;
S1 und S2 jeweils unabhängig voneinander ein Spacer mit einer Wertigkeit von mindestens 2 sind;
[Y1]n1 die erste funktionelle Gruppe umfasst und [Y2]n2 die zweite funktionelle Gruppe umfasst, wobei:
Y1 und Y2 bei jedem Auftreten jeweils unabhängig voneinander eine funktionelle Gruppe sind, die ein oder mehrere Synthone umfasst,
n1 und n2 jeweils unabhängig voneinander eine ganze Zahl von mindestens 1 sind;
Y1 und Y2 die gleiche funktionelle Einheit sind, und
Z1 und Z2 im wesentlichen komplementär sind.

2. Verbindung nach Anspruch 1, wobei X ein Kohlenstoffatom oder ein Phosphoratom ist.

3. Verbindung nach Anspruch 1, wobei die ersten und/oder zweiten funktionellen Gruppen Amingruppen sind, wobei gegebenenfalls die ersten und/oder zweiten funktionellen Gruppen in der Lage sind, zwei kovalente Bindungen zu bilden.

4. Verbindung nach Anspruch 1, wobei S1 und S2 jeweils unabhängig voneinander eine Alkylenkette (wie -(CH₂)n-) oder eine Poly(ethylenglykol)kette (wie -(CH₂CH₂O)ₙ-) umfassen, wobei n eine ganze Zahl von 1 bis 20, vorzugsweise von 2 bis etwa 12, besonders bevorzugt von 4 bis 10 ist.

5. Verbindung nach Anspruch 1, wobei Z1 und Z2 jeweils weiterhin eine PCR-Primer-Bindungsstellensequenz umfassen.

6. Verbindung nach Anspruch 1, wobei Y1 und Y2 jeweils unabhängig voneinander eine funktionelle Gruppe umfassen, die vorzugsweise aus einer Aminogruppe, einer Hydroxylgruppe, einer Carbonsäuregruppe, einer Thiolgruppe, einem Halogenid, einer Azidogruppe, einer Alkinogruppe oder einer Alkengruppe ausgewählte ist.

7. Verbindung nach Anspruch 1, wobei L1 und L2 jeweils -OPO⁻₃-(CH₂CH₂O)ₙ-PO⁻₃- sind, wobei n eine ganze Zahl von 1 bis 10, vorzugsweise von 2 bis etwa 6, besonders bevorzugt 3, ist.

8. Bibliothek von Verbindungen, umfassend mindestens etwa 10² verschiedene verbindungsspezifische Spezies, wobei jede Spezies die Verbindung nach einem der Ansprüche 1-7 aufweist, wobei jede Spezies mindestens zwei funktionelle Einheiten umfasst, die operativ mit einem doppelsträngigen Oligonukleotid verbunden sind, das die mindestens zwei funktionellen Einheiten eindeutig identifiziert.

9. Bibliothek von Verbindungen nach Anspruch 8, wobei die Bibliothek mindestens etwa 10⁵ oder mindestens etwa 10⁷ Kopien von jeder der verschiedenen verbindungsspezifischen Spezies umfasst.

10. Verfahren zur Synthetisierung der Verbindung nach einem der Ansprüche 1-7, umfassend:
(a) Bereitstellen einer Initiator-Verbindung, die mindestens zwei reaktive Gruppen umfasst, die operativ an ein initiales Oligonukleotid gebunden sind,
(b) Reagierenlassen der Initiator-Verbindung mit einem ersten Synthon, das eine komplementäre reaktive Gruppe umfasst, die komplementär zu den mindestens zwei reaktiven Gruppen ist, wodurch ein Zyklus-1-Produkt erzeugt wird, an das zwei Kopien des ersten Synthons gebunden sind,
(c) Verbinden des initialen Oligonukleotids mit einem ersten ankommenden Oligonukleotid, das das erste Synthon identifiziert, wodurch ein erstes kodierendes Oligonukleotid gebildet wird, das das Zyklus-1-Produkt kodiert;
(d) gegebenenfalls Reagierenlassen des Zyklus-1-Produkts mit einem zweiten Synthon und Verbinden des ersten kodierenden Oligonukleotids mit einem zweiten ankommenden Oligonukleotid, wodurch ein Zyklus-2-Produkt erzeugt wird, das durch ein zweites kodierendes Oligonukleotid kodiert wird, und
(e) gegebenenfalls Wiederholen von Schritt (d) i-1 mehrmals unter Verwendung eines i-tenSynthons und eines i-ten ankommenden Oligonukleotids, wobei i eine ganze Zahl von 2 oder größer ist, wodurch die Verbindung nach einem der Ansprüche 4-10 gebildet wird, die die erste und zweite funktionelle Einheiten aufweist.

11. Verfahren nach Anspruch 10, wobei der Schritt (c) dem Schritt (b) oder der Schritt (b) dem Schritt (c) vorausgeht.

12. Verfahren nach Anspruch 10 oder 11, wobei jedes Synthon eine Aminosäure oder eine aktivierte Aminosäure ist.

13. Verfahren nach Anspruch 10 oder 11, wobei die reaktiven Gruppen und die komplementäre reaktive Gruppe jeweils unabhängig voneinander aus der Gruppe ausgewählt sind, die aus einer Aminogruppe, einer Aldehydgruppe, einer Ketongruppe, einer Hydroxylgruppe, einer Alkinogruppe, einer Azidgruppe, einer Carboxylgruppe, einer Sulfonylgruppe, einer Phosphonylgruppe, einer Epoxidgruppe, einer Aziridingruppe, einer Phosphor-Ylid-Gruppe, einer Isocyanatgruppe, einer halogenierten heteroaromatischen Gruppe und einem Nukleophil besteht, wobei die halogenierte heteroaromatische Gruppe vorzugsweise aus der Gruppe ausgewählt ist, die aus chlorierten Pyrimidinen, chlorierten Triazinen und chlorierten Purinen besteht, und/oder wobei das Nucleophil vorzugsweise eine Aminogruppe umfasst.

14. Verfahren nach Anspruch 10 oder 11, wobei Schritt (b) unter reduzierenden Bedingungen durchgeführt wird.

15. Verfahren nach Anspruch 10 oder 11, wobei Schritt (b) das Reagieren über Cycloaddition zur Bildung einer cyclischen Struktur umfasst.

16. Verfahren nach Anspruch 10 oder 11, wobei Schritt (c) in Gegenwart eines Enzyms durchgeführt wird, das vorzugsweise aus der Gruppe ausgewählt ist, die aus einer DNA-Ligase, einer RNA-Ligase, einer DNA-Polymerase, einer RNA-Polymerase und einer Topoisomerase besteht.

17. Verfahren nach Anspruch 10 oder 11, wobei das initiale Oligonukleotid eine PCR-Primer-Bindungsstellensequenz umfasst.

18. Verfahren nach Anspruch 10 oder 11, wobei jedes ankommende Oligonukleotid eine Länge von 3 bis 30 Nukleotiden aufweist.

19. Verfahren nach Anspruch 10, wobei das i-te ankommende Oligonukleotid einen PCR-Abschlussprimer umfasst.

20. Verfahren nach einem der Ansprüche 10, 11 und 19, ferner umfassend nach Schritt (e), das Zyklisieren der ersten und/oder zweiten funktionellen Einheit.

21. Verfahren nach Anspruch 20, wobei die erste und/oder zweite funktionelle Einheit jeweils eine Alkinylgruppe und eine Azidogruppe umfasst, und wobei die Verbindung Bedingungen unterworfen wird, die für eine Cycloaddition der Alkinylgruppe und der Azidogruppe geeignet sind, um eine Triazolgruppe zu bilden, wodurch die erste und/oder zweite funktionelle Einheit zyklisiert wird.

22. Verfahren zur Synthetisierung einer Bibliothek von Verbindungen nach einem der Ansprüche 8 bis 9, wobei die Verbindungen mindestens zwei funktionelle Einheiten umfassen, die zwei oder mehr Bausteine umfassen, die operativ mit einem Oligonukleotid verbunden sind, das die Struktur der mindestens zwei funktionellen Einheiten identifiziert, wobei das Verfahren die folgenden Schritte umfasst:
(a) Bereitstellen einer Lösung, die m Initiator-Verbindungen umfasst, wobei m eine ganze Zahl von 1 oder größer ist, wobei die m Initiator-Verbindungen jeweils mindestens zwei initiale funktionelle Einheiten umfassen, die n Bausteine umfassen, wobei n eine ganze Zahl von 1 oder größer ist, die operativ an ein initiales Oligonukleotid gebunden sind, das die n Bausteine identifiziert,
(b) Aufteilen der Lösung von Schritt (a) in r Reaktionsgefäße, wobei r eine ganze Zahl von 2 oder größer ist, wodurch r Aliquots der Lösung hergestellt werden,
(c) Reagierenlassen der Initiator-Verbindungen in jedem Reaktionsgefäß mit einem von r Bausteinen, wodurch r Aliquots hergestellt werden, die Verbindungen umfassen, die mindestens zwei funktionelle Einheiten umfassen, die n+1 Bausteine umfassen, die operativ mit dem initialen Oligonukleotid verbunden sind, und
(d) Reagierenlassen des initialen Oligonukleotids in jedem Aliquot mit einem aus einem Satz von r verschiedenen ankommenden Oligonukleotiden in Gegenwart eines Enzyms, das die Ligation des ankommenden Oligonukleotids und des initialen Oligonukleotids katalysiert, unter Bedingungen, die für die enzymatische Ligation des ankommenden Oligonukleotids und des initialen Oligonukleotids geeignet sind,
wodurch r Aliquots hergestellt werden, die Verbindungen umfassen, die mindestens zwei funktionelle Einheiten umfassen, die operativ mit einem verlängerten Oligonukleotid verbunden sind, das die Bausteine kodiert.

23. Verfahren nach Anspruch 22, das ferner den Schritt (e) des Kombinierens von zwei oder mehr der r Aliquots umfasst, wodurch eine Lösung hergestellt wird, die Verbindungen umfasst, die mindestens zwei funktionelle Einheiten umfassen, die funktionsfähig mit einem verlängerten Oligonukleotid verbunden sind, das die Bausteine kodiert.

24. Verfahren nach Anspruch 23, wobei die Schritte (a) bis (e) einmal oder mehrmals durchgeführt werden, um die Zyklen 1 bis i zu ergeben, wobei i eine ganze Zahl von 2 oder größer ist, wobei im Zyklus s+1, wobei s eine ganze Zahl von i-1 oder weniger ist, die Lösung, die m Initiator-Verbindungen von Schritt (a) umfasst, die Lösung von Schritt (e) des Zyklus s ist.

25. Verfahren nach Anspruch 24, wobei in mindestens einem der Zyklen 1 bis i der Schritt (d) dem Schritt (c) vorausgeht.

26. Verfahren zum Identifizieren einer oder mehrerer Verbindungen, die an ein biologisches Target binden, wobei das Verfahren die folgenden Schritte umfasst:
(a) Inkontaktbringen des biologischen Targets mit einer Bibliothek von Verbindungen, die durch das Verfahren nach einem der Ansprüche 22-25 hergestellt wurde, unter Bedingungen, die geeignet sind, dass mindestens ein Mitglied der Bibliothek von Verbindungen an das Target bindet;
(b) Entfernen von Mitgliedern der Bibliothek von Verbindungen, die nicht an das Target binden;
(c) Amplifizieren der kodierenden Oligonukleotide des mindestens einen Mitglieds der Bibliothek von Verbindungen, das an das Target bindet;
(d) Sequenzieren der kodierenden Oligonukleotide aus Schritt (c); und
(e) Verwenden der in Schritt (d) bestimmten Sequenzen, um die Struktur der funktionellen Teile der Mitglieder der Bibliothek von Verbindungen zu bestimmen, die an das biologische Target binden;
wodurch eine oder mehrere Verbindungen identifiziert werden, die an das biologische Target binden.

27. Verfahren zum Identifizieren einer Verbindung, die an ein biologisches Target bindet, wobei das Verfahren die folgenden Schritte umfasst:
(a) Inkontaktbringen des biologischen Targets mit der Bibliothek von Verbindungen nach Anspruch 8 unter Bedingungen, die geeignet sind, dass mindestens ein Mitglied der Bibliothek von Verbindungen an das Target bindet;
(b) Entfernen von Mitgliedern der Bibliothek von Verbindungen, die nicht an das Target binden;
(c) Amplifizieren der kodierenden Oligonukleotide des mindestens einen Mitglieds der Bibliothek von Verbindungen, das an das Target bindet;
(d) Sequenzieren der kodierenden Oligonukleotide aus Schritt (c); und
(e) Verwendung der in Schritt (d) bestimmten Sequenzen zur Bestimmung der Struktur der funktionellen Teile der Mitglieder der Bibliothek von Verbindungen, die an das biologische Target binden;
wodurch eine oder mehrere Verbindungen identifiziert werden, die an das biologische Target binden.

28. Verfahren nach Anspruch 27, wobei die Bibliothek mindestens etwa 10⁵ oder mindestens etwa 10⁷ Kopien von jeder der verschiedenen verbindungsspezifischen Spezies umfasst.

## Revendications

1. Un composé de formule II : 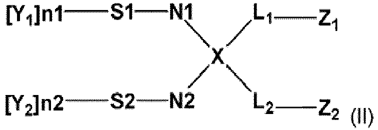 dans lequel :
X est un atome ayant une valence d'au moins 4;
L1 est un premier lieur ayant une valence d'au moins 2, comprenant éventuellement un groupe phosphate;
Z1 est un premier oligonucléotide monocaténaire attaché à son extrémité 3' audit premier lieur, comprenant éventuellement un groupe phosphate;
L2 est un deuxième lieur ayant une valence d'au moins 2;
Z2 est un deuxième oligonucléotide monocaténaire attaché à son extrémité 5' audit deuxième lieur;
N1 comprend un premier groupe fonctionnel capable de former au moins une liaison covalente avec S1;
N2 comprend un deuxième groupe fonctionnel capable de former au moins une liaison covalente avec S2;
S1 et S2 sont chacun indépendamment un espaceur ayant une valence d'au moins 2;
[Y1]n1 comprend ledit premier fragment fonctionnel et [Y2]n2 comprend ledit deuxième fragment fonctionnel, dans lequel :
Y1 et Y2, à chaque occurrence, sont chacun indépendamment un fragment fonctionnel comprenant un ou plusieurs synthons;
n1 et n2 sont chacun indépendamment un nombre entier d'au moins 1;
Y1 et Y2 sont le même fragment fonctionnel; et
Z1 et Z2 sont sensiblement complémentaires.

2. Ledit composé selon la revendication 1, dans lequel X est un atome de carbone ou un atome de phosphore.

3. Ledit composé selon la revendication 1, dans lequel lesdits premier et/ou deuxième groupes fonctionnels sont des groupes amine; dans lequel éventuellement lesdits premier et/ou deuxième groupes fonctionnels sont capables de former deux liaisons covalentes.

4. Ledit composé selon la revendication 1, dans lequel S1 et S2 chacun indépendamment comprennent une chaîne alkylène (telle que -(CH₂)ₙ-) ou une chaîne poly(éthylène glycol) (telle que -(CH₂CH₂O)ₙ-), dans lequel n est un nombre entier de 1 à 20, de préférence de 2 à environ 12, plus préférablement de 4 à 10.

5. Ledit composé selon la revendication 1, dans lequel Z1 et Z2 chacun comprennent en outre une séquence de site de liaison d'amorce de PCR.

6. Ledit composé selon la revendication 1, dans lequel Y1 et Y2 chacun indépendamment comprennent un groupe fonctionnel, de préférence choisi parmi un groupe amino, un groupe hydroxyle, un groupe acide carboxylique, un groupe thiol, un halogénure, un groupe azido, un groupe alcyne, ou un groupe alcène.

7. Ledit composé selon la revendication 1, dans lequel L1 et L2 chacun sont -OPO⁻₃-(CH₂CH₂O)ₙ-PO⁻₃-, dans lequel n est un nombre entier choisi de 1 à 10, de préférence de 2 à environ 6, plus préférablement 3.

8. Une bibliothèque de composés comprenant au moins environ 10² espèces de composés distinctes, chaque espèce ayant ledit composé selon l'une quelconque des revendications 1 à 7, dans laquelle chaque espèce comprend au moins deux fragments fonctionnels qui sont liés de manière opérationnelle à un oligonucléotide bicaténaire qui identifie de manière unique lesdites au moins deux fragments fonctionnels.

9. Ladite bibliothèque de composés selon la revendication 8, dans laquelle ladite bibliothèque comprend au moins environ 10⁵ ou au moins environ 10⁷ copies de chacune desdites espèces de composés distinctes.

10. Procédé de synthèse dudit composé selon l'une quelconque des revendications 1 à 7, comprenant :
(a) fournir un composé initiateur comprenant au moins deux groupes réactifs liés de manière fonctionnelle à un oligonucléotide initial;
(b) faire réagir ledit composé initiateur avec un premier synthon comprenant un groupe réactif complémentaire qui est complémentaire desdits au moins deux groupes réactifs, produisant ainsi un produit du cycle 1 auquel sont attachées deux copies dudit premier synthon;
(c) joindre ledit oligonucléotide initial avec un premier oligonucléotide entrant qui identifie ledit premier synthon, formant ainsi un premier oligonucléotide codant qui code ledit produit du cycle 1;
(d) éventuellement, faire réagir ledit produit du cycle 1 avec un deuxième synthon et joindre ledit premier oligonucléotide codant avec un deuxième oligonucléotide entrant, produisant ainsi un produit du cycle 2 codé par un deuxième oligonucléotide codant; et
(e) éventuellement, répéter l'étape (d) i-1 fois de plus en utilisant un i^{ème} synthon et un i^{ème} oligonucléotide entrant, dans lequel i est un nombre entier de 2 ou plus, formant ainsi ledit composé selon l'une quelconque des revendications 4 à 10 ayant ledit premier et deuxième fragments fonctionnels.

11. Ledit procédé selon la revendication 10, dans lequel l'étape (c) précède l'étape (b) ou l'étape (b) précède l'étape (c).

12. Ledit procédé selon la revendication 10 ou 11, dans lequel chaque synthon est un acide aminé ou un acide aminé activé.

13. Ledit procédé selon la revendication 10 ou 11, dans lequel lesdits groupes réactifs et ledit groupe réactif complémentaire sont chacun indépendamment sélectionnés parmi le groupe consistant en un groupe amino, un groupe aldéhyde, un groupe cétone, un groupe hydroxyle, un groupe alcyne, un groupe azide, un groupe carboxyle, un groupe sulfonyle, un groupe phosphonyle, un groupe époxyde, un groupe aziridine, un groupe ylure phosphoreux, un groupe isocyanate, un groupe hétéroaromatique halogéné et un nucléophile; dans lequel, de préférence, ledit groupe hétéroaromatique halogéné est choisi dans le groupe constitué de pyrimidines chlorées, de triazines chlorées et de purines chlorées, et/ou dans lequel, de préférence, ledit nucléophile comprend un groupe amino.

14. Ledit procédé selon la revendication 10 ou 11, dans lequel l'étape (b) est conduite dans des conditions réductrices.

15. Ledit procédé selon la revendication 10 ou 11, dans lequel l'étape (b) comprend la réaction par cycloaddition pour former une structure cyclique.

16. Ledit procédé selon la revendication 10 ou 11, dans lequel l'étape (c) est conduite en présence d'une enzyme qui est de préférence choisie dans le groupe consistant en une ADN ligase, une ARN ligase, une ADN polymérase, une ARN polymérase et une topoisomérase.

17. Ledit procédé selon la revendication 10 ou 11, dans lequel ledit oligonucléotide initial comprend une séquence de site de liaison d'amorce de PCR.

18. Ledit procédé selon la revendication 10 ou 11, dans lequel chaque oligonucléotide entrant a une longueur de 3 à 30 nucléotides.

19. Ledit procédé selon la revendication 10, dans lequel ledit i^{ème} oligonucléotide entrant comprend une amorce de fermeture de PCR.

20. Ledit procédé selon l'une quelconque des revendications 10, 11 et 19, comprenant en outre après l'étape (e), cycliser ledit premier et/ou deuxième fragment fonctionnel.

21. Ledit procédé selon la revendication 20, dans lequel ledit premier et/ou deuxième fragment fonctionnel chacun comprend un groupe alcynyle et un groupe azido, et dans lequel ledit composé est soumis à des conditions appropriées pour la cycloaddition dudit groupe alcynyle et dudit groupe azido pour former un groupe triazole, cyclisant ainsi ledit premier et/ou deuxième fragment fonctionnel.

22. Procédé de synthèse d'une bibliothèque de composés selon l'une quelconque des revendications 8 et 9, dans lequel lesdits composés comprennent au moins deux fragments fonctionnels comprenant deux blocs de construction ou plus qui sont liés de manière opérationnelle à un oligonucléotide qui identifie la structure desdits au moins deux fragments fonctionnels, ledit procédé comprenant les étapes consistant à :
(a) fournir une solution comprenant m composés initiateurs, dans lequel m est un nombre entier de 1 ou plus, dans laquelle lesdits m composés initiateurs comprennent chacun au moins deux fragments fonctionnels initiaux comprenant n blocs de construction, où n est un entier supérieur ou égal à 1, qui est lié de manière opérationnelle à un oligonucléotide initial qui identifie lesdits n blocs de construction,
(b) diviser ladite solution de l'étape (a) dans r récipients de réaction, où r est un nombre entier de 2 ou plus, produisant ainsi r aliquotes de ladite solution;
(c) faire réagir lesdits composés initiateurs dans chaque récipient de réaction avec l'un des r blocs de construction, produisant ainsi r aliquotes comprenant des composés comprenant au moins deux fragments fonctionnels comprenant n+1 blocs de construction liés de manière opérationnelle audit oligonucléotide initial; et
(d) faire réagir ledit oligonucléotide initial dans chaque aliquote avec l'un d'un ensemble de r oligonucléotides entrants distincts en présence d'une enzyme qui catalyse ladite ligature dudit oligonucléotide entrant et dudit oligonucléotide initial, dans des conditions appropriées pour la ligature enzymatique dudit oligonucléotide entrant et ledit oligonucléotide initial;
produisant ainsi des aliquotes r comprenant des composés comprenant au moins deux fragments fonctionnels liés de manière opérationnelle à un oligonucléotide allongé qui code lesdits blocs de construction.

23. Ledit procédé selon la revendication 22, comprenant en outre l'étape (e) combiner deux ou plusieurs desdites r aliquotes, produisant ainsi une solution comprenant des composés comprenant au moins deux fragments fonctionnels qui sont liés de manière opérationnelle à un oligonucléotide allongé qui code lesdits blocs de construction .

24. Ledit procédé selon la revendication 23, dans lequel les étapes (a) à (e) sont effectuées une ou plusieurs fois pour conduire aux cycles 1 à i, où i est un nombre entier de 2 ou plus, où dans le cycle s+1, où s est un nombre entier de i-1 ou moins, ladite solution comprenant m composés initiateurs de l'étape (a) est ladite solution de l'étape (e) du cycle s.

25. Ledit procédé selon la revendication 24, dans lequel dans au moins un des cycles 1 à i, l'étape (d) précède l'étape (c).

26. Procédé d'identification d'un ou plusieurs composés qui se lient à une cible biologique, ledit procédé comprenant les étapes consistant à :
(a) mettre en contact ladite cible biologique avec une bibliothèque de composés préparée par ledit procédé selon l'une quelconque des revendications 22 à 25 dans des conditions appropriées pour qu'au moins un membre de ladite bibliothèque de composés se lie à ladite cible;
(b) retirer les membres de la bibliothèque qui ne se lient pas à ladite cible;
(c) amplifier lesdits oligonucléotides codants dudit au moins un membre de ladite bibliothèque de composés qui se lie à ladite cible;
(d) séquencer lesdits oligonucléotides codants de l'étape (c); et
(e) utiliser lesdites séquences déterminées à l'étape (d) pour déterminer la structure desdites fragments fonctionnels desdits membres de ladite bibliothèque de composés qui se lient à ladite cible biologique,
identifiant ainsi un ou plusieurs composés qui se lient à ladite cible biologique.

27. Procédé d'identification d'un composé qui se lie à une cible biologique, ladite méthode comprenant les étapes consistant à :
(a) mettre en contact ladite cible biologique avec ladite bibliothèque de composés selon la revendication 8 dans des conditions appropriées pour qu'au moins un membre de ladite bibliothèque de composés se lie à ladite cible;
(b) retirer les membres de la bibliothèque qui ne se lient pas à ladite cible;
(c) amplifier lesdits oligonucléotides codants dudit au moins un membre de ladite bibliothèque de composés qui se lie à ladite cible;
(d) séquencer lesdits oligonucléotides codants de l'étape (c); et
(e) utiliser lesdites séquences déterminées à l'étape (d) pour déterminer la structure desdites fragments fonctionnels desdits membres de ladite bibliothèque de composés qui se lient à ladite cible biologique,
identifiant ainsi un ou plusieurs composés qui se lient à ladite cible biologique.

28. Ledit procédé selon la revendication 27, dans lequel ladite bibliothèque comprend au moins environ 10⁵ ou au moins environ 10⁷ copies de chacune desdites espèces composées distinctes.
